(19) Europäisches Patentamt
European Patent Office
Office européen des brevets

(11) Veröffentlichungsnummer : **0 065 483**
**B1**

(12) # EUROPÄISCHE PATENTSCHRIFT

(45) Veröffentlichungstag der Patentschrift : **18.09.85**

(21) Anmeldenummer : **82810169.1**

(22) Anmeldetag : **23.04.82**

(51) Int. Cl.⁴ : **C 07 C103/48**, C 07 C117/00, C 07 C125/065, C 07 D307/32, C 07 D307/68, C 07 D307/24, C 07 D231/12, C 07 D249/08, C 07 F 9/165, C 07 C121/43

(54) **Mikrobizide Acylanilin- und Acylnaphthylamin-Derivate, Verfahren zu deren Herstellung und ihre Verwendung.**

(30) Priorität : **29.04.81 CH 2783/81**

(43) Veröffentlichungstag der Anmeldung : **24.11.82 Patentblatt 82/47**

(45) Bekanntmachung des Hinweises auf die Patenterteilung : **18.09.85 Patentblatt 85/38**

(84) Benannte Vertragsstaaten : **AT BE CH DE FR GB IT LI LU NL SE**

(56) Entgegenhaltungen :
**FR-A- 2 267 042**
**FR-A- 2 442 226**
**Die Akte enthält technische Angaben, die nach dem Eingang der Anmeldung eingereicht wurden und die nicht in dieser Patentschrift enthalten sind.**

(73) Patentinhaber : **CIBA-GEIGY AG**
**Postfach**
**CH-4002 Basel (CH)**

(72) Erfinder : **Riebli, Peter**
**Dittingerstrasse 12**
**CH-4053 Basel (CH)**
Erfinder : **Fischer, Hanspeter, Dr.**
**Burggartenstrasse 14**
**CH-4103 Bottmingen (CH)**
Erfinder : **Thummel, Rudolph C.**
**Route d'Alle 424**
**CH-2892 Courgenay (CH)**
Erfinder : **Hubele, Adolf, Dr.**
**Obere Egg 9**
**CH-4465 Magden (CH)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 065 483 B1

**Beschreibung**

Die vorliegende Erfindung betrifft mikrobizid wirksame Acylalanin- und Acylnaphthylamin-Derivate, Verfahren zu ihrer Herstellung, Mittel, in denen diese als aktive Komponente vorliegen, sowie deren Verwendung zur Bekämpfung von Pilzen und Bakterien.

Die neuen Verbindungen entsprechen der allgemeinen Formel I

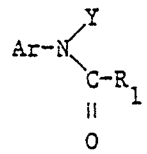 (I)

worin Ar einen Rest der Formeln

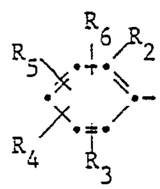 oder 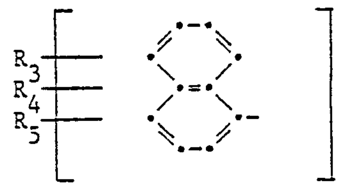 ,

$R_1$ gegebenenfalls durch Halogen substituiertes 2-Furyl, 2-Tetra-hydrofuryl, $C_2$-$C_4$-Alkenyl, Cyclopropyl, β-($C_1$-$C_4$)-Alkoxyethyl oder den Rest $-CH_2Z$ bedeutet, wobei Z für

a) —OH

b) —1H-1,2,4-Triazolyl, 1-Imidazolyl, 1-Pyrazolyl,

c) —$S(O)_n$—$R_{13}$

mit $R_{13}$ gleich $C_1$-$C_4$-Alkyl und n gleich 1 oder 2,

d) —X—$R_{14}$

mit X gleich Sauerstoff oder Schwefel und $R_{14}$ gleich gegebenenfalls durch $C_1$-$C_2$-Alkoxy substituiertes $C_1$-$C_6$-Alkyl, $C_3$-$C_4$-Alkenyl oder $C_3$-$C_4$-Alkinyl,

e) —$OSO_2$—$R_{15}$

mit $R_{15}$ gleich $C_1$-$C_4$ Alkyl, Mono- oder Di-($C_1$-$C_3$)-Alkylamin,

f) 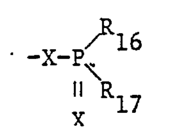

mit X gleich Sauerstoff oder Schwefel, $R_{16}$ gleich $C_1$-$C_4$-Alkyl, $C_1$-$C_4$-Alkoxy oder $C_1$-$C_4$-Alkylamino und $R_{17}$ gleich $C_1$-$C_4$-Alkoxy oder $C_1$-$C_4$ Alkylamino,

oder

g) 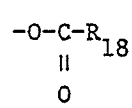

mit $R_{18}$ gleich gegebenenfalls durch $C_1$-$C_2$-Alkoxy substituiertes $C_1$-$C_3$-Alkyl steht,

$R_2$ $C_1$-$C_3$-Alkyl, $C_1$-$C_3$-Alkoxy oder Halogen,

$R_3$ 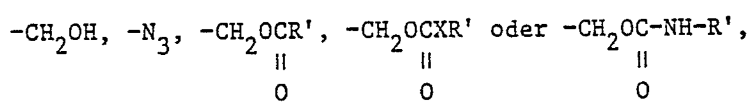

wobei R′ gegebenenfalls durch Halogen oder $C_1$-$C_3$-Alkoxy substituiertes $C_1$-$C_6$-Alkyl, $C_2$-$C_4$-Alkenyl oder Phenyl darstellt, und X für Sauerstoff oder Schwefel steht,

$R_4$ Wasserstoff, $C_1$-$C_3$-Alkyl, $C_1$-$C_3$-Alkoxy, Halogen, $—NO_2$ oder $—NH_2$,

$R_5$ Wasserstoff oder $C_1$-$C_3$-Alkyl,

$R_6$ Wasserstoff oder $C_1$-$C_3$-Alkyl,

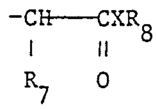 , 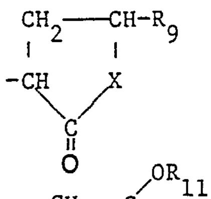 , 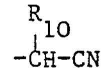

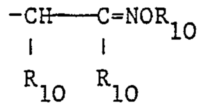 , 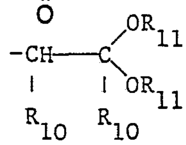 oder

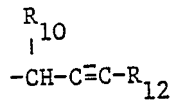 darstellt,

wobei

$R_7$ für Wasserstoff, gegebenenfalls durch Halogen, —OH, —$OC_1$-$C_3$-Alkyl oder $-O\underset{O}{\underset{\|}{C}}NH-C_1$-$C_3$-Alkyl substituiertes $C_1$-$C_2$-Alkyl,

$R_8$ für gegebenenfalls durch $C_1$-$C_2$-Alkoxy substituiertes $C_1$-$C_4$-Alkyl,

$R_9$ für Wasserstoff oder Methyl,

$R_{10}$ für Wasserstoff oder $C_1$-$C_3$-Alkyl,

$R_{11}$ für $C_1$-$C_4$-Alkyl, wobei $R_{11}$ zusammen eine ein- oder mehrfach durch $C_1$-$C_3$-Alkyl substituierte $C_2$-$C_3$-Alkylenbrücke darstellen können,

$R_{12}$ für Wasserstoff, Halogen, $C_1$-$C_2$-Alkyl oder Phenyl stehen und

X Sauerstoff oder Schwefel darstellt.

Die Substituenten $R_3$, $R_4$ und $R_5$ der von der Formel I umfassten Naphthylamin-Derivate fluktuieren, so dass jeder dieser Substituenten die Positionen 2, 3, 4, 5, 6, 7 oder 8 im Naphthylring einnehmen kann.

Unter Alkyl oder Alkylteil eines anderen Substituenten sind je nach Zahl der angegebenen Kohlenstoffatome beispielsweise folgende Gruppen zu verstehen : Methyl, Ethyl, Propyl oder Butyl sowie ihre Isomeren wie Isopropyl, iso-Butyl, sek.-Butyl oder tert.-Butyl. Alkenyl steht beispielsweise für Vinyl, Propenyl-(1), Allyl, Butenyl-(1), Butenyl-(2) oder Butenyl-(3), und Alkinyl bedeutet insbesondere Propargyl. Halogen steht hier und im folgenden für Fluor, Chlor, Brom oder Jod, vorzugsweise für Chlor oder Brom.

Eine bevorzugte Gruppe von erfindungsgemässen Mikrobiziden besteht aus Verbindungen der Formel I mit folgenden Substituenten-Kombinationen :

$R_1$ : 2-Furyl, 2-Tetrahydrofuryl, $C_2$-$C_4$-Alkenyl, Cyclopropyl, β—($C_1$-$C_2$)-Alkoxyethyl oder —$CH_2$—Z, wobei Z für

a) —OH

b) 1H—1,2,4-Triazolyl, 1-Imidazolyl,

c) —$S(O)_n$—$R_{13}$ mit $R_{13}$ gleich $C_1$-$C_2$-Alkyl und n gleich 2,

d) —X'—$R_{14}$ mit X' gleich Sauerstoff oder Schwefel und $R_{14}$ gleich gegebenenfalls durch $C_1$-$C_2$-Alkoxy substituiertes $C_1$-$C_6$-Alkyl, $C_3$-$C_4$-Alkenyl oder $C_3$-$C_4$-Alkinyl,

e) —$OSO_2$—$R_{15}$ mit $R_{15}$ gleich $C_1$-$C_2$-Alkyl, Mono- oder Di-($C_1$-$C_2$)-Alkylamin

f) 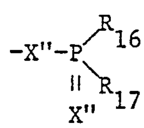

mit X" gleich Schwefel, $R_{16}$ gleich $C_1$-$C_2$-Alkyl, $C_1$-$C_4$-Alkoxy, $C_1$-$C_2$-Alkylamino und $R_{17}$ gleich $C_1$-$C_4$-Alkoxy oder $C_1$-$C_4$-Alkylamino, oder

g) $-O-\underset{O}{\underset{\|}{C}}-R_{18}$ mit $R_{18}$ gleich gegebenenfalls durch $C_1$-$C_2$-Alkoxy substituiertes $C_1$-$C_2$-Alkyl steht,

$R_2$ : $C_1$-$C_2$-Alkyl, $C_1$-$C_2$-Alkoxy oder Halogen,

$R_3$ :

$$-CH_2OH,\ -N_3,\ -CH_2O\underset{O}{\underset{\|}{C}}-R',\ -CH_2O\underset{O}{\underset{\|}{C}}X'''-R'\ \text{oder}\ -CH_2O\underset{O}{\underset{\|}{C}}NH-R'$$

wobei R′ für gegebenenfalls durch Chlor oder Methoxy substituiertes $C_1$-$C_3$-Alkyl oder $C_2$-$C_3$-Alkenyl steht und X‴ Sauerstoff oder Schwefel darstellt,

$R_4$: Wasserstoff, $C_1$-$C_2$-Alkyl, $C_1$-$C_2$-Alkoxy, $NO_2$ oder $NH_2$,

$R_5$ : Wasserstoff oder $C_1$-$C_2$-Alkyl,

$R_6$ : Wasserstoff oder $C_1$-$C_2$-Alkyl,

Y : die unter Formel I angegebenen Bedeutungen hat, wobei

R : Wasserstoff oder gegebenenfalls durch Halogen, —OH, —O($C_1$-$C_2$)-Alkyl oder $-O\underset{\underset{O}{\|}}{C}NH-(C_1-C_2)-$Alkyl substituiertes $C_1$-$C_2$-Alkyl,

$R_8$ : gegebenenfalls durch $C_1$-$C_2$-Alkoxy substituiertes $C_1$-$C_3$-Alkyl,

$R_9$ : Wasserstoff,

$R_{10}$ : Wasserstoff oder $C_1$-$C_2$-Alkyl,

$R_{11}$ : $C_1$-$C_2$-Alkyl, wobei $R_{11}$ zusammen auch $C_2$-$C_3$-Alkylen darstellen können,

$R_{12}$ : Wasserstoff, Halogen oder $C_1$-$C_2$-Alkyl und

X : Sauerstoff oder Schwefel bedeuten.

Eine weitere bevorzugte Gruppe von Mikrobiziden der Formel I wird durch folgende Substituenten-Kombinationen charakterisiert :

$R_1$ : 2-Furyl, 2-Tetrahydrofuryl, $C_2$-$C_4$-Alkenyl, Cyclopropyl, β-Methoxyethyl oder —$CH_2Z$, wobei Z für

a) 1H-1,2,4-Triazolyl,

b) —$S(O)_n$—$R_{13}$ mit $R_{13}$ gleich $CH_3$ und n gleich 2,

c) —$X^{IV}$—$R_{14}$ mit $X^{IV}$ gleich Sauerstoff und $R_{14}$ gleich gegebenenfalls durch $C_1$-$C_2$-Alkoxy substituiertes $C_1$-$C_4$-Alkyl, $C_3$-$C_4$-Alkenyl oder $C_3$-$C_4$-Alkinyl,

d) —$OSO_2$—$R_{15}$ mit $R_{15}$ gleich Mono-($C_1$-$C_3$)-Alkylamin,

e) 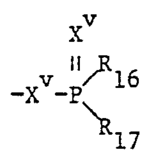

mit $X^V$ gleich Schwefel, $R_{16}$ und $R_{17}$ unabhängig voneinander gleich $C_1$-$C_3$-Alkoxy oder

f) $-O\underset{\underset{O}{\|}}{C}-R_{18}$ mit $R_{18}$ gleich $C_1$-$C_3$-Alkyl steht,

$R_2$: Methyl, Methoxy oder Chlor,

$R_3$ : —$CH_2OH$, —$N_3$ oder $-CH_2O\underset{\underset{O}{\|}}{C}CH_3$

$R_4$ : Wasserstoff, Methyl, Methoxy, $NO_2$ oder $NH_2$,

$R_5$ : Wasserstoff oder Methyl,

$R_6$ : Wasserstoff oder Methyl,

Y : die unter Formel I angegebenen Bedeutungen hat, wobei

$R_7$ : gegebenenfalls durch Chlor, —OH, —$OC_1$-$C_2$-Alkyl oder $-O\underset{\underset{O}{\|}}{C}NH-C_1-C_2$-Alkyl substituiertes $C_1$-$C_2$-Alkyl

$R_8$ : $C_1$-$C_3$-Alkyl,

$R_9$ : Wasserstoff,

$R_{10}$ : Methyl,

$R_{11}$ : $C_1$-$C_2$-Alkyl, wobei $R_{11}$ zusammen auch $C_2$-$C_3$-Alkylen darstellen kann,

$R_{12}$ : Wasserstoff, Brom, Jod oder $C_1$-$C_2$-Alkyl und

X : Sauerstoff bedeutet.

Verbindungen der letztgenannten Gruppe, worin $R_3$ Azido (—$N_3$) bedeutet, stellen besonders bevorzugte Wirkstoffe dar, insbesondere jene, worin Y die Reste —$CH(CH_3)COOCH_3$ oder

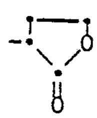

bedeutet.

Folgende Einzelverbindungen sind infolge ihrer ausgeprägten Wirkung gegen phytopathogene Mikroorganismen hervorzuheben :

N-(1'-Methoxycarbonyl-ethyl)-N-methoxyacetyl-2-methyl-6-azido-anilin,
N-(Tetrahydro-2'-on-fur-3'-yl)-N-methoxyacetyl-2,6-dimethyl-3-azido-anilin,
N-(1'-Methoxycarbonyl-ethyl)-N-methoxyacetyl-2,6-dimethyl-3-azidoanilin,
N-(1'-Methoxycarbonyl-ethyl)-N-(2-tetrahydrofuryl-carbonyl)-2-methyl-6-azido-anilin,
N-(1'-Methoxycarbonyl-3'-hydroxy-n-propyl)-N-methoxyacetyl-2-methyl-6-azido-anilin,
N-(1'-Methoxycarbonyl-ethyl)-N-methylaminosulfonyloxyacetyl-2,6-dimethyl-3-azido-anilin,
N-(1'-Methoxycarbonyl-3-hydroxy-n-propyl)-N-cyclopropylcarbonyl-2-methyl-6-azido-anilin,
N-(1'-Methoxycarbonyl-ethyl)-N-methoxyacetyl-2,3-dimethyl-6-azido-anilin,
N-(1'-Cyanoethyl)-N-methoxyacetyl-2-methyl-6-azido-anilin,
N-(1'-Methyl-2'-dimethoxy-ethyl)-N-methoxyacetyl-2,6-dimethyl-3-azido-anilin,
N-(2'-Methyl-3'-methoximino-n-propyl)-N-methoxy-acetyl-2-methyl-6-azido-anilin,
N-(Tetrahydro-2'-on-fur-3'-yl)-N-methoxyacetyl-2,3-dimethyl-4-azido-α-naphthylamin,
N-(1'Cyano-ethyl)-N-methoxyacetyl-2,3-dimethyl-4-azido-α-naphthylamin,
N-(1'-Cyanoethyl)-N-(2-tetrahydrofuryl-carbonyl)-2,3-dimethyl-4-azido-α-naphthylamin.

Phenylacylalanine mit mikrobiziden Wirkungen sowie ihre Herstellung sind in der Literatur schon seit einiger Zeit bekannt. In diesem Zusammenhang sind beispielsweise folgende Veröffentlichungen zu nennen : DE-OS 2 847 287, 2 908 739, 2 920 435, 2 922 759, 2 927 461 und 2 929 525 sowie EP 5591, 12 703 und 17 850.

Es sind in der FR-PS 2,442,226 schon strukturähnliche Mikrobizide vorgeschlagen worden, die im aromatischen Ring Ar eine verätherte Hydroxymethylgruppe besitzen. Es sind ferner in der FR-PS 2,267,042 ähnliche Mikrobizide vorgeschlagen worden, die einen kernhalogenierten Substituenten besitzen.

Die Verbindungen der vorliegenden Erfindung zeichnen sich durch eine besonders gleichmässige Fungizid-Wirkung und durch besonders pflanzenschonende Eigenschaften aus.

Die Verbindungen der Formel I können in bekannter Weise beispielsweise, wie nachfolgend beschrieben, hergestellt werden. In den Formeln II-VII haben die Symbole Ar, Y und $R_1$—$R_6$ die unter Formel I angegebenen Bedeutungen. Das Symbol A steht für eine der üblichen Abgangsgruppen, wie beispielsweise für Alkoxy, Benzolsulfonyl-oxy, p-Brombenzolsulfonyloxy, p-Tosyloxy, Trifluoracetyloxy, Nieder-alkylsulfonyloxy wie Mesyloxy oder insbesondere für Halogen wie Fluor, Chlor, Brom oder Jod, bevorzugt Chlor oder Brom.

Herstellungsverfahren

Wirkstoffe der Formel I können hergestellt werden :

A) durch Acylierung von Verbindungen der Formel II

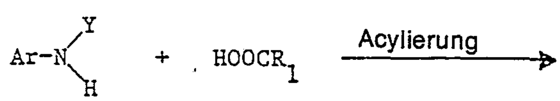


(II) (III) (I)

mit Carbonsäuren der Formel III oder ihren reaktionsfähigen Derivaten oder

B) durch Umsetzung von Verbindungen der Formel IV

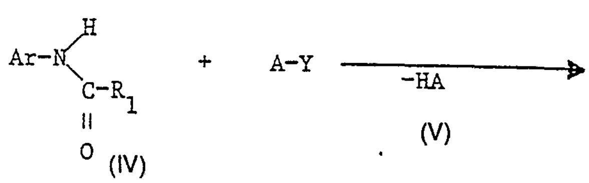


(IV) (V) (I)

mit Verbindungen der Formel V, worin A eine übliche Abgangsgruppe bedeutet, oder

C) für den Fall, dass $R_3$ die $N_3$-Gruppe darstellt, durch Bildung der Diazoniumsalze der Verbindungen der Formel VI und anschliessende variable Umsetzung wie folgt :

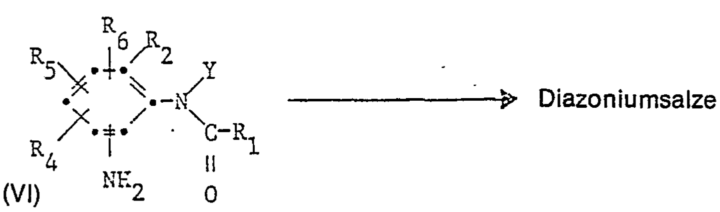


(VI)

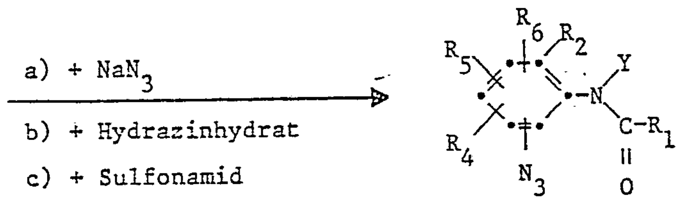


(Ia)

oder

D) für den Fall, dass $R_3$ die $HOCH_2$-Gruppe darstellt, durch Umsetzung von Verbindungen der Formel VII mit NaOH in Aceton

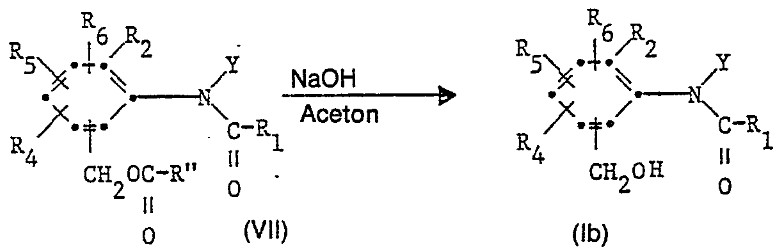


(VII) (Ib)

worin R" $C_1$-$C_4$-Alkyl bedeutet, oder

E) für den Fall, dass $R_3$ eine der Gruppen

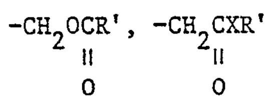

oder

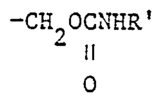

darstellt, durch folgende Umsetzungen von Verbindungen

der Formel Ib

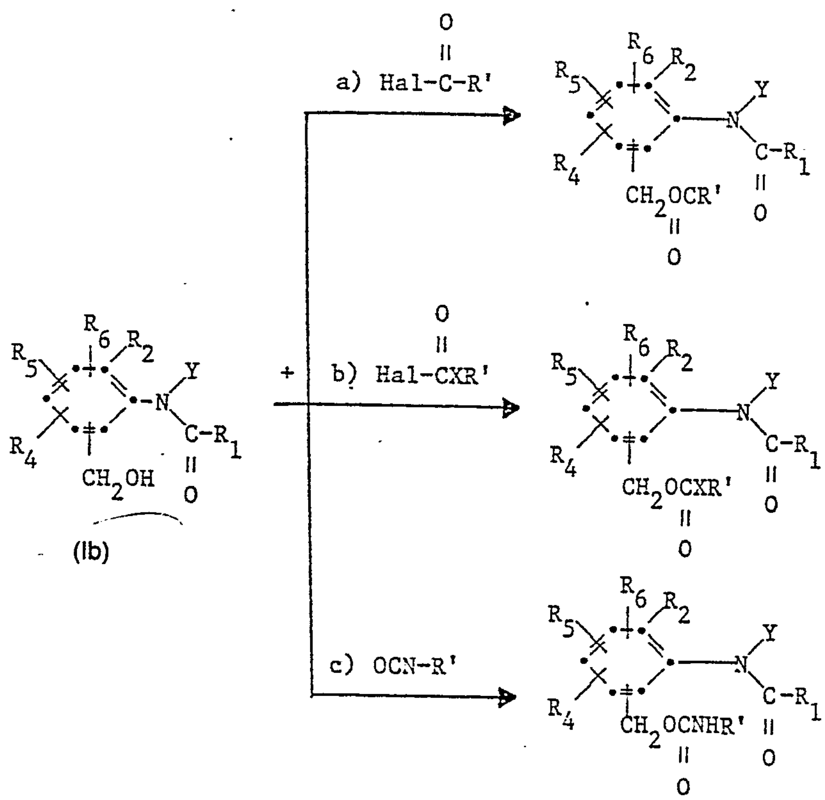


(Ib) (Ic) (Id) (Ie)

worin Hal Halogen, vorzugsweise Chlor oder Brom, bedeutet. Bei den Umsetzungen (a) und (b) arbeitet man vorzugsweise in Gegenwart eines säurebindenden Mittels.

Für die angeführten Herstellungsverfahren sind folgende Reaktionsbedingungen vorteilhaft :

Verfahren A :

Es kann hierbei vorzugsweise ein reaktionsfähiges Derivat einer Verbindung der Formel III, wie z. B. das Säurehalogenid, Säureanhydrid oder der Ester eingesetzt werden. Besonders geeignet ist das Säurechlorid oder das Säurebromid.

Die Reaktionstemperaturen liegen zwischen 0° und 180 °C, vorzugsweise 0° und 150 °C bzw. am Siedepunkt des Lösungsmittels bzw. Lösungsmittelgemisches. In manchen Fällen ist die Verwendung von säurebindenden Mitteln bzw. Kondensationsmitteln vorteilhaft. Als solche kommen organische und anorganische Basen in Betracht, z. B. tertiäre Amine wie Trialkylamine (Trimethylamin, Triethylamin, Tripropylamin usw.), Pyridin und Pyridinbasen (4-Dimethylaminopyridin, 4-Pyrrolidylaminopyridin usw.), Oxide und Hydroxide, Carbonate und Hydrogencarbonate von Alkali- und Erdalkalimetallen sowie Alkaliacetate.

Entstehender Halogenwasserstoff kann in manchen Fällen auch mittels Durchleiten von Inertgas, z. B. Stickstoff aus dem Reaktionsgemisch vertrieben werden.

Die N-Acylierung kann in Anwesenheit von reaktionsinerten Lösungs- oder Verdünnungsmitteln durchgeführt werden. In Frage kommen beispielsweise aliphatische und aromatische Kohlenwasserstoffe wie Chlorbenzol, Methylenchlorid, Ethylenchlorid, Chloroform, Tetrachlorkohlenstoff, Tetrachlorethylen ; Ether und etherartige Verbindungen wie Dialkylether (Diethylether, Diisopropylether, tert.-Butylmethylether usw.), Anisol, Dioxan, Tetrahydrofuran ; Nitrile wie Acetonitril, Propionitril, N,N-dialkylierte Amide wie Dimethylformamid ; Dimethylsulfoxid ; Ketone wie Aceton, Diethylketon, Methylethylketon und Gemische solcher Lösungsmittel untereinander. In manchen Fällen kann das Acylierungsmittel selbst als Lösungsmittel dienen.

Bei der Acylierung kann die Gegenwart eines Reaktionskatalysators wie Dimethylformamid von Vorteil sein.

Verfahren B :

Hierbei steht der Substituent A in Formel V für eine der üblichen Abgangsgruppen, beispielsweise für Alkoxy, Benzolsulfonyloxy, p-Brombenzolsulfonyloxy, p-Tosyloxy, Trifluoracetyloxy, Niederalkylsulfonyloxy wie Mesyloxy oder insbesondere für ein Halogen wie Fluor, Chlor, Brom oder Jod, bevorzugt für Chlor oder Brom. Bei dieser Variante führt man zweckmässigerweise die Ausgangsverbindung der Formel IV zuerst mit Butyl-Lithium oder mit Natriumhydrid in das entsprechende Alkalisalz über oder aber man arbeitet in Gegenwart eines säurebindenden Mittels analog zu Variante A, vorzugsweise unter Zusatz katalytischer Mengen Alkalijodid.

Weitere Methoden zur Synthese von Verbindungen der Formel I sind beispielsweise an folgenden Stellen in der Literatur angegeben : Houben-Weyl Bd. 11/2, S.3 und Bd. 8, S.653.

Die Ausgangsstoffe für die Herstellung der erfindungsgemässen Verbindungen der Formel I sind teils neu, teils bekannt. Die neuen Ausgangsstoffe lassen sich nach bekannten Methoden in analoger Weise herstellen. Solche Referenzmethoden sind beispielsweise an folgenden Stellen in der Literatur beschrieben :

J. Org. Chem. *30,* 4101 (1965) ; Tetrahedron *1967,* 487 ; Tetrahedron *1967,* 493 ; Org. Syntheses III, 650 ; Org. Syntheses III, 652 ; Org. Syntheses III, 710 ; J. Org. Chem. *34,* 3430 (1969).

Auf einige Herstellungsmethoden von neuen Ausgangsstoffen sei detailliert hingewiesen. So können die Ausgangsstoffe für Verbindungen der Formel I, in denen $R_3$ einen $N_3$-Rest bedeutet, wie folgt hergestellt werden :

Nitrierung von Verbindungen der Formel VIII mit einem reaktionsfähigen Nitrierungsreagens zu Verbindungen der Formel IX und anschliessender Hydrierung der Nitro-Verbindungen mit einem üblichen Hydrierungsmittel zu Verbindungen der Formel VI :

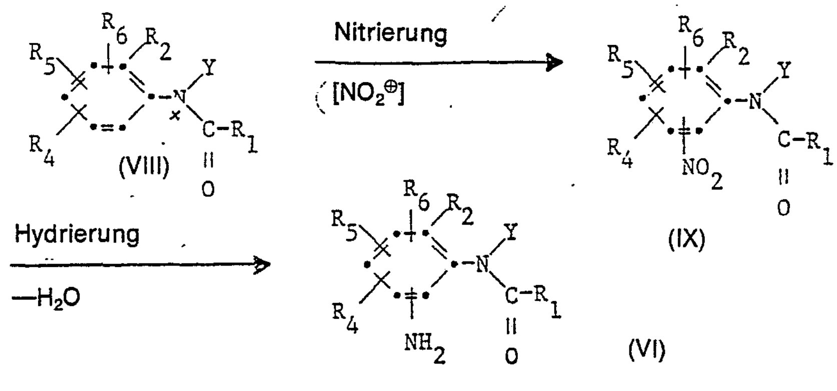


(VIII) (IX) (VI)

Die Verbindungen der Formel I besitzen teilweise im Molekülteil Y mindestens ein asymmetrisches Kohlenstoffatom. Hergestellte Racemate können nach bekannten Racematspaltungsmethoden in die optischen Antipoden zerlegt werden. Die optischen Antipoden der Formel I besitzen unterschiedliche mikrobizide Wirkungen.

Je nach Art der Substitution können weitere asymmetrische Kohlenstoffatome im Molekül der Verbindungen der Formel I vorhanden sein.

Unabhängig vor der beschriebenen optischen Isomerie wird bei den Verbindungen der Formel I eine Atropisomerie um die Aryl—N$\lesssim$-Achse beobachtet.

Sofern keine gezielte Synthese zur Isolierung reiner Isomeren durchgeführt wird, fällt normalerweise ein Produkt der Formel I als Gemisch dieser möglichen Isomeren an.

Es wurde überraschend gefunden, dass Verbindungen der Formel I ein für praktische Bedürfnisse sehr günstiges Mikrobizid-Spektrum aufweisen. Sie lassen sich beispielsweise zum Schutz von Kulturpflanzen verwenden.

Das Haupteinsatzgebiet von Verbindungen der Formel I liegt in der Bekämpfung von schädlichen Mikroorganismen, vor allem von phytopathogenen Pilzen. So besitzen die Verbindungen der Formel I eine für praktische Bedürfnisse sehr günstige kurative, präventive und systemische Wirkung zum Schutz von Kulturpflanzen ohne diese durch unerwünschte Nebenwirkungen zu beeinflussen. Kulturpflanzen seien im Rahmen vorliegender Erfindung beispielsweise : Getreide : (Weizen, Gerste, Roggen, Hafer, Reis) ; Rüben : (Zucker- und Futterrüben) ; Kern-, Stein- und Beerenobst : (Aepfel, Birnen, Pflaumen, Pfirsiche, Mandeln, Kirschen, Erd-, Him- und Brombeeren) ; Hülsenfrüchte : (Bohnen, Linsen, Erbsen, Soja) ; Oelkulturen : (Raps, Senf, Mohn, Oliven, Sonnenblumen, Kokos, Rizinus, Kakao, Erdnüsse) ; Gurkengewächse : Kürbis, Gurken, Melonen) ; Fasergewächse : (Baumwolle, Flachs, Hanf, Jute) ; Citrusfrüchte : (Orangen, Zitronen, Pampelmusen, Mandarinen) ; Gemüsesorten : (Spinat, Kopfsalat, Spargel, Kohlarten, Möhren, Zwiebeln, Tomaten, Kartoffeln, Paprika) oder Pflanzen wie Mais, Tabak, Nüsse, Kaffee, Zuckerrohr, Tee, Weinreben, Hopfen, Bananen- und Naturkautschukgewächse sowie Zierpflanzen.

Mit den Wirkstoffen der Formel I können an Pflanzen oder an Pflanzenteilen (Früchte, Blüten, Laubwerk, Stengel, Knollen, Wurzeln) dieser und verwandter Nutzkulturen die auftretenden Mikroorganismen eingedämmt oder vernichtet werden, wobei auch später zuwachsende Pflanzenteile von derartigen Mikroorganismen verschont bleiben. Die Wirkstoffe sind gegen die den folgenden Klassen angehörenden phytopathogenen Pilze wirksam : Gegen die der Klasse der Oomycetes angehörenden Peronosporales (Phytophthora, Pythium, Plasmopara) sowie gegen Ascomycetes wie Erysiphe- und Venturia-Erreger.

Ueberdies wirken die Verbindungen der Formel I systemisch. Sie können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden.

Die Erfindung betrifft somit ferner die Verwendung der Verbindungen der Formel I zur Bekämpfung phytopathogener Mikroorganismen bzw. zur präventiven Verhütung eines Befalls an Pflanzen.

Die Verbindungen der Formel I werden in unveränderter Form oder vorzugsweise zusammen mit den in der Formulierungstechnik üblichen Hilfsmitteln eingesetzt und werden daher z. B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z. B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Wirkstoffe der Formel I können gleichzeitig oder nacheinander mit weiteren Wirkstoffen auf die zu behandelnde Fläche oder Pflanze gegeben werden. Diese Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematozide Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Die Formulierungen d. h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Geeignete Träger und Zusätze können fest oder flüssig sein und entsprechen den in der Formulierungstechnik zweckdienlichen Stoffen, wie z. B. natürlichen oder regenerierten mineralischen Stoffen, Lösungs-, Dispergier-, Netz-, Haft-, Verdickungs-, Binde- oder Düngemitteln.

Als Lösungsmittel können in Frage kommen : Aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes

Kokosnussöl oder Sojaöl ; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden.

Die in der Formulierungstechnik gebräuchlichen Tenside sind u. a. in folgenden Publikationen beschrieben :

« Mc Cutcheon's Detergents and Emulsifiers Annual » MC Publishing Corp., Ringwood New Jersey, 1980.

Sisely and Wood, « Encyclopedia of Surface Active Agents », Chemical Publishing Co., Inc. New York, 1980.

Die pestiziden Zubereitungen enthalten in der Regel 0,1 bis 99 Gew.-%, insbesondere 0,1 bis 95 Gew.-%, Wirkstoff der Formel I, 1 bis 99 Gew.-% eines festen oder flüssigen Zusatzstoffes und 0 bis 25 Gew.-%, insbesondere 0,1 bis 25 Gew.-%, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die Erfindung umfasst auch Mittel, die als mindestens eine Aktivsubstanz eine Verbindung der Formel I enthalten, sowie die Verwendung der Mittel zur Bekämpfung und/oder Verhütung eines Befalls von Mikroorganismen. Darüber hinaus schliesst die vorliegende Erfindung auch die Herstellung der Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Bekämpfung von Mikroorganismen, das sich durch Applikation der Verbindungen der Formel I bzw. der neuen Mittel auszeichnet.

Die nachfolgenden Beispiele dienen zur näheren Erläuterung der Erfindung, ohne dieselbe einzuschränken. Die Temperaturen sind in Celsiusgraden angegeben. Prozente und Teile beziehen sich auf Gewicht. Sofern nicht anders vermerkt, ist bei der Nennung eines Wirkstoffs der Formel I stets das Isomeren-Gemisch gemeint.

## Beispiel 1

Herstellung von

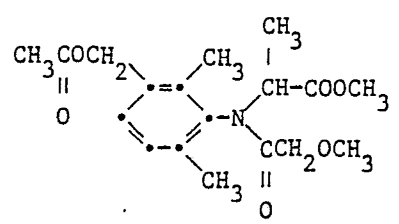

N-(1'-Methoxycarbonyl-ethyl)-N-methoxyacetyl-2,6-dimethyl-3-acetoxymethylanilin

35,0 g N-(1'-Methoxycarbonyl-ethyl)-2,6-dimethyl-3-acetoxymethylanilin und 13,3 g Soda wurden in 250 ml Toluol vorgelegt und langsam mit 20,0 g Methoxyessigsäurechlorid, gelöst in 50 ml Toluol, versetzt. Nach Abklingen der exothermen Reaktion wurde die Temperatur mittels Wasserbad 7 Stunden bei 45° gehalten. Das abgekühlte Reaktionsgemisch wurde filtriert und das Filtrat am Rotationsverdampfer eingeengt. Der ölige Rückstand wurde destilliert. Kp. 167-171° bei 0,07 mbar.

## Beispiel 2

Herstellung von

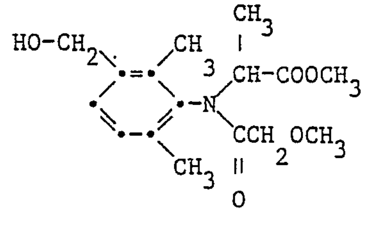

N-(1'-Methoxycarbonyl-ethyl)-N-methoxyacetyl-2,6-dimethyl-3-hydroxymethylanilin

23,0 g N-(1'-Methoxycarbonyl-ethyl)-N-methoxyacetyl-2,6-dimethyl-3-acetoxymethylanilin, 9,2 g 30 %ige Natronlauge und 100 ml Aceton wurden 3 Stunden bei Raumtemperatur gerührt, dann mit 20 ml Wasser versetzt und 2 mal mit je 50 ml Diethylether extrahiert. Die vereinigten Etherextrakte wurden über Natriumsulfat getrocknet und das Lösungsmittel verdampft. Das ölige Rohprodukt wurde über eine Kieselgelsäule mittels Ether/Chloroform (1 : 1) chromatographiert. Dabei wurde das Produkt als farbloses Oel erhalten ; $n_D^{14}$ = 1,535 2.

Beispiel 3

Herstellung von

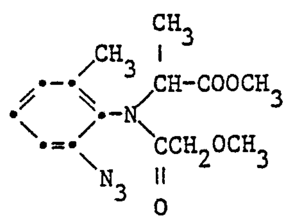

N-(1'-Methoxycarbonyl-ethyl)-N-methoxyacetyl-2-methyl-6-azidoanilin

10,0 g N-(1'-Methoxycarbonyl-ethyl)-N-methoxyacetyl-2-methyl-6-aminoanilin wurden mit 75 g Eis und 15 ml Salzsäure konz. bei 0 bis 5° verrührt, bis eine klare Lösung entstand. Bei 0 bis 5° wurden innert 10 Minuten 2,5 g Natriumnitrit, gelöst in 10 ml Wasser, zugetropft. Die rötlichbraune Lösung wurde 30 Minuten bei obiger Temperatur nachgerührt und dann mit 100 ml Ether versetzt. Darauf wurde innert 15 Minuten eine Lösung von 2,5 g Natriumazid in 10 ml Wasser bei 0 bis 5° zugetropft und die Temperatur dann innert 30 Minuten auf Raumtemperatur steigen gelassen. Die Reaktionsmischung wurde mit Ether extrahiert, die organische Phase mit Natriumhydrogenkarbonatwasser gewaschen, mit Magnesiumsulfat getrocknet und das Lösungsmittel verdampft. Das resultierende Oel wurde bei 40° unter Vakuum getrocknet. $n_D^{26}$ : 1.539 6. $n_D^{28,5}$ : 1.538 9.

Beispiel 4

Herstellung von

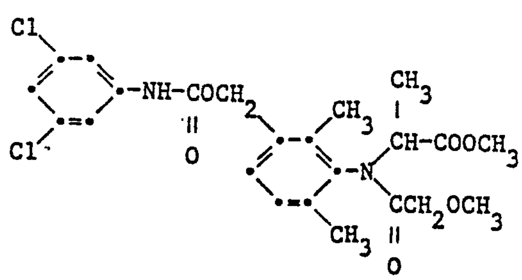

N-(1'-Methoxycarbonyl-ethyl)-N-methoxyacetyl-2,6-dimethyl-3-(3',5'-dichlorphenylcarbamoylmethyl)-anilin

16,2 g N-(1'-Methoxycarbonyl-ethyl)-2,6-dimethyl-3(3',5'-dichlorphenylcarbamoylmethyl)-anilin und 3,2 g Pyridin wurden in 120 ml Toluol vorgelegt und innert 15 Minuten mit 4,7 g Methoxyessigsäurechlorid, gelöst in 30 ml Toluol versetzt. Nach Abklingen der exothermen Reaktion wurde 4 Stunden bei Raumtemperatur nachgerührt und die Reaktionssuspension dann abgesaugt. Das gelbe Filtrat wurde mit Wasser gewaschen, über Natriumsulfat getrocknet und am Rotationsverdampfer eingeengt. Der feste Rückstand wurde mit Ether digeriert und die weissen Kristalle abgesaugt. Smp. 126-128°.

Auf analoge Weise, wie vorstehend beschrieben, werden die nachfolgenden Verbindungen der Formel I hergestellt.

(Siehe Tabelle 1.1 Seite 11 ff.)

Tabelle 1.1 Verbindungen der Formel $R'_4$, $R'_5$ = H

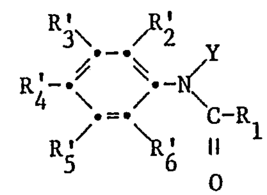

| Verb.Nr. | $R_1$ | $R'_2$ | $R'_3$ | $R'_6$ | Y | Physikal. Daten |
|---|---|---|---|---|---|---|
| 1.1.1. | $-CH_2OCH_3$ | $-CH_3$ | $-CH_2OCOCH_3$ | $-CH_3$ | $-CH-COOCH_3$<br>$CH_3$ | Kp. 167-171°C/ 0,07mbar |
| 1.1.2. | $-CH_2OCH_3$ | $-CH_3$ | $-CH_2OH$ | $-CH_3$ | $-CH-COOCH_3$<br>$CH_3$ | $n_D^{14}$ = 1,5352 |
| 1.1.3. | $-CH_2OCH_3$ | $-CH_3$ | H | $-N_3$ | $-CH-COOCH_3$<br>$CH_3$ | $n_D^{28,5}$ = 1,5389 |
| 1.1.4. | $-CH_2OCH_3$ | $-CH_3$ | $-CH_2OCO-NH$<br>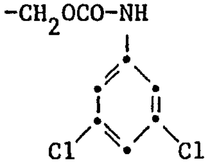 | $-CH_3$ | $-CH-COOCH_3$<br>$CH_3$ | Fp. 126 - 128°C |
| 1.1.5. | $-CH_2OCH_3$ | $-CH_3$ | $-N_3$ | $-CH_3$ | 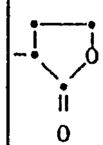 | Fp. 99 - 118°C |
| 1.1.6. | $-CH_2OCH_3$ | $-CH_3$ | H | $-CH_2OH$ | $-CH-COOCH_3$<br>$CH_3$ | viskos. Oel |

Tabelle 1.1 (Fortsetzung)

| Verb. Nr. | $R_1$ | $R'_2$ | $R'_3$ | $R'_6$ | Y | Physikal. Daten |
|---|---|---|---|---|---|---|
| 1.1.7. | 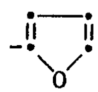 | $-CH_3$ | $-CH_2OCOCH_3$ | $-CH_3$ | $-CH-COOCH_3$<br>$CH_3$ | viskos. Oel |
| 1.1.8. | $-CH_2OCH_3$ | $-CH_3$ | $-CH_2OCONH$<br>$CH$<br>$CH_3$ $CH_3$ | $-CH_3$ | $-CH-COOCH_3$<br>$CH_3$ | viskos. Oel |
| 1.1.9. | $-CH_2OCH_3$ | $-CH_3$ | $-CH_2OCOOCH_3$ | $-CH_3$ | $-CH-COOCH_3$<br>$CH_3$ | $n_D^{14}$ = 1,5172 |
| 1.1.10. | 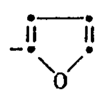 | $-CH_3$ | $-CH_2OH$ | $-CH_3$ | $-CH-COOCH_3$<br>$CH_3$ | Oel |
| 1.1.11. | $-CH_2OCH_3$ | $-CH_3$ | $-CH_3$ | $-N_3$ | $-CH-COOCH_3$<br>$CH_3$ | Fp. 80 - 82°C |
| 1.1.12. | $-CH_2OCH_3$ | $-CH_3$ | $-N_3$ | $-CH_3$ | $-CH-COOCH_3$<br>$CH_3$ | Fp. 88-93°C |

Tabelle 1.1 (Fortsetzung)

| Verb. Nr. | $R_1$ | $R'_2$ | $R'_3$ | $R'_6$ | Y | Physikal. Daten |
|---|---|---|---|---|---|---|
| 1.1.13. | $-CH_2OCH_3$ | $-CH_3$ | H | $-N_3$ | 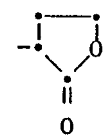 | |
| 1.1.14. | 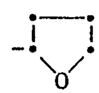 | $-CH_3$ | H | $-N_3$ | $-CH-COOCH_3$<br>\|<br>$CH_3$ | Oel |
| 1.1.15. | 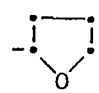 | $-CH_3$ | H | $-N_3$ | $CH_2CH_2OH$<br>\|<br>$-CH-COOCH_3$ | |
| 1.1.16. | 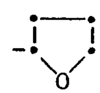 | $-CH_3$ | $-N_3$ | $-CH_3$ | 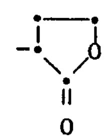 | |
| 1.1.17. | $-CH_2OCH_3$ | $-CH_3$ | $-N_3$ | $OCH_3$ | $-CH-COOCH_3$<br>\|<br>$CH_3$ | |
| 1.1.18. | $-CH_2OCH_3$ | $-CH_3$ | $-N_3$ | $-CH_3$ | $CH_2CH_2OH$<br>\|<br>$-CH-COOCH_3$ | |

Tabelle 1.1 (Fortsetzung)

| Verb. Nr. | $R_1$ | $R'_2$ | $R'_3$ | $R'_6$ | Y | Physikal. Daten |
|---|---|---|---|---|---|---|
| 1.1.19. | $-CH_2OCH_3$ | $-CH_3$ | H | $-N_3$ | 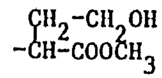 | gelbes Oel |
| 1.1.20. | 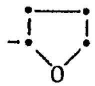 | $-CH_3$ | H | $-N_3$ | 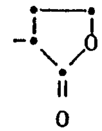 | |
| 1.1.21. | 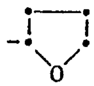 | $-CH_3$ | $-N_3$ | $-CH_3$ | 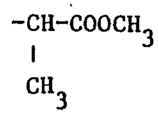 | |
| 1.1.22. | 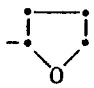 | $-CH_3$ | $-N_3$ | $-CH_3$ | 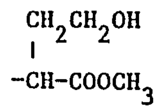 | |
| 1.1.23. | $-CH_2OCH_3$ | $-CH_3$ | $-N_3$ | $-OCH_3$ | 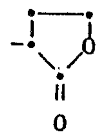 | |

Tabelle 1.1 (Fortsetzung)

| Verb. Nr. | $R_1$ | $R'_2$ | $R'_3$ | $R'_6$ | Y | Physikal. Daten |
|---|---|---|---|---|---|---|
| 1.1.24. | $-CH_2OCH_3$ | $-CH_3$ | $-N_3$ | $OCH_3$ | $CH_2-CH_2OH$ <br> $-CH-COOCH_3$ (CH₂–CH₂OH on CH) | |
| 1.1.25. | $-CH_2OSO_2NH$ (with $CH_3$ on NH) | $-CH_3$ | $-N_3$ | $-CH_3$ | $-CH-COOCH_3$ (with $CH_3$ on CH) | |
| 1.1.26. | $-CH_2OSO_2NH$ (with $CH_3$ on NH) | $-CH_3$ | $-N_3$ | $-CH_3$ | $CH_2-CH_2OH$ <br> $-CH-COOCH_3$ | |
| 1.1.27. | $-CH=CH_2$ | $-CH_3$ | H | $-N_3$ | 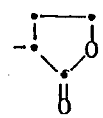 | |
| 1.1.28. | 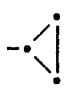 | $-CH_3$ | H | $-N_3$ | 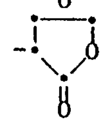 | |
| 1.1.29. | $-CH_2-$ 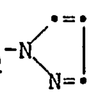 | $-CH_3$ | H | $-N_3$ | $-CH-COOCH_3$ (with $CH_3$ on CH) | |

Tabelle 1.1 (Fortsetzung)

| Verb.Nr. | $R_1$ | $R'_2$ | $R'_3$ | $R'_6$ | Y | Physikal. Daten |
|---|---|---|---|---|---|---|
| 1.1.30. | 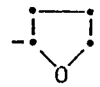 | $-CH_3$ | $-N_3$ | $-OCH_3$ | $-CH-COOCH_3$<br>$CH_3$ | |
| 1.1.31. | $-CH_2OSO_2NH$<br>$CH_3$ | $-CH_3$ | $-N_3$ | $-CH_3$ | 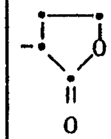 | |
| 1.1.32. | $-CH-CH=CH$<br>$CH_3$ | $-CH_3$ | H | $-N_3$ | $-CH-COOCH_3$<br>$CH_3$ | |
| 1.1.33. | 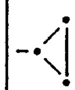 | $-CH_3$ | H | $-N_3$ | $-CH-COOCH_3$<br>$CH_3$ | |
| 1.1.34. | 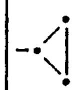 | $-CH_3$ | H | $-N_3$ | $CH_2-CH_2OH$<br>$-CH-COOCH_3$ | Fp. 87-92 $^{o}C$ |
| 1.1.35. | $-CH_2-$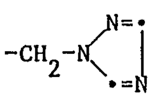 | $-CH_3$ | H | $-N_3$ | 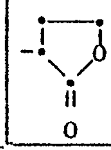 | |

Tabelle 1.1 (Fortsetzung)

| Verb. Nr. | $R_1$ | $R'_2$ | $R'_3$ | $R'_6$ | Y | Physikal. Daten |
|---|---|---|---|---|---|---|
| 1.1.36. | 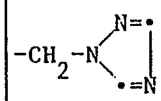 | $-CH_3$ | $-N_3$ | $-CH_3$ | $-CH(CH_3)-COOCH_3$ | |
| 1.1.37. | 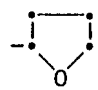 | $-CH_3$ | $-CH_2OCOCH_3$ | $-CH_3$ | 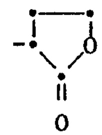 | |
| 1.1.38. | $-CH_2SO_2CH_3$ | $-CH_3$ | $-CH_2OCOCH_3$ | $-CH_3$ | $-CH(CH_3)-COOCH_3$ | |
| 1.1.39. | $-CH_2OC_2H_5$ | $-CH_3$ | H | $-N_3$ | $-CH(CH_3)-COOCH_3$ | |
| 1.1.40. | 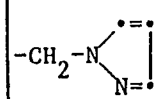 | $-CH_3$ | $-N_3$ | $-CH_3$ | 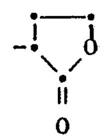 | |
| 1.1.41. | $-CH_2OSO_2NH(CH_3)$ | $-CH_3$ | $-CH_2OCOCH_3$ | $-CH_3$ | 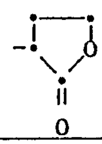 | |

Tabelle 1.1 (Fortsetzung)

| Verb. Nr. | $R_1$ | $R'_2$ | $R'_3$ | $R'_6$ | Y | Physikal. Daten |
|---|---|---|---|---|---|---|
| 1.1.42. | $-CH_2OCH_2-CH=CH_2$ | $-CH_3$ | $-CH_2OCOCH_3$ | $-CH_3$ | $-CH-COOCH_3$<br>\|<br>$CH_3$ | |
| 1.1.43. | $-CH_2OCH_2-CH=CH_2$ | $-CH_3$ | H | $-N_3$ | 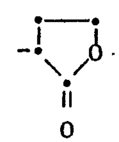 | |
| 1.1.44. | $-CH_2OCH_3$ | $-CH_3$ | $-N_3$ | $-CH_3$ | $-CH-COO-i-C_3H_7$<br>\|<br>$CH_3$ | |
| 1.1.45. | $-CH_2OCH_3$ | $-CH_3$ | $-CH_3$ | $-N_3$ | $CH_2CH_2OCH_3$<br>\|<br>$-CH-COOCH_3$ | |

Tabelle 1.1 (Fortsetzung)

| Verb. Nr. | $R_1$ | $R'_2$ | $R'_3$ | $R'_6$ | Y | Physikal. Daten |
|---|---|---|---|---|---|---|
| 1.1.46. | 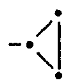 | $-CH_3$ | $-N_3$ | $-CH_3$ | $-CH-COOCH_3$<br>\|<br>$CH_3$ | Fp. 117-120°C |
| 1.1.47. | $-CH_2OC_2H_5$ | $-CH_3$ | $-N_3$ | $-CH_3$ | $-CH-COOCH_3$<br>\|<br>$CH_3$ | |
| 1.1.48. | $-CH_2OCH_3$ | $-Cl$ | H | $-N_3$ | $-CH-COOCH_3$<br>\|<br>$CH_3$ | |
| 1.1.49. | 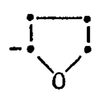 | $-CH_3$ | $-CH_3$ | $-N_3$ | 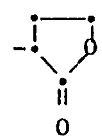 | |
| 1.1.50. | 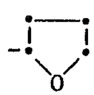 | $-CH_3$ | $-CH_3$ | $-N_3$ | $CH_2CH_2OH$<br>\|<br>$-CH-COOC_2H_5$ | |

Tabelle 1.1 (Fortsetzung)

| Verb. Nr. | $R_1$ | $R'_2$ | $R'_3$ | $R'_6$ | Y | Physikal. Daten |
|---|---|---|---|---|---|---|
| 1.1.51. | $-CH_2S-P(OC_2H_5)_2$<br>‖<br>S | $-CH_3$ | H | $-N_3$ | $-CH-COOCH_3$<br>\|<br>$CH_3$ | |
| 1.1.52. | $-CH_2S-P(OCH_3)_2$<br>‖<br>O | $-CH_3$ | H | $-N_3$ | 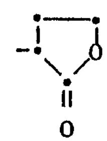 | |
| 1.1.53. | $-CH_2CH_2OCH_3$ | $-CH_3$ | $-N_3$ | $-CH_3$ | $-CH-COOCH_3$<br>\|<br>$CH_3$ | |
| 1.1.54. | $-CH_2OCH_3$ | $-CH_3$ | H | $-N_3$ | $-CH-CN$<br>\|<br>$CH_3$ | |
| 1.1.55. | 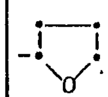 | $-CH_3$ | H | $-N_3$ | $-CH-CN$<br>\|<br>$CH_3$ | |

Tabelle 1.1 (Fortsetzung)

| Verb. Nr. | $R_1$ | $R_2'$ | $R_3'$ | $R_6'$ | Y | Physikal. Daten |
|---|---|---|---|---|---|---|
| 1.1.56. | $-CH_2S-P(OCH_3)_2$<br>‖<br>S | $-CH_3$ | H | $-N_3$ | $-CH-COOCH_3$<br>\|<br>$CH_3$ | |
| 1.1.57. | $-CH_2S-P(OCH_3)_2$<br>‖<br>S | $-CH_3$ | $-N_3$ | $-CH_3$ | $-CH-COOCH_3$<br>\|<br>$CH_3$ | |
| 1.1.58. | $-CH_2OCOCH_3$ | $-CH_3$ | H | $-N_3$ | $-CH-COOCH_3$<br>\|<br>$CH_3$ | |
| 1.1.59. | $-CH_2CH_2OCH_3$ | $-CH_3$ | $-N_3$ | $-CH_3$ | 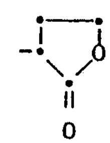 | |
| 1.1.60. | $-CH_2OCH_3$ | $-CH_3$ | $-N_3$ | $-CH_3$ | $-CH-CN$<br>\|<br>$CH_3$ | |
| 1.1.61. | $-CH_2OCH_3$ | $-CH_3$ | $-N_3$ | $-CH_3$ | $-CH-CH(OCH_3)_2$<br>\|<br>$CH_3$ | |

Tabelle 1.1 (Fortsetzung)

| Verb. Nr. | $R_1$ | $R'_2$ | $R'_3$ | $R'_6$ | Y | Physikal. Daten |
|---|---|---|---|---|---|---|
| 1.1.62. | $-CH_2OCH_3$ | $-CH_3$ | H | $-N_3$ | 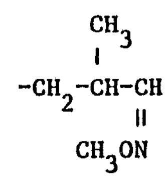 | |
| 1.1.63. | 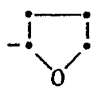 | $-CH_3$ | H | $-N_3$ | 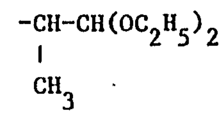 | |
| 1.1.64. | $-CH_2OCH_3$ | $-CH_3$ | H | $-N_3$ | $-CH_2-C{\equiv}CH$ | |
| 1.1.65. | $-CH_2OCH_3$ | $-CH_3$ | $-N_3$ | $-CH_3$ | $-CH_3-C{\equiv}CJ$ | |
| 1,1.66. | 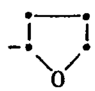 | $-CH_3$ | $-N_3$ | $-CH_3$ | 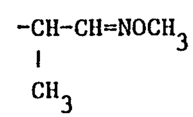 | |
| 1.1.67. | $-CH{=}CH-CH_3$ | $-CH_3$ | $-N_3$ | $-CH_3$ | 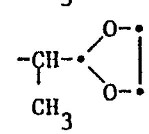 | |
| 1.1.68. | 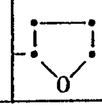 | $-CH_3$ | $-N_3$ | $-CH_3$ | $-CH_2-C{\equiv}C-CH_3$ | |

Tabelle 1.1 (Fortsetzung)

| Verb. Nr. | $R_1$ | $R'_2$ | $R'_3$ | $R'_6$ | Y | Physikal. Daten |
|---|---|---|---|---|---|---|
| 1.1.69. | $-CH_2OCH_3$ | $-CH_3$ | $-CH_2OH$ | $-CH_3$ | $-CH(CH_3)-CN$ | |
| 1.1.70. | $-CH_2OC_2H_5$ | $-CH_3$ | $-CH_2OH$ | $-CH_3$ | $-CH(CH_3)-COOCH_3$ | |
| 1.1.71. | $-CH_2OCH_3$ | $-CH_3$ | $-CH_2OH$ | $-CH_3$ | $-CH(CH_3)-CH(OCH_3)_2$ | |
| 1.1.72. | $-CH_2OCH_3$ | $-CH_3$ | $-CH_3$ | $-CH_2OH$ | $-CH(CH_3)-COOCH_3$ | |
| 1.1.73. | $-CH_2OCH_3$ | $-CH_3$ | $-CH_2OH$ | $-CH_3$ | 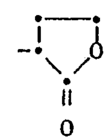 | |
| 1.1.74. | $-CH_2OCH_3$ | $-CH_3$ | $-CH_2OH$ | $-CH_3$ | $-CH(CH_3)-CH=N-OH$ | |

Tabelle 1.1 (Fortsetzung)

| Verb. Nr. | $R_1$ | $R'_2$ | $R'_3$ | $R'_6$ | Y | Physikal. Daten |
|---|---|---|---|---|---|---|
| 1.1.75. | $-CH_2OCH_3$ | $-CH_3$ | $-CH_2OH$ | $-CH_3$ | $-CH-CH=N$<br>\| \|<br>$CH_3$ $OCH_3$ | |
| 1.1.76. | $-CH_2OCH_3$ | $-Cl$ | H | $-CH_2OH$ | $-CH-COOCH_3$<br>\|<br>$CH_3$ | |

Tabelle 1.2 Verbindungen der Formel

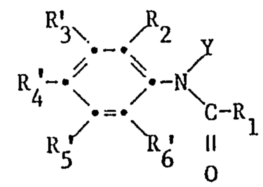

| Verb.Nr. | $R_1$ | $R'_2$ | $R'_3$ | $R'_4$ | $R'_5$ | $R'_6$ | Y | Physikal. Daten |
|---|---|---|---|---|---|---|---|---|
| 1.2.1. | $-CH_2OCH_3$ | $-CH_3$ | $-CH_3$ | $-N_3$ | $-CH_3$ | $-CH_3$ | $-CH-COOCH_3$<br>\|<br>$CH_3$ | |
| 1.2.2. | $-CH_2O-i-C_3H_7$ | $-CH_3$ | $-CH_3$ | $-N_3$ | $-CH_3$ | $-CH_3$ | $-CH-COOCH_3$<br>\|<br>$CH_3$ | |
| 1.2.3. | $-CH_2OCH_3$ | $-CH_3$ | $-CH_3$ | $-N_3$ | $-CH_3$ | $-CH_3$ | $-CH-COO-i-C_3H_7$<br>\|<br>$CH_3$ | |
| 1.2.4. | 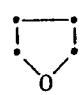 | $-CH_3$ | $-CH_3$ | $-N_3$ | $-CH_3$ | $-CH_3$ | 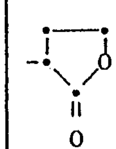 | |
| 1.2.5. | $-CH_2SO_2CH_3$ | $-CH_3$ | $-CH_3$ | $-N_3$ | $-CH_3$ | $-CH_3$ | $-CH-COOCH_3$<br>\|<br>$CH_3$ | |

Tabelle 2.1 Verbindungen der Formel $R'_4 = H$

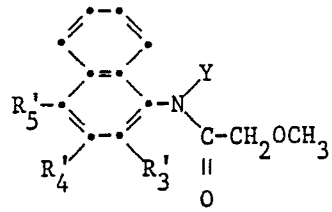

| Verb. Nr. | $R'_3$ | $R'_5$ | Y | physikal. Daten |
|---|---|---|---|---|
| 2.1.1. | $-N_3$ | H | $-CH-COOCH_3$<br>\|<br>$CH_3$ | Harz |
| 2.1.2. | $-CH_3$ | $-N_3$ | $-CH-COOCH_3$<br>\|<br>$CH_3$ | |
| 2.1.3. | $-N_3$ | H | 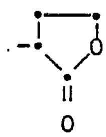 | |
| 2.1.4. | $-CH_2OH$ | H | $-CH-CN$<br>\|<br>$CH_3$ | |
| 2.1.5. | $-CH_3$ | $-N_3$ | $-CH-CH{=}NOH$<br>\|<br>$CH_3$ | |
| 2.1.6. | $-N_3$ | H | $-CH-C{\equiv}CH$<br>\|<br>$CH_3$ | |
| 2.1.7. | $-CH_3$ | $-N_3$ | 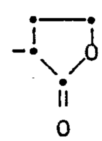 | |
| 2.1.8. | $-CH_2OCOCH_3$ | $-NO_2$ | $-CH-COOCH_3$<br>\|<br>$CH_3$ | |
| 2.1.9. | $-CH_3$ | $-N_3$ | $-CH-CN$<br>\|<br>$CH_3$ | |
| 2.1.10. | $-N_3$ | H | $-CH-CH(OCH_3)_2$<br>\|<br>$CH_3$ | |

Tabelle 2.2 Verbindungen der Formel

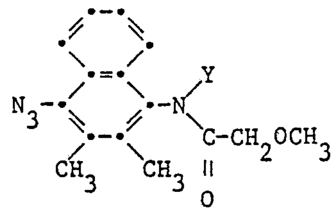

| Verb. Nr. | Y | Physikal. Daten |
|---|---|---|
| 2.2.1. | $-CH-COOCH_3$<br>$CH_3$ | |
| 2.2.2. | 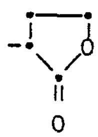 | Fp. 45-57°C |
| 2.2.3. | $-CH-CN$<br>$CH_3$ | |
| 2.2.4. | $-CH-CH=NOH$<br>$CH_3$ | |
| 2.2.5. | $-CH-CH(OCH_3)_2$<br>$CH_3$ | |
| 2.2.6. | $-CH-COOCH_3$<br>$(CH_2)_2OH$ | |
| 2.2.7. | $-CH-C{\equiv}CH$<br>$CH_3$ | |

Tabelle 2.3 Verbindungen der Formel

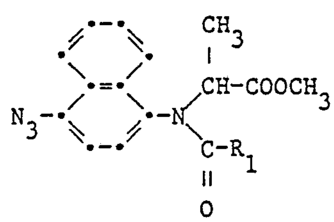

(Fortsetzung)

| Verb. Nr. | $R_1$ | Physikal. Daten |
|---|---|---|
| 2.3.1. | $-CH_2OC_2H_5$ | Fp. 93-96°C |
| 2.3.2. | 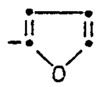 | |
| 2.3.3. | 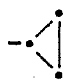 | |
| 2.3.4. | $-CH_2-$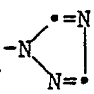 | |
| 2.3.5. | $-CH_2OSO_2NH-CH_3$ | |
| 2.3.6. | $-CH_2SO_2CH_3$ | |
| 2.3.7. | $-CH_2CH_2OCH_3$ | |

Tabelle 2.4 Verbindungen der Formel

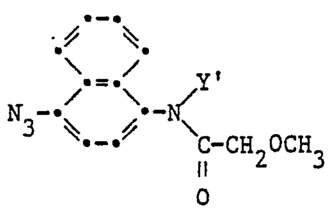

| Verb. Nr. | Y' | Physikal. Daten |
|---|---|---|
| 2.4.1. | $-CH_2CN$ | wachsartig |
| 2.4.2. | $-CH_2CH(OCH_3)_2$ | viskoses Oel |
| 2.4.3. | $-CH_2C{\equiv}CH$ | Fp. 57-63°C |

Biologische Beispiele

Die in den nachfolgenden Beispielen verwendeten Mittel werden, wie nachfolgend in den Formulierungsbeispielen beschrieben, formuliert.

Beispiel 5

Wirkung gegen Phytophthora infestans auf Tomatenpflanzen

a) Residual-protektive Wirkung

Tomatenpflanzen wurden nach 3-wöchiger Anzucht mit einer aus Spritzpulver des Wirkstoffes hergestellten Spritzbrühe (0,02 % Aktivsubstanz) besprüht. Nach 24 Stunden wurden die behandelten Pflanzen mit einer Sporangiensuspension des Pilzes infiziert. Die Beurteilung des Pilzbefalls erfolgte nach einer Inkubation der infizierten Pflanzen während 5 Tagen bei 90-100 % relativer Luftfeuchtigkeit und 20 °C.

Im Vergleich zu unbehandelten Kontrollpflanzen (100 % Befall) zeigten Pflanzen, die mit einer der Verbindungen Nr. 1.1.1, 1.1.2, 1.1.3, 1.1.4, 1.1.5, 1.1.7, 1.1.9, 1.1.10, 1.1.12, 1.1.14, 1.1.19, 1.1.34, 1.1.61, 1.1.62 und Nr. 2.1.1, 2.2.2 und 2.2.3 behandelt waren, weniger als 10 % Befall.

b) Systemische Wirkung

Zu Tomatenpflanzen wurde nach 3-wöchiger Anzucht eine aus Spritzpulver des Wirkstoffes hergestellte Spritzbrühe gegossen (0,006 % Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden wurden die behandelten Pflanzen mit einer Sporangiensuspension des Pilzes infiziert. Die Beurteilung des Pilzbefalls erfolgte nach einer Inkubation der infizierten Pflanzen während 5 Tagen bei 90-100 % relativer Luftfeuchtigkeit und 20 °C.

Unter anderen zeigten in obigem Versuch die Verbindungen Nr. 1.1.1, 1.1.2, 1.1.3, 1.1.5, 1.1.9, 1.1.10, 1.1.12, 1.1.14, 1.1.19, 1.1.34, 1.1.54, 1.1.61 und Nr. 2.1.1 und 2.2.2 eine sehr gute systemische Wirkung. Gegenüber unbehandelten Kontrollpflanzen (100 % Befall) bewirkten diese Verbindungen eine fast vollständige Unterdrückung des Pilzbefalls (0 bis 5 %).

c) Residual-kurative Wirkung

Tomatenpflanzen wurden nach 3-wöchiger Anzucht mit einer Sporangiensuspension des Pilzes infiziert. Nach einer Inkubation von 22 Stunden in einer Feuchtkammer bei 90-100 % relativer Luftfeuchtigkeit und 20 °C wurden die infizierten Pflanzen getrocknet und mit einer aus Spritzpulver des Wirkstoffes hergestellten Spritzbrühe (0,06 % Aktivsubstanz) besprüht. Nach dem Antrocknen des Spritzbelages wurden die behandelten Pflanzen wieder in die Feuchtkammer gebracht. Die Beurteilung des Pilzbefalls erfolgte 5 Tage nach der Infektion.

Im Vergleich zu unbehandelten aber infizierten Kontrollpflanzen (100 % Befall) zeigten Pflanzen, die mit einer der Verbindungen Nr. 1.1.1, 1.1.2, 1.1.3, 1.1.10, 1.1.12, 1.1.14, 1.1.19, 1.1.34, 1.1.61 und Nr. 2.2.1, 2.2.2 und 2.2.3 behandelt waren, weniger als 10 % Befall.

Beispiel 6

Residual protektive Wirkung gegen Venturia inaequalis auf Apfeltrieben

Apfelstecklinge mit 10-20 cm langen Frischtrieben wurden mit einer aus Spritzpulver des Wirkstoffes hergestellten Spritzbrühe (0,06 % Aktivsubstanz) besprüht. Nach 24 Stunden wurden die behandelten Pflanzen mit einer Konidiensuspension des Pilzes infiziert. Die Pflanzen wurden dann während 5 Tagen bei 90-100 % relativer Luftfeuchtigkeit inkubiert und während 10 weiteren Tagen in einem Gewächshaus bei 20-24 °C aufgestellt. Der Schorfbefall wurde 15 Tage nach der Infektion beurteilt. Unter anderen drängten die Verbindungen Nr. 1.1.1, 1.1.2, 1.1.10, 1.1.37, 1.1.42 und Nr. 2.1.1, 1.1.69, 2.1.4 und 2.1.8 den Krankheitsbefall auf 10 bis 15 % zurück.

Beispiel 7

Wirkung gegen Pythium debaryanum auf Rüben

a) Wirkung nach Bodenapplikation

Der Pilz wurde auf Karottenschnitzel-Nährlösung kultiviert und einer Erde-Sand-Mischung beigegeben. Die so infizierte Erde wurde in Blumentöpfe abgefüllt und mit Zuckerrübensamen besät. Gleich nach der Aussaat wurden die als Spritzpulver formulierten Versuchspräparate als wässrige Suspension über die Erde gegossen (20 ppm einer der Verbindungen aus den Tabellen 1 und 2 bezogen auf das Erdvolumen). Die Töpfe wurden darauf während 2-3 Wochen im Gewächshaus bei ca. 20 °C aufgestellt. Die Erde wurde dabei durch leichtes Ueberbrausen stets gleichmässig feucht gehalten. Bei der Auswertung des Tests wurde der Auflauf der Zuckerrübenpflanzen sowie der Anteil gesunder und kranker Pflanzen bestimmt.

b) Wirkung nach Beizapplikation

Der Pilz wurde auf Karottenschnitzel-Nährlösung kultiviert und einer Erde-Sand-Mischung beigegeben. Die so infizierte Erde wurde in Erdschalen abgefüllt und mit Zuckerrübensamen besät, die mit den als Beizpulver formulierten Versuchspräparaten gebeizt worden waren (0,06 % einer der Verbindungen aus den Tabellen 1 und 2).

Die besäten Töpfe wurden während 2-3 Wochen im Gewächshaus bei ca. 20 °C aufgestellt. Die Erde wurde dabei durch leichtes Ueberbrausen stets gleichmässig feucht gehalten. Bei der Auswertung wurde der Auflauf der Zuckerrübenpflanzen bestimmt.

Die Verbindungen Nr. 1.1.1, 1.1.2, 1.1.3, 1.1.5, 1.1.7, 1.1.9, 1.1.10, 1.1.12, 1.1.14, 1.1.19, 1.1.25, 1.1.34, 1.1.54, 1.1.61, 1.1.62 und Nr. 2.1.1, 2.2.2 und 2.2.3 zeigten in beiden Versuchen a) und b) gegen Pythium-Erreger (über 90 % aufgelaufene Pflanzen) volle Wirkung. Die Pflanzen hatten ein gesundes Aussehen.

Eine gleich gute Wirkung wurde bei analogen Versuchen gegen Pythium-Erreger auf Mais, erzielt.

## Beispiel 8

### Wirkung gegen Podosphaera leucotricha auf Apfelsämlingen Residual-protektive Wirkung

Apfelsämlinge mit ca. 5 entwickelten Blättern wurden mit einer aus Spritzpulver des Wirkstoffes hergestellten Spritzbrühe (0,06 % Aktivsubstanz) besprüht. Nach 24 Stunden wurden die behandelten Pflanzen mit einer Konidiensuspension des Pilzes infiziert und in einer Klimakammer bei 70 % relativer Luftfeuchtigkeit und 20° aufgestellt. Die Beurteilung des Pilzbefalls erfolgte 12 Tage nach der Infektion.

Im Vergleich zu unbehandelten aber infizierten Apfelsämlingen (100 % Befall), zeigten Sämlinge, die mit einer der Verbindungen Nr. 1.1.1, 1.1.2, 1.1.3, 1.1.4, 1.1.5, 1.1.7, 1.1.9, 1.1.10, 1.1.14, 1.1.19, 1.1.25, 1.1.34, 1.1.54, 1.1.61, 1.1.62 und Nr. 2.1.1, 2.2.2 und 2.2.3 behandelt waren, weniger als 10 % Befall.

### Formulierungsbeispiele für flüssige Wirkstoffe der Formel I (% = Gewichtsprozent)

## Beispiel 9

### Emulsions-Konzentrate

| | a) | b) | c) |
|---|---|---|---|
| Wirkstoff aus Tabellen 1.1-2.4 | 25 % | 40 % | 50 % |
| Ca-Dodecylbenzolsulfonat | 5 % | 8 % | 6 % |
| Ricinusöl-polyäthylenglykoläther (36 Mol AeO) | 5 % | — | — |
| Tributylphenoyl-polyäthylenglykoläther (30 Mol AeO) | — | 12 % | 4 % |
| Cyclohexanon | — | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

## Beispiel 10

### Lösungen

| | a) | b) | c) | d) |
|---|---|---|---|---|
| Wirkstoff aus Tabellen 1.1-2.4 | 80 % | 10 % | 5 % | 95 % |
| Aethylenglykol-monomethyläther | 20 % | — | — | — |
| Polyäthylenglykol MG 400 | — | 70 % | — | — |
| N-Methyl-2-pyrrolidon | — | 20 % | — | — |
| Epoxydiertes Kokosnussöl | — | — | 1 % | 5 % |
| Benzin (Siedegrenzen 160-190 °C) | — | — | 94 % | — |

Die Lösungen sind zur Anwendung in Form kleinster Tropfen geeignet.

(Siehe Beispiel 11 Seite 31 f.)

Beispiel 11

Granulate

| | a) | b) |
|---|---|---|
| Wirkstoff aus Tabellen 1.1-2.4 | 5 % | 10 % |
| Kaolin | 94 % | — |
| Hochdisperse Kieselsäure | 1 % | — |
| Attapulgit | — | 90 % |

Der Wirkstoff wird in Methylenchlorid gelöst, auf den Träger aufgesprüht und das Lösungsmittel anschliessend im Vakuum abgedampft.

Beispiel 12

Stäubemittel

| | a) | b |
|---|---|---|
| Wirkstoff aus Tabellen 1.1-2.4 | 2 % | 5 % |
| Hochdisperse Kieselsäure | 1 % | 5 % |
| Talkum | 97 % | — |
| Kaolin | — | 90 % |

Durch inniges Vermischen der Trägerstoffe mit dem Wirkstoff erhält man gebrauchsfertige Stäubemittel.

Formulierungsbeispiele für feste Wirkstoffe der Formel I (% = Gewichtsprozent)

Beispiel 13

Spritzpulver

| | a) | b) | c) |
|---|---|---|---|
| Wirkstoff aus Tabellen 1.1-2.4 | 25 % | 50 % | 75 % |
| Na-Ligninsulfonat | 5 % | 5 % | — |
| Na Laurylsulfat | 3 % | — | 5 % |
| Na-Diisobutylnaphthalinsulfonat | — | 6 % | 10 % |
| Octylphenolpolyäthylenglykoläther (7-8 Mol AeO) | — | 2 % | — |
| Hochdisperse Kieselsäure | 5 % | 10 % | 10 % |
| Kaolin | 62 % | 27 % | — |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

Beispiel 14

Emulsions-Konzentrat

| | |
|---|---|
| Wirkstoff aus Tabellen 1.1-2.4 | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

Beispiel 15

Stäubemittel

| | a) | b) |
|---|---|---|
| Wirkstoff aus Tabellen 1.1-2.4 | 5 % | 8 % |
| Talkum | 95 % | — |
| Kaolin | — | 92 % |

Man erhält anwendungsfertige Stäubemittel, indem der Wirkstoff mit den Träger vermischt und auf einer geeigneten Mühle vermahlen wird.

Beispiel 16

Extruder Granulat

| | |
|---|---|
| Wirkstoff aus Tabellen 1.1-2.4 | 10 |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

Beispiel 17

Umhüllungs-Granulat

| | |
|---|---|
| Wirkstoff aus Tabellen 1.1-2.4 | 3 % |
| Polyethylenglykol (MG 200) | 3 % |
| Kaolin | 94 % |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

Beispiel 18

Suspensions-Konzentrat

| | |
|---|---|
| Wirkstoff aus Tabellen 1.1-2.4 | 40 % |
| Ethylenglykol | 10 % |
| Nonylphenyolpolyethylenglykolether (15 Mol AeO) | 6 % |
| Na-Ligninsulfonat | 10 % |
| Carboxymethylcellulose | 1 % |
| 37 %ige wässrige Formaldehyd-Lösung | 0,2 % |
| Silikonöl in Form einer 75 %igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

**Patentansprüche** (für die Vertragsstaaten : BE, CH, DE, FR, GB, IT, LI, LU, NL, SE)

1. Verbindungen der allgemeinen Formel I

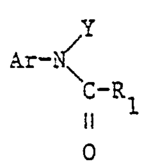

(I)

worin Ar einen Rest der Formeln

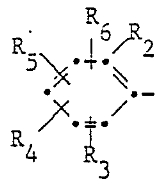

oder

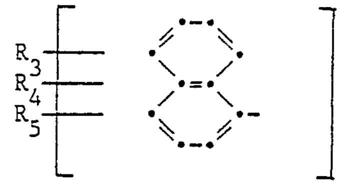

,

$R_1$ gegebenenfalls durch Halogen substituiertes 2-Furyl, 2-Tetrahydrofuryl, $C_2$-$C_4$-Alkenyl, Cyclopropyl, β-($C_1$-$C_4$)-Alkoxyethyl oder den Rest —$CH_2Z$ bedeutet, wobei Z für

a) —OH

b) —1H-1,2,4-Triazolyl, 1-Imidazolyl, 1-Pyrazolyl,

c) —$S(O)_n$—$R_{13}$ mit $R_{13}$ gleich $C_1$-$C_4$-Alkyl und n gleich 1 oder 2,

d) —X—$R_{14}$ mit X gleich Sauerstoff oder Schwefel und $R_{14}$ gleich gegebenenfalls durch $C_1$-$C_2$-Alkoxy substituiertes $C_1$-$C_6$-Alkyl, $C_3$-$C_4$-Alkenyl oder $C_3$-$C_4$-Alkinyl,

e) —$OSO_2$—$R_{15}$ mit $R_{15}$ gleich $C_1$-$C_4$ Alkyl, Mono- oder Di-($C_1$-$C_3$)-Alkylamin,

f)

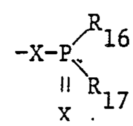

mit X gleich Sauerstoff oder Schwefel, $R_{16}$ gleich $C_1$-$C_4$-Alkyl, $C_1$-$C_4$-Alkoxy oder $C_1$-$C_4$-Alkylamino und $R_{17}$ gleich $C_1$-$C_4$-Alkoxy oder $C_1$-$C_4$-Alkylamino,

oder

g) $-O-\underset{\underset{O}{\|}}{C}-R_{18}$ mit $R_{18}$ gleich gegebenenfalls durch $C_1$-$C_2$-Alkoxy substituiertes $C_1$-$C_3$-Alkyl steht,

$R_2$ $C_1$-$C_3$-Alkyl, $C_1$-$C_3$-Alkoxy oder Halogen,

$R_3$

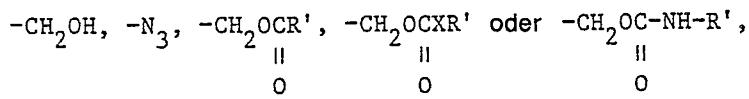

oder

wobei R′ gegebenenfalls durch Halogen oder $C_1$-$C_3$-Alkoxy substituiertes $C_1$-$C_6$-Alkyl, $C_2$-$C_4$-Alkenyl oder Phenyl darstellt, und X für Sauerstoff oder Schwefel steht,

$R_4$ Wasserstoff, $C_1$-$C_3$-Alkyl, $C_1$-$C_3$-Alkoxy, Halogen, —$NO_2$ oder —$NH_2$,

$R_5$ Wasserstoff oder $C_1$-$C_3$-Alkyl,

$R_6$ Wasserstoff oder $C_1$-$C_3$-Alkyl,

Y

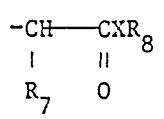

,

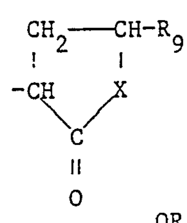

,

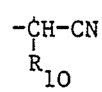

,

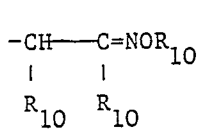

,

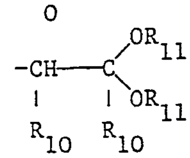

oder

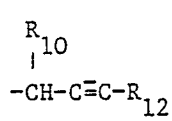

darstellt,

wobei

$R_7$ für Wasserstoff, gegebenenfalls durch Halogen, —OH, —$OC_1$-$C_3$-Alkyl oder —$O\underset{\overset{\|}{O}}{C}NH$-$C_1$-$C_3$-Alkyl substituiertes $C_1$-$C_2$-Alkyl,

$R_8$ für gegebenenfalls durch $C_1$-$C_2$-Alkoxy substituiertes $C_1$-$C_4$-Alkyl,

$R_9$ für Wasserstoff oder Methyl,

$R_{10}$ für Wasserstoff oder $C_1$-$C_3$-Alkyl,

$R_{11}$ für $C_1$-$C_4$-Alkyl, wobei $R_{11}$ zusammen eine ein- oder mehrfach durch $C_1$-$C_3$-Alkyl substituierte $C_2$-$C_3$-Alkylenbrücke darstellen können,

$R_{12}$ für Wasserstoff, Halogen, $C_1$-$C_2$-Alkyl oder Phenyl stehen und

X Sauerstoff oder Schwefel darstellt.

2. Verbindungen der Formel I gemäss Anspruch 1, worin

$R_1$ 2-Furyl, 2-Tetrahydrofuryl, $C_2$-$C_4$-Alkenyl, Cyclopropyl, β-($C_1$-$C_2$)-Alkoxyethyl oder $CH_2$—Z, wobei Z für

a) —OH

b) 1H-1,2,4-Triazolyl, 1-Imidazolyl,

c) —$S(O)_n$—$R_{13}$ mit $R_{13}$ gleich $C_1$-$C_2$-Alkyl und n gleich 2,

d) —X'—$R_{14}$ mit X' gleich Sauerstoff oder Schwefel und $R_{14}$ gleich gegebenenfalls durch $C_1$-$C_2$-Alkoxy substituiertes $C_1$-$C_6$-Alkyl, $C_3$-$C_4$-Alkenyl oder $C_3$-$C_4$-Alkinyl,

e) —$OSO_2$—$R_{15}$ mit $R_{15}$ gleich $C_1$-$C_2$-Alkyl, Mono- oder Di-($C_1$-$C_2$)-Alkylamin

f)

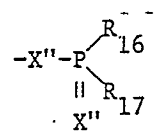

mit X" gleich Schwefel,

$R_{16}$ gleich $C_1$-$C_2$-Alkyl, $C_1$-$C_4$-Alkoxy, $C_1$-$C_2$-Alkylamino und $R_{17}$ gleich $C_1$-$C_4$-Alkoxy oder $C_1$-$C_4$-Alkylamino, oder

g)

$$-O-\underset{\overset{\|}{O}}{C}-R_{18}$$

mit $R_{18}$ gleich gegebenenfalls durch $C_1$-$C_2$-Alkoxy substituiertes $C_1$-$C_2$-Alkyl steht,

$R_2$ $C_1$-$C_2$-Alkyl, $C_1$-$C_2$-Alkoxy oder Halogen,

$R_3$ $-CH_2OH$, $-N_3$, $-CH_2O\underset{\overset{\|}{O}}{C}-R'$, $-CH_2O\underset{\overset{\|}{O}}{C}X'''-R'$ oder $-CH_2O\underset{\overset{\|}{O}}{C}NH-R'$

wobei

R' für gegebenenfalls durch Chlor oder Methoxy substituiertes $C_1$-$C_3$-Alkyl oder $C_2$-$C_3$-Alkenyl steht und X'" Sauerstoff oder Schwefel darstellt,

$R_4$ Wasserstoff, $C_1$-$C_2$-Alkyl, $C_1$-$C_2$-Alkoxy, $NO_2$ oder $NH_2$,

$R_5$ Wasserstoff oder $C_1$-$C_2$-Alkyl,

$R_6$ Wasserstoff oder $C_1$-$C_2$-Alkyl,

Y die unter Formel I angegebenen Bedeutungen hat, wobei

$R_7$ Wasserstoff oder gegebenenfalls durch Halogen, —OH, —O($C_1$-$C_2$)-Alkyl oder —$O\underset{\overset{\|}{O}}{C}NH$-($C_1$-$C_2$)-Alkyl substituiertes $C_1$-$C_2$-Alkyl,

$R_8$ gegebenenfalls durch $C_1$-$C_2$-Alkoxy substituiertes $C_1$-$C_3$-Alkyl,

$R_9$ Wasserstoff,

$R_{10}$ Wasserstoff oder $C_1$-$C_2$-Alkyl,

$R_{11}$ $C_1$-$C_2$-Alkyl, wobei $R_{11}$ zusammen auch $C_2$-$C_3$-Alkylen darstellen können,

$R_{12}$ Wasserstoff, Halogen oder $C_1$-$C_2$-Alkyl und

X Sauerstoff oder Schwefel bedeuten.

3. Verbindungen der Formel I gemäss Anspruch 1, worin

$R_1$ 2-Furyl, 2-Tetrahydrofuryl, $C_2$-$C_4$-Alkenyl, Cyclopropyl, 4-Methoxyethyl oder —$CH_2Z$, wobei Z für

a) 1H-1,2,4-Triazol,

b) —$S(O)_n$—$R_{13}$ mit $R_{13}$ gleich $CH_3$ und n gleich 2,

c) —$X'^v$—$R_{14}$ mit $X'^v$ gleich Sauerstoff und $R_{14}$ gleich gegebenenfalls durch $C_1$-$C_2$-Alkoxy substituiertes $C_1$-$C_4$-Alkyl, $C_3$-$C_4$-Alkenyl oder $C_3$-$C_4$-Alkinyl,

d) $—OSO_2—R_{15}$ mit $R_{15}$ gleich Mono-($C_1$-$C_3$)-Alkylamin,

e)

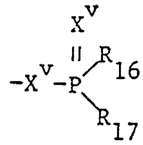

mit $X^v$ gleich Schwefel, $R_{16}$ und $R_{17}$ unabhängig voneinander gleich $C_1$-$C_3$-Alkoxy oder

f) $—\underset{\underset{O}{\|}}{OC}-R_{18}$ mit $R_{18}$ gleich $C_1$-$C_3$-Alkyl steht,

$R_2$ Methyl, Methoxy oder Chlor,

$R_3$ $—CH_2OH$, $—N_3$ oder $—CH_2O\underset{\underset{O}{\|}}{C}CH_3$

$R_4$ Wasserstoff, Methyl, Methoxy, $NO_2$ oder $NH_2$,

$R_5$ Wasserstoff oder Methyl,

$R_6$ Wasserstoff oder Methyl,

Y die unter Formel I angegebenen Bedeutungen hat, wobei

$R_7$ gegebenenfalls durch Chlor, —OH, $—OC_1$-$C_2$-Alkyl oder $—O\underset{\underset{O}{\|}}{C}NH$-$C_1$-$C_2$-Alkyl substituiertes $C_1$-$C_2$-Alkyl

$R_8$ $C_1$-$C_3$-Alkyl,

$R_9$ Wasserstoff,

$R_{10}$ Methyl,

$R_{11}$ $C_1$-$C_2$-Alkyl, wobei $R_{11}$ zusammen auch $C_2$-$C_3$-Alkylen darstellen kann,

$R_{12}$ Wasserstoff, Brom, Jod oder $C_1$-$C_2$-Alkyl und

X Sauerstoff bedeutet.

4. Verbindungen der Formel I gemäss Anspruch 3, worin $R_3$ die Azidogruppe ($-N_3$) bedeutet und die übrigen Reste $R_1$ bis $R_{12}$, X und Y die in Anspruch 3 angegebenen Bedeutungen besitzen.

5. Verbindungen der Formel I gemäss Anspruch 4, worin

Y die Reste $—CH(CH_3)COOCH_3$ oder

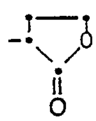

bedeutet,

$R_3$ die Azidogruppe $—(N_3)$ darstellt und die übrigen Reste $R_1$ bis $R_{12}$ und X die in Anspruch 3 angegebenen Bedeutungen besitzen.

6. Eine Verbindung der Formel I gemäss Anspruch 4, ausgewählt aus der Gruppe folgender Azido-Verbindungen :

N-(1'-Methoxycarbonyl-ethyl)-N-methoxyacetyl-2-methyl-6-azido-anilin,
N-(Tetrahydro-2'-on-fur-3'-yl)-N-methoxyacetyl-2,6-dimethyl-3-azido-anilin,
N-(1'-Methoxycarbonyl-ethyl)-N-methoxyacetyl-2,6-dimethyl-3-azido-anilin,
N-(1'-Methoxycarbonyl-ethyl)-N-(2-tetrahydrofuryl-carbonyl)-2-methyl-6-azido-anilin,
N-(1'-Methoxycarbonyl-3'-hydroxy-n-propyl)-N-methoxyacetyl-2-methyl-6-azido-anilin,
N-(1'-Methoxycarbonyl-ethyl)-N-methylaminosulfonyloxyacetyl-2,6-dimethyl-3-azido-anilin,
N-(1'-Methoxycarbonyl-3-hydroxy-n-propyl)-N-cyclopropylcarbonyl-2-methyl-6-azido-anilin,
N-(1'-Methoxycarbonyl-ethyl)-N-methoxyacetyl-2,3-dimethyl-6-azido-anilin,
N-(1'-Cyanoethyl)-N-methoxyacetyl-2-methyl-6-azido-anilin,
N-(1'-Methyl-2'-dimethoxy-ethyl)-N-methoxyacetyl-2,6-dimethyl-3-azido-anilin,
N-(2'-Methyl-3'-methoximino-n-propyl)-N-methoxy-acetyl-2-methyl-6-azido-anilin,
N-(Tetrahydro-2'-on-fur-3'-yl)-N-methoxyacetyl-2,3-dimethyl-4-azido-α-naphthylamin,
N-(1'-Cyano-ethyl)-N-methoxyacetyl-2,3-dimethyl-4-azido-α-naphthylamin,
N-(1'-Cyanoethyl)-N-(2-tetrahydrofuryl-carbonyl)-2,3-dimethyl-4-azido-α-naphthylamin.

7. Verfahren zur Herstellung von Verbindungen der Formel I nach Anspruch 1, gekennzeichnet

A) durch Acylierung von Verbindungen der Formel II

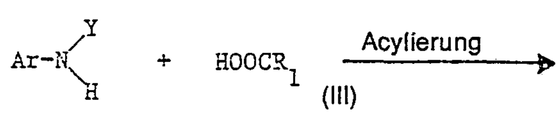


(II) (III) (I)

mit Carbonsäuren der Formel III oder ihren reaktionsfähigen Derivaten oder

B) durch Umsetzung von Verbindungen der Formel IV

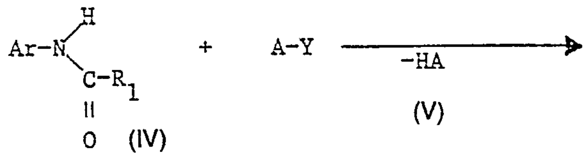


(IV) (V) (I)

mit Verbindungen der Formel V, worin A eine übliche Abgangsgruppe bedeutet.

8. Verfahren zur Herstellung von Verbindungen der Formel Ia, in denen $R_3$ die $N_3$-Gruppe bedeutet und die Reste $R_1$, $R_2$ und $R_4$ bis $R_{12}$ und X und Y die unter Formel I angegebenen Bedeutungen besitzen, durch Bildung der Diazoniumsalze der Verbindungen der Formel VI

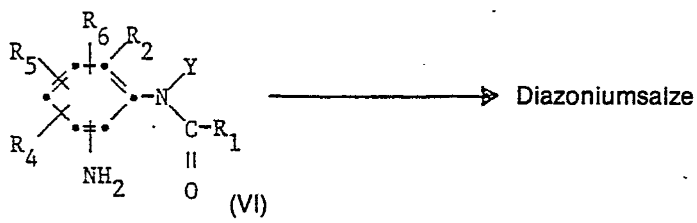


(VI)

und anschliessende variable Umsetzung

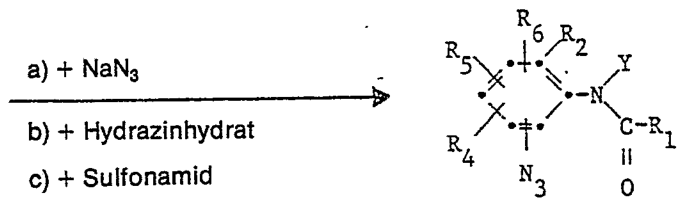


(Ia)

9. Verfahren zur Herstellung von Verbindungen der Formel Ib, in denen $R_3$ die $HOCH_2$-Gruppe bedeutet und die Reste $R_1$, $R_2$ und $R_4$ bis $R_{12}$ und X und Y die unter Formel I angegebenen Bedeutungen besitzen, durch Umsetzung von Verbindungen der Formel VII mit NaOH in Aceton

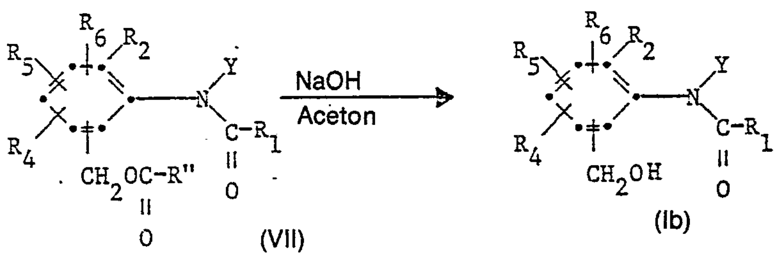


(VII) (Ib)

worin R" $C_1$-$C_4$-Alkyl bedeutet.

10. Verfahren zur Herstellung von Verbindungen der Formel Ic, Id und Ie, in denen $R_3$ eine der

Gruppen $-CH_2O\underset{\underset{O}{\|}}{C}R'$, $-CH_2\underset{\underset{O}{\|}}{C}XR'$ oder $-CH_2O\underset{\underset{O}{\|}}{C}NHR'$

darstellt und die Reste $R_1$, $R_2$ und $R_4$ bis $R_{12}$ und X und Y die unter Formel I angegebenen Bedeutungen besitzen, durch Umsetzung von Verbindungen der Formel Ib mit (a) Hal—CO—R' oder (b) $Hal{-}\underset{\underset{O}{\|}}{C}X{-}R'$ oder (c) OCN—R'

(Siehe Schema Seite 37 f.)

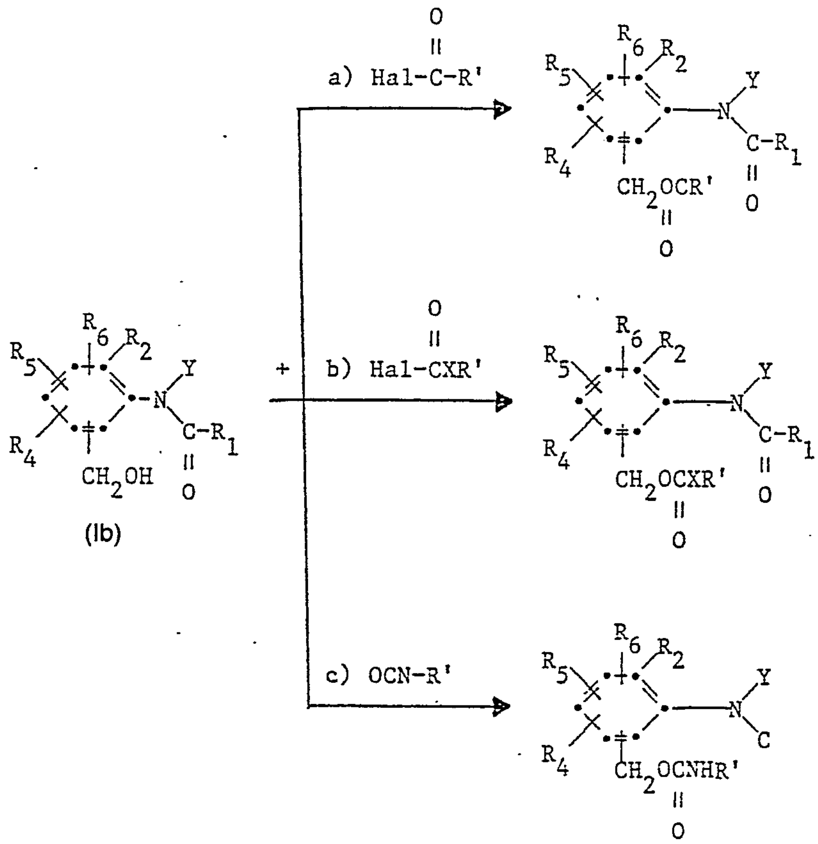


(Ib) (Ic) (Id) (Ie)

worin Hal Halogen bedeutet.

11. Verfahren nach Anspruch 7, dadurch gekennzeichnet, dass in Methode (A) als reaktionsfähiges Säurederivat der Formel III ein Säurehalogenid, Säureanhydrid oder ein Ester verwendet wird.

12. Verfahren nach Anspruch 11, dadurch gekennzeichnet, dass das Säurehalogenid das Säurechlorid oder das Säurebromid bedeutet.

13. Verfahren nach den Ansprüchen 7, 11 und 12, dadurch gekennzeichnet, dass die Methode (A) in Gegenwart eines säurebindenden Mittels oder eines Kondensationsmittels durchgeführt wird.

14. Verfahren nach den Ansprüchen 7, 11, 12 und 13, dadurch gekennzeichnet, dass die Methode (A) in Gegenwart eines reaktionsinerten Lösungsmittels oder Lösungsmittelgemisches durchgeführt wird.

15. Verfahren nach Anspruch 7, dadurch gekennzeichnet, dass man die Methode (A) bei Temperaturen von 0° bis 180 °C durchführt.

16. Verfahren nach Anspruch 15, dadurch gekennzeichnet, dass die Reaktionstemperatur 0° bis 150 °C beträgt.

17. Verfahren nach Anspruch 7, dadurch gekennzeichnet, dass in Methode (B) als Abgangsgruppe A Alkoxy, Benzolsulfonyloxy, p-Brombenzolsulfonyloxy, p-Toxyloxy, Trifluoracetyloxy, Niederalkylsulfonyloxy oder Halogen verwendet wird.

18. Verfahren zur Herstellung von Verbindungen der Formel VI, dadurch gekennzeichnet, dass Verbindungen der Formel VIII mit einem reaktionsfähigen Nitrierungsreagenz zu Verbindungen der Formel IX nitriert und anschliessend mit einem üblichem Hydrierungsmittel hydriert werden :

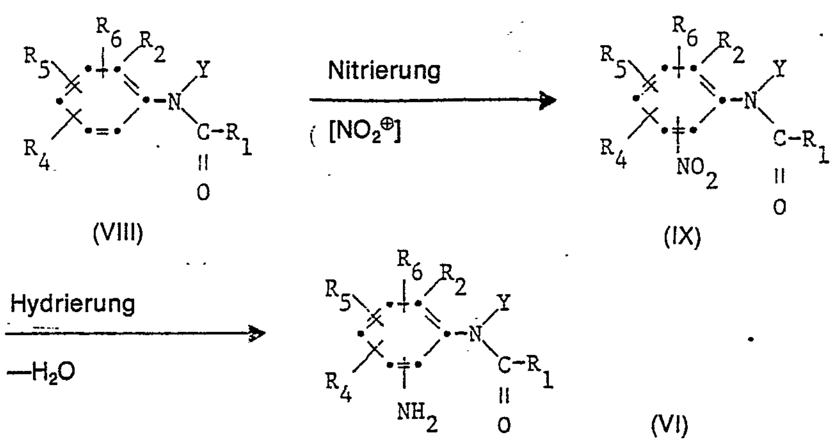


(VIII) (IX) (VI)

19. Mittel zur Bekämpfung und/oder Verhütung eines Befalls von schädlichen Mikroorganismen, dadurch gekennzeichnet, dass es als aktive Komponente mindestens einen Wirkstoff der Formel I gemäss Anspruch 1 enthält.

20. Mittel nach Anspruch 19, dadurch gekennzeichnet, dass es 0,1 bis 99 Gewichtsprozent eines Wirkstoffes der Formel I gemäss Anspruch 1, 1 bis 99 Gewichtsprozent an Zusatzstoffen und 0 bis 25 Gewichtsprozent eines Tensides enthält.

21. Mittel nach Anspruch 19, dadurch gekennzeichnet, dass es 0,1 bis 95 Gew.-% eines Wirkstoffes der Formel I gemäss Anspruch 1 enthält.

22. Mittel nach den Ansprüchen 20 und 21, dadurch gekennzeichnet, dass es 0,1 bis 25 Gew.-% eines Tensids enthält.

23. Mittel nach einem der Ansprüche 19 bis 22, dadurch gekennzeichnet, dass es als Aktivsubstanz eine der Verbindungen der Formel I gemäss einem der Ansprüche 2 bis 6 enthält.

24. Verfahren zur Herstellung von Mitteln zur Bekämpfung und/oder Verhütung eines Befalls von Mikroorganismen, dadurch gekennzeichnet, dass man eine Verbindung der Formel I nach einem der Ansprüche 1 bis 6, zusammen mit geeigneten Trägerstoffen und/oder Tensiden innig vermischt.

25. Verwendung von Verbindungen der Formel I nach Anspruch 1 zur Regulierung des Pflanzenwachstums und/oder zur Bekämpfung und/oder Verhütung eines Befalls von phytopathogenen Mikroorganismen.

26. Verwendung nach Anspruch 25 von Verbindungen der Formel I gemäss einem der Ansprüche 2 bis 6.

27. Verwendung nach Anspruch 25, dadurch gekennzeichnet, dass die Mikroorganismen phytopathogene Pilze sind.

28. Verfahren zur Bekämpfung und/oder Verhütung eines Befalls der Pflanzen durch phytopathogene Pilze, dadurch gekennzeichnet, dass man eine Verbindung der Formel I nach einem der Ansprüche 1 bis 6 in verdünnter Form auf die Pflanze oder deren Standort appliziert.

**Patentansprüche** (für den Vertragsstaat AT)

1. Mittel zur Bekämpfung und/oder Verhütung eines Befalls von schädlichen Mikroorganismen, dadurch gekennzeichnet, dass es als aktive Komponente mindestens einen Wirkstoff der allgemeinen Formel I

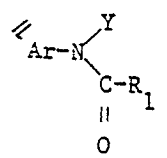

(I)

worin Ar einen Rest der Formeln

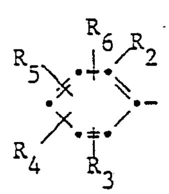

oder

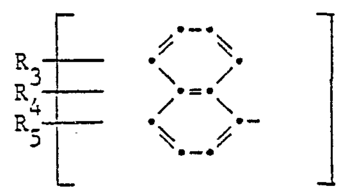

$R_1$ gegebenenfalls durch Halogen substituiertes 2-Furyl, 2-Tetrahydrofuryl, $C_2$-$C_4$-Alkenyl, Cyclopropyl, β-($C_1$-$C_4$)-Alkoxyethyl oder den Rest —$CH_2Z$ bedeutet, wobei Z für

a) —OH

b) —1H-1,2,4-Triazolyl, 1-Imidazolyl, 1-Pyrazolyl,

c) —$S(O)_n$—$R_{13}$ mit $R_{13}$ gleich $C_1$-$C_4$-Alkyl und n gleich 1 oder 2,

d) —X—$R_{14}$ mit X gleich Sauerstoff oder Schwefel und $R_{14}$ gleich gegebenenfalls durch $C_1$-$C_2$-Alkoxy substituiertes $C_1$-$C_6$-Alkyl, $C_3$-$C_4$-Alkenyl oder $C_3$-$C_4$-Alkinyl,

e) —$OSO_2$—$R_{15}$ mit $R_{15}$ gleich $C_1$-$C_4$ Alkyl, Mono- oder Di-($C_1$-$C_3$)-Alkylamin,

f)

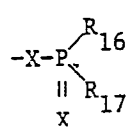

mit X gleich Sauerstoff oder Schwefel, $R_{16}$ gleich $C_1$-$C_4$-Alkyl, $C_1$-$C_4$-Alkoxy oder $C_1$-$C_4$-Alkylamino und $R_{17}$ gleich $C_1$-$C_4$-Alkoxy oder $C_1$-$C_4$-Alkylamino,

oder

g) 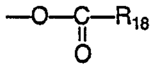 mit $R_{18}$ gleich gegebenenfalls durch $C_1$-$C_2$-Alkoxy substituiertes $C_1$-$C_3$-Alkyl

steht,

$R_2$ $C_1$-$C_3$-Alkyl, $C_1$-$C_3$-Alkoxy oder Halogen,

$R_3$ 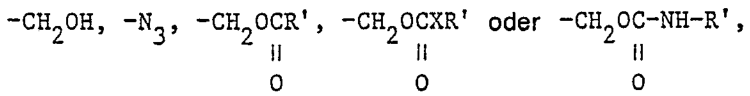

wobei

R' gegebenenfalls durch Halogen oder $C_1$-$C_3$-Alkoxy substituiertes $C_1$-$C_6$-Alkyl, $C_2$-$C_4$-Alkenyl oder Phenyl darstellt, und X für Sauerstoff oder Schwefel steht,

$R_4$ Wasserstoff, $C_1$-$C_3$-Alkyl, $C_1$-$C_3$-Alkoxy, Halogen, —$NO_2$ oder —$NH_2$,

$R_5$ Wasserstoff oder $C_1$-$C_3$-Alkyl,

$R_6$ Wasserstoff oder $C_1$-$C_3$-Alkyl,

Y 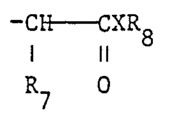 , 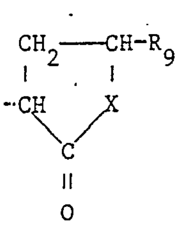 , 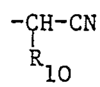

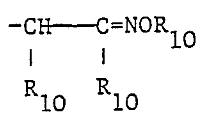 , 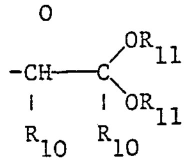 oder

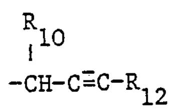 darstellt,

wobei

$R_7$ für Wasserstoff, gegebenenfalls durch Halogen, —OH, —$OC_1$-$C_3$-Alkyl oder —OCNH-$C_1$-$C_3$-Alkyl substituiertes $C_1$-$C_2$-Alkyl,

$R_8$ für gegebenenfalls durch $C_1$-$C_2$-Alkoxy substituiertes $C_1$-$C_4$ Alkyl,

$R_9$ für Wasserstoff oder Methyl,

$R_{10}$ für Wasserstoff oder $C_1$-$C_3$-Alkyl,

$R_{11}$ für $C_1$-$C_4$-Alkyl, wobei $R_{11}$ zusammen eine ein- oder mehrfach durch $C_1$-$C_3$-Alkyl substituierte $C_2$-$C_3$-Alkylenbrücke darstellen können,

$R_{12}$ für Wasserstoff, Halogen, $C_1$-$C_2$-Alkyl oder Phenyl stehen und

X Sauerstoff oder Schwefel darstellt.

2. Mittel nach Anspruch 1 enthaltend als aktive Komponente eine Verbindung der Formel 1, worin

$R_1$ 2-Furyl, 2-Tetrahydrofuryl, $C_2$-$C_4$-Alkenyl, Cyclopropyl, β-($C_1$-$C_2$)-Alkoxyethyl oder $CH_2$-Z, wobei Z für

a) —OH

b) 1H-1,2,4-Triazolyl, 1-Imidazolyl,

c) —$S(O)_n$—$R_{13}$ mit $R_{13}$ gleich $C_1$-$C_2$-Alkyl und n gleich 2,

d) —X'—$R_{14}$ mit X' gleich Sauerstoff oder Schwefel und $R_{14}$ gleich gegebenenfalls durch $C_1$-$C_2$-Alkoxy substituiertes $C_1$-$C_6$-Alkyl, $C_3$-$C_4$-Alkenyl oder $C_3$-$C_4$-Alkinyl,

e) —$OSO_2$—$R_{15}$ mit $R_{15}$ gleich $C_1$-$C_2$-Alkyl, Mono- oder Di-($C_1$-$C_2$)-Alkylamin

f)

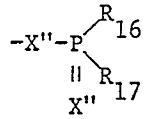 mit X" gleich Schwefel,

$R_{16}$ gleich $C_1$-$C_2$-Alkyl, $C_1$-$C_4$-Alkoxy, $C_1$-$C_2$-Alkylamino und $R_{17}$ gleich $C_1$-$C_4$-Alkoxy oder $C_1$-$C_4$-Alkylamino,
oder
g) $-O-\underset{\underset{O}{\|}}{C}-R_{18}$ mit $R_{18}$ gleich gegebenenfalls durch $C_1$-$C_2$-Alkoxy substituiertes $C_1$-$C_2$-Alkyl steht,

$R_2$ $C_1$-$C_2$-Alkyl, $C_1$-$C_2$-Alkoxy oder Halogen,

$R_3$ $-CH_2OH$, $-N_3$, $-CH_2O\underset{\underset{O}{\|}}{C}-R'$, $-CH_2O\underset{\underset{O}{\|}}{C}X'''-R'$ oder $-CH_2O\underset{\underset{O}{\|}}{C}NH-R'$

wobei
R' für gegebenenfalls durch Chlor oder Methoxy substituiertes $C_1$-$C_3$-Alkyl oder $C_2$-$C_3$-Alkenyl steht und X''' Sauerstoff oder Schwefel darstellt,
$R_4$ Wasserstoff, $C_1$-$C_2$-Alkyl, $C_1$-$C_2$-Alkoxy, $NO_2$ oder $NH_2$,
$R_5$ Wasserstoff oder $C_1$-$C_2$-Alkyl,
$R_6$ Wasserstoff oder $C_1$-$C_2$-Alkyl,
Y die unter Formel I angegebenen Bedeutungen hat, wobei
$R_7$ Wasserstoff oder gegebenenfalls durch Halogen, $-OH$, $-O(C_1$-$C_2)$-Alkyl oder $-O\underset{\underset{O}{\|}}{C}NH-(C_1$-$C_2)$-Alkyl substituiertes $C_1$-$C_2$-Alkyl,
$R_8$ gegebenenfalls durch $C_1$-$C_2$-Alkoxy substituiertes $C_1$-$C_3$-Alkyl,
$R_9$ Wasserstoff,
$R_{10}$ Wasserstoff oder $C_1$-$C_2$-Alkyl,
$R_{11}$ $C_1$-$C_2$-Alkyl, wobei $R_{11}$ zusammen auch $C_2$-$C_3$-Alkylen darstellen können,
$R_{12}$ Wasserstoff, Halogen oder $C_1$-$C_2$-Alkyl und
X Sauerstoff oder Schwefel bedeuten.

3. Mittel nach den Ansprüchen 1 und 2 enthaltend als aktive Komponente eine Verbindung der Formel I, worin
$R_1$ 2-Furyl, 2-Tetrahydrofuryl, $C_2$-$C_4$-Alkenyl, Cyclopropyl, 4-Methoxyethyl oder $-CH_2Z$, wobei Z für
a) 1H-1,2,4-Triazolyl,
b) $-S(O)_n-R_{13}$ mit $R_{13}$ gleich $CH_3$ und n gleich 2,
c) $-X^{IV}-R_{14}$ mit $X^{IV}$ gleich Sauerstoff und $R_{14}$ gleich gegebenenfalls durch $C_1$-$C_2$-Alkoxy substituiertes $C_1$-$C_4$-Alkyl, $C_3$-$C_4$-Alkenyl oder $C_3$-$C_4$-Alkinyl,
d) $-OSO_2-R_{15}$ mit $R_{15}$ gleich Mono-$(C_1$-$C_3)$-Alkylamin,
e)

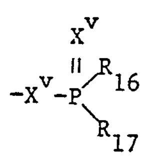

mit $X^V$ gleich Schwefel, $R_{16}$ und $R_{17}$ unabhängig voneinander gleich $C_1$-$C_3$-Alkoxy
oder
f) $-O\underset{\underset{O}{\|}}{C}-R_{18}$ mit $R_{18}$ gleich $C_1$-$C_3$-Alkyl steht,
$-CH_2OCR'$, $-CH_2CXR'$ oder $-CH_2OCNHR'$

$R_2$ Methyl, Methoxy oder Chlor,
$R_3$ $-CH_2OH$, $-N_3$ oder $-CH_2O\underset{\underset{O}{\|}}{C}CH_3$

$R_4$ Wasserstoff, Methyl, Methoxy, $NO_2$ oder $NH_2$,
$R_5$ Wasserstoff oder Methyl,
$R_6$ Wasserstoff oder Methyl,
Y die unter Formel I angegebenen Bedeutungen hat, wobei
$R_7$ gegebenenfalls durch Chlor, $-OH$, $-OC_1$-$C_2$-Alkyl oder $-O\underset{\underset{O}{\|}}{C}NH$-$C_1$-$C_2$-Alkyl substituiertes $C_1$-$C_2$-Alkyl
$R_8$ $C_1$-$C_3$-Alkyl,
$R_9$ Wasserstoff,
$R_{10}$ : Methyl,

$R_{11}$ $C_1$-$C_2$-Alkyl, wobei $R_{11}$ zusammen auch $C_2$-$C_3$-Alkylen darstellen kann,
$R_{12}$ Wasserstoff, Brom, Jod oder $C_1$-$C_2$-Alkyl und
X : Sauerstoff bedeutet.

4. Mittel nach den Ansprüchen 1-3 enthaltend als aktive Komponente eine Verbindung der Formel I, worin $R_3$ die Azidogruppe (—$N_3$) bedeutet und die übrigen Reste $R_1$ bis $R_{12}$, X und Y die in Anspruch 3 angegebenen Bedeutungen besitzen.

5. Mittel nach den Ansprüchen 1-4 enthaltend als aktive Komponente eine Verbindung der Formel I, worin

Y die Reste —$CH(CH_3)COOCH_3$ oder 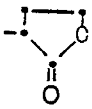 bedeutet,

$R_3$ die Azidogruppe —($N_3$) darstellt und die übrigen Reste $R_1$ bis $R_{12}$ und X die in Anspruch 3 angegebenen Bedeutungen besitzen.

6. Mittel nach den Ansprüchen 1-5, dadurch gekennzeichnet, dass es 0,1 bis 99 Gewichtsprozent eines Wirkstoffes der Formel I gemäss den Ansprüchen 1-5, 1 bis 99 Gew.-% an Zusatzstoffen und 0 bis 25 Gew.-% eines Tensides enthält.

7. Mittel nach den Ansprüchen 1-5, dadurch gekennzeichnet, dass es 0,1 bis 95 Gew.-% eines Wirkstoffes der Formel I gemäss Anspruch 1 enthält.

8. Mittel nach den Ansprüchen 6 und 7, dadurch gekennzeichnet, dass es 0,1 bis 25 Gew.-% eines Tensids enthält.

9. Mittel nach den Ansprüchen 1-8, dadurch gekennzeichnet, dass es als Aktivsubstanz eine der Verbindungen der Formel I gemäss einem der Ansprüche 2 bis 6 enthält.

10. Verfahren zur Herstellung von Mitteln zur Bekämpfung und/oder Verhütung eines Befalls von Mikroorganismen, dadurch gekennzeichnet, dass man eine Verbindung der Formel I nach einem der Ansprüche 1-5, zusammen mit geeigneten Trägerstoffen und/oder Tensiden innig vermischt.

11. Verwendung von Verbindungen der Formel I nach den Ansprüchen 1-5 zur Regulierung des Pflanzenwachstums und/oder zur Bekämpfung und/oder Verhütung eines Befalls von phytopathogenen Mikroorganismen.

12. Verwendung nach Anspruch 11 von Verbindungen der Formel I gemäss einem der Ansprüche 2 bis 5.

13. Verwendung nach Anspruch 11, dadurch gekennzeichnet, dass die Mikroorganismen phytopathogene Pilze sind.

14. Verfahren zur Bekämpfung und/oder Verhütung eines Befalls der Pflanzen durch phytopathogene Pilze, dadurch gekennzeichnet, dass man eine Verbindung der Formel I nach einem der Ansprüche 1 bis 5 in verdünnter Form auf die Pflanze oder deren Standort appliziert.

15. Verfahren zur Herstellung von Verbindungen der Formel I in Anspruch 1, gekennzeichnet

A) durch Acylierung von Verbindungen der Formel II

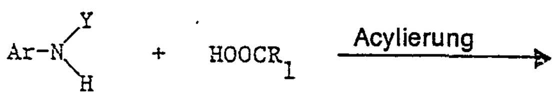

(I)

(II) (III)

mit Carbonsäuren der Formel III oder ihren reaktionsfähigen Derivaten oder

B) durch Umsetzung von Verbindungen der Formel IV

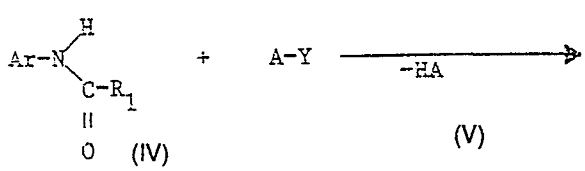

(I)

(IV) (V)

mit Verbindungen der Formel V, worin A eine übliche Abgangsgruppe bedeutet.

16. Verfahren zur Herstellung von Verbindungen der Formel Ia, in welcher $R_3$ die $N_3$-Gruppe bedeutet und die Reste $R_1$, $R_2$ und $R_4$ bis $R_{12}$ und X und Y die unter Formel I angegebenen Bedeutungen besitzen, durch Bildung der Diazoniumsalze der Verbindungen der Formel VI

(VI) 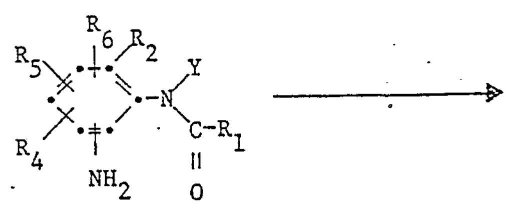 Diazoniumsalze

und anschliessende variable Umsetzung

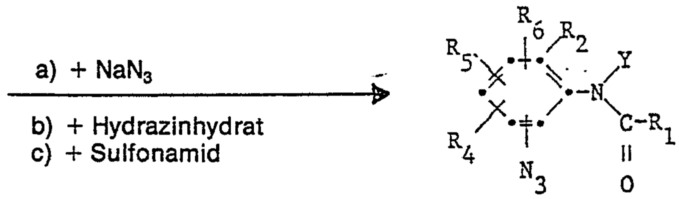


(Ia)

17. Verfahren zur Herstellung von Verbindungen der Formel Ib, in welcher $R_3$ die $HOCH_2$-Gruppe bedeutet und die Reste $R_1$, $R_2$ und $R_4$ bis $R_{12}$ und X und Y die unter Formel I angegebenen Bedeutungen besitzen, durch Umsetzung von Verbindungen der Formel VII mit NaOH in Aceton

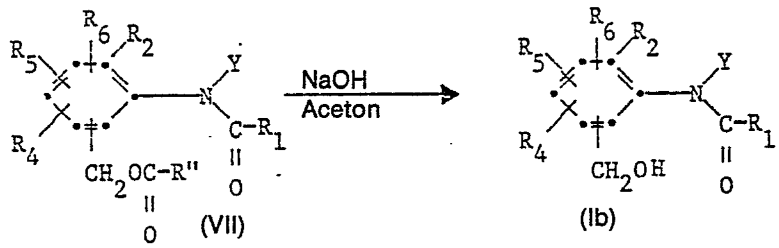


(VII) (Ib)

worin R" $C_1$-$C_4$-Alkyl bedeutet.

18. Verfahren zur Herstellung von Verbindungen der Formeln Ic, Id und Ie, in denen $R_3$ eine der Gruppen

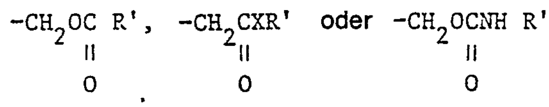

oder

darstellt und die Reste $R_1$, $R_2$ und $R_4$ bis $R_{12}$ und X und Y die unter Formel I angegebenen Bedeutungen besitzen, durch Umsetzung von Verbindungen der Formel Ib mit (a) Hal—CO—R' oder (b) Hal—CX—R' oder (c) OCN—R'

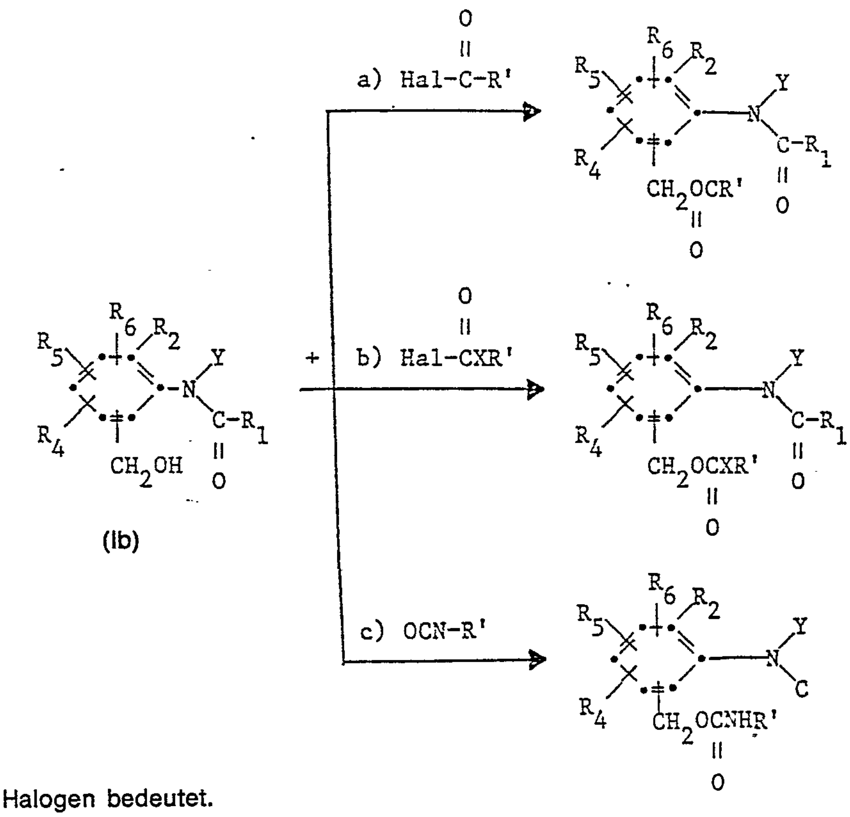


(Ib)

(Ic)

(Id)

(Ie)

worin Hal Halogen bedeutet.

19. Verfahren nach Anspruch 15, dadurch gekennzeichnet, dass in Methode (A) als reaktionsfähiges Säurederivat der Formel III ein Säurehalogenid, Säureanhydrid oder ein Ester verwendet wird.

20. Verfahren nach Anspruch 19, dadurch gekennzeichnet, dass das Säurehalogenid das Säurechlorid oder das Säurebromid bedeutet.

21. Verfahren nach den Ansprüchen 15, 19 und 20, dadurch gekennzeichnet, dass die Methode (A) in Gegenwart eines säurebindenden Mittels oder eines Kondensationsmittels durchgeführt wird.

22. Verfahren nach den Ansprüchen 15, 19, 20 und 21, dadurch gekennzeichnet, dass die Methode (A) in Gegenwart eines reaktionsinerten Lösungsmittels oder Lösungsmittelgemisches durchgeführt wird.

23. Verfahren nach Anspruch 15, dadurch gekennzeichnet, dass man die Methode (A) bei Temperaturen von 0° bis 180 °C durchführt.

24. Verfahren nach Anspruch 23, dadurch gekennzeichnet, dass die Reaktionstemperatur 0° bis 150 °C beträgt.

25. Verfahren nach Anspruch 15, dadurch gekennzeichnet, dass in Methode (B) als Abgangsgruppe A Alkoxy, Benzolsulfonyloxy, p-Brombenzolsulfonyloxy, p-Toxyloxy, Trifluoracetyloxy, Niederalkylsulfonyloxy oder Halogen verwendet wird.

26. Verfahren zur Herstellung von Verbindungen der Formel VI, dadurch gekennzeichnet, dass Verbindungen der Formel VIII mit einem reaktionsfähigen Nitrierungsreagenz zu Verbindungen der Formel IX nitriert und anschliessend mit einem üblichem Hydrierungsmittel hydriert werden :

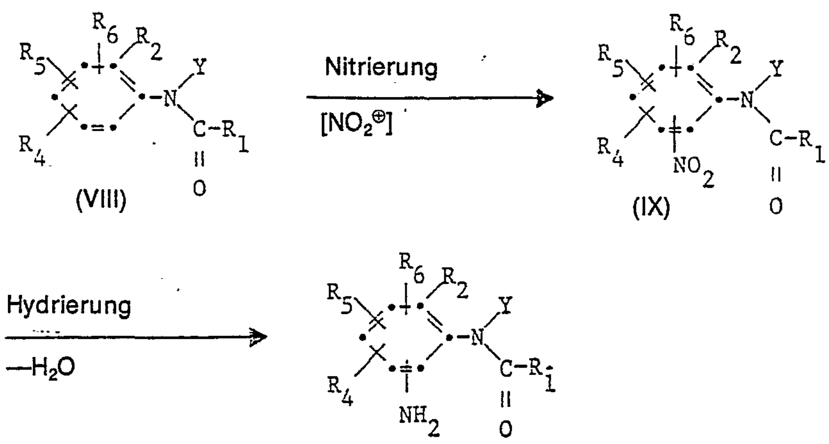


(VIII) (IX) (VI)

**Claims** (for the Contracting States : BE, CH, DE, FR, GB, IT, LI, LU, NL, SE)

1. A compound of the general formula I

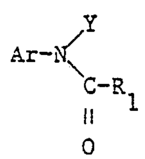

(I)

wherein

Ar is a group of the formula

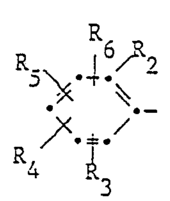

or

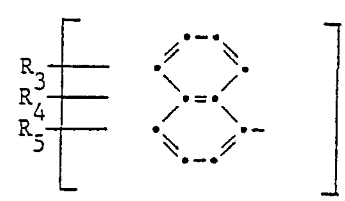

$R_1$ is 2-furyl, 2-tetrahydrofuryl, $C_2$-$C_4$ alkenyl, cyclopropyl, β-($C_1$-$C_4$) alkoxyethyl or $—CH_2Z$, each being unsubstituted or substituted by halogen, and Z in the group $—CH_2Z$ being

a) —OH,

b) —1H—1,2,4-triazolyl, 1-imidazolyl or 1-pyrazolyl,

c) $—S(O)_n—R_{13}$ where $R_{13}$ is $C_1$-$C_4$ alkyl, and n is 1 or 2,

d) $-X-R_{14}$ where X is oxygen or sulfur, and $R_{14}$ is $C_1$-$C_6$-alkyl, $C_3$-$C_4$ alkenyl or $C_3$-$C_4$ alkynyl, each unsubstituted or substituted by $C_1$-$C_2$ alkoxy,

e) $-OSO_2-R_{15}$ where $R_{15}$ is $C_1$-$C_4$ alkyl, or mono- or di ($C_1$-$C_3$)-alkylamine,

f)

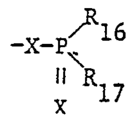

where X is oxygen or sulfur, $R_{16}$ is $C_1$-$C_4$ alkyl, $C_1$-$C_4$ alkoxy or $C_1$-$C_4$ alkylamino, and $R_{17}$ is $C_1$-$C_4$ alkoxy or $C_1$-$C_4$ alkylamino, or

g) $-O-\underset{\underset{O}{\|}}{C}-R_{18}$ where $R_{18}$ is $C_1$-$C_3$ alkyl which is unsubstituted or substituted by $C_1$-$C_2$ alkoxy,

$R_2$ is $C_1$-$C_3$-alkyl, $C_1$-$C_3$-alkoxy or halogen,

$R_3$ is

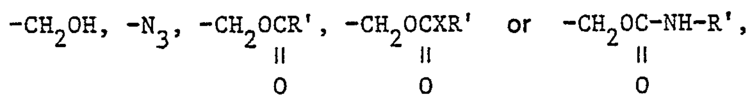

where R' is $C_1$-$C_6$ alkyl or $C_2$-$C_4$ alkenyl, each unsubstituted or substituted by halogen or $C_1$-$C_3$-alkoxy, or is phenyl, and X is oxygen or sulfur,

$R_4$ is hydrogen, $C_1$-$C_3$ alkyl, $C_1$-$C_3$ alkoxy, halogen, $-NO_2$ or $-NH_2$,

$R_5$ is hydrogen or $C_1$-$C_3$ alkyl,

$R_6$ is hydrogen or $C_1$-$C_3$ alkyl,

Y is

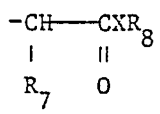 , 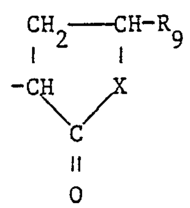 , 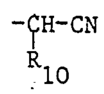 ,

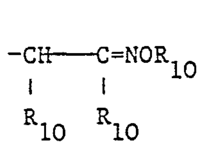 , 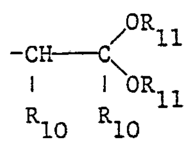 or

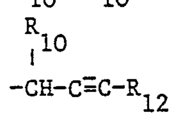

wherein

$R_7$ is hydrogen, or $C_1$-$C_2$ alkyl which is unsubstituted or substituted by halogen, $-OH$, $-OC_1$-$C_3$ alkyl or $-O\underset{\underset{O}{\|}}{C}NH$-$C_1$-$C_3$ alkyl,

$R_8$ is $C_1$-$C_4$ alkyl which is unsubstituted or substituted by $C_1$-$C_2$ alkoxy,

$R_9$ is hydrogen or methyl,

$R_{10}$ is hydrogen or $C_1$-$C_3$ alkyl,

$R_{11}$ is $C_1$-$C_4$ alkyl, and both substituents $R_{11}$ together can form a $C_2$-$C_3$ alkylene bridge which is substituted by one or more $C_1$-$C_3$ alkyl groups,

$R_{12}$ is hydrogen, halogen, $C_1$-$C_2$ alkyl or phenyl, and

X is oxygen or sulfur.

2. A compound of the formula I according to claim 1, wherein

$R_1$ is 2-furyl, 2-tetrahydrofuryl, $C_2$-$C_4$ alkenyl, cyclopropyl, β-($C_1$-$C_2$) alkoxyethyl or $CH_2-Z$, where Z is

a) $-OH$,

b) 1H-1,2,4-triazolyl or 1-imidazolyl,

c) $-S(O)_n-R_{13}$ where $R_{13}$ is $C_1$-$C_2$ alkyl, and n is 2,

d) $-X'-R_{14}$ where X' is oxygen or sulfur, and $R_{14}$ is $C_1$-$C_6$-alkyl, $C_3$-$C_4$ alkenyl or $C_3$-$C_4$ alkynyl,

each unsubstituted or substituted by $C_1$-$C_2$ alkoxy,

e) $-OSO_2-R_{15}$ where $R_{15}$ is $C_1$-$C_2$ alkyl, or mono- or di ($C_1$-$C_2$)-alkylamine,

f)

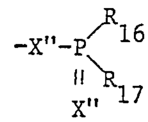

where X″ is sulfur, $R_{16}$ is $C_1$-$C_2$ alkyl, $C_1$-$C_4$-alkoxy or $C_1$-$C_2$ alkylamino, and $R_{17}$ is $C_1$-$C_4$ alkoxy or $C_1$-$C_4$ alkylamino, or

g) $-O-\underset{\underset{O}{\|}}{C}-R_{18}$ where $R_{18}$ is $C_1$-$C_2$ alkyl which is unsubstituted or substituted by $C_1$-$C_2$ alkoxy,

$R_2$ is $C_1$-$C_2$ alkyl, $C_1$-$C_2$ alkoxy or halogen,

$R_3$ is

$$-CH_2OH,\ -N_3,\ -CH_2O\underset{\underset{O}{\|}}{C}-R',\ -CH_2O\underset{\underset{O}{\|}}{C}X'''-R' \text{ or } -CH_2O\underset{\underset{O}{\|}}{C}NH-R'$$

where R′ is $C_1$-$C_3$ alkyl or $C_2$-$C_3$ alkenyl, each unsubstituted or substituted by chlorine or methoxy, and X‴ is oxygen or sulfur,

$R_4$ is hydrogen, $C_1$-$C_2$ alkyl, $C_1$-$C_2$ alkoxy, $-NO_2$ or $-NH_2$,

$R_5$ is hydrogen or $C_1$-$C_2$ alkyl,

$R_6$ is hydrogen or $C_1$-$C_2$ alkyl,

Y is as defined for formula I, and $R_7$ is hydrogen, or $C_1$-$C_2$ alkyl which is unsubstituted or substituted by halogen, $-OH$, $-O(C_1$-$C_2)$ alkyl or $-O\underset{\underset{O}{\|}}{C}NH-(C_1$-$C_2)$ alkyl,

$R_8$ is $C_1$-$C_3$ alkyl which is unsubstituted or substituted by $C_1$-$C_2$ alkoxy,

$R_9$ is hydrogen,

$R_{10}$ is hydrogen or $C_1$-$C_2$ alkyl,

$R_{11}$ is $C_1$-$C_2$ alkyl, and the substituents $R_{11}$ together can also be $C_2$-$C_3$ alkylene,

$R_{12}$ is hydrogen, halogen or $C_1$-$C_2$ alkyl, and

X is oxygen or sulfur.

3. A compound of the formula I according to claim 1, wherein

$R_1$ is 2-furyl, 2-tetrahydrofuryl, $C_2$-$C_4$ alkenyl, cyclopropyl, 4-methoxyethyl or $-CH_2Z$, where Z is

a) 1H-1,2,4-triazolyl,

b) $-S(O)_n-R_{13}$ where $R_{13}$ is $CH_3$, and n is 2,

c) $-X^{IV}-R_{14}$ where $X^{IV}$ is oxygen, and $R_{14}$ is $C_1$-$C_4$ alkyl, $C_3$-$C_4$ alkenyl or $C_3$-$C_4$ alkynyl, each unsubstituted or substituted by $C_1$-$C_2$ alkoxy;

d) $-OSO_2-R_{15}$ where $R_{15}$ is mono ($C_1$-$C_3$) alkylamine,

e)

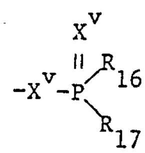

where $X^V$ is sulfur, and $R_{16}$ and $R_{17}$ are each independently of the other $C_1$-$C_3$ alkoxy, or

f) $-O\underset{\underset{O}{\|}}{C}-R_{18}$ where $R_{18}$ is $C_1$-$C_3$ alkyl,

$R_2$ is methyl, methoxy or chlorine,

$R_3$ is $-CH_2OH$, $-N_3$ or $-CH_2O\underset{\underset{O}{\|}}{C}CH_3$

$R_4$ is hydrogen, methyl, methoxy, $NO_2$ or $NH_2$,

$R_5$ is hydrogen or methyl,

$R_6$ is hydrogen or methyl,

Y is as defined for formula I, and $R_7$ is $C_1$-$C_2$ alkyl which is unsubstituted or substituted by chlorine, $-OH$, $-OC_1$-$C_2$ alkyl or $-O\underset{\underset{O}{\|}}{C}NH-C_1$-$C_2$ alkyl,

$R_8$ is $C_1$-$C_3$ alkyl,
$R_9$ is hydrogen,
$R_{10}$ is methyl,
$R_{11}$ is $C_1$-$C_2$ alkyl, and the substituents $R_{11}$ together can also be $C_2$-$C_3$ alkylene,
$R_{12}$ is hydrogen, bromine, iodine or $C_1$-$C_2$ alkyl, and
X is oxygen.

4. A compound of the formula I according to claim 3, wherein
$R_3$ is the azido group ($-N_3$), and the remaining symbols $R_1$ to $R_{12}$, X and Y are as defined in claim 3.

5. A compound of the formula I according to claim 4, wherein
Y is the group $-CH(CH_3)COOH_3$ or

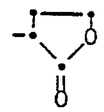

$R_3$ is the azido group $-(N_3)$, and the remaining symbols $R_1$ to $R_{12}$ and X are as defined in claim 3.

6. A compound of the formula I according to claim 4, selected from the group of the following azido compounds : N-(1'-methoxycarbonylethyl)-N-methoxyacetyl-2-methyl-6-azidoaniline,
N-(tetrahydro-2'-furon-3'-yl)-N-methoxyacetyl-2,6-dimethyl-3-azidoaniline,
N-(1'-methoxycarbonylethyl)-N-methoxyacetyl-2,6-dimethyl-3-azidoaniline,
N-(1'-methoxycarbonylethyl)-N-(2-tetrahydrofurylcarbonyl)-2-methyl-6-azidoaniline,
N-(1'-methoxycarbonyl-3'-hydroxy-n-propyl)-N-methoxyacetyl-2-methyl-6-azidoaniline,
N-(1'-methoxycarbonylethyl)-N-methylaminosulfonyloxyacetyl-2,6-dimethyl-3-azidoaniline,
N-(1'-methoxycarbonyl-3-hydroxy-n-propyl)-N-cyclopropylcarbonyl-2-methyl-6-azidoaniline,
N-(1'-methoxycarbonylethyl)-N-methoxyacetyl-2,3-dimethyl-6-azidoaniline,
N-(1'-cyanoethyl)-N-methoxyacetyl-2-methyl-6-azidoaniline,
N-(1'-methyl-2'-dimethoxyethyl)-N-methoxyacetyl-2,6-dimethyl-3-azidoaniline,
N-(2'-methyl-3'-methoximino-n-propyl)-N-methoxyacetyl-2-methyl-6-azidoaniline,
N-(tetrahydro-2'-furon-3'-yl)-N-methoxyacetyl-2,3-dimethyl-4-azido-α-naphthylamine,
N-(1'-cyanoethyl)-N-methoxyacetyl-2,3-dimethyl-4-azido-α-naphthylamine, and
N-(1'-cyanoethyl)-N-(2-tetrahydrofurylcarbonyl)-2,3-dimethyl-4-azido-α-naphthylamine.

7. A process for the preparation of a compound of formula I according to claim 1, which comprises
A) acylating a compound of formula II with a carboxylic acid of formula III or a reactive derivative thereof, in accordance with the reaction scheme

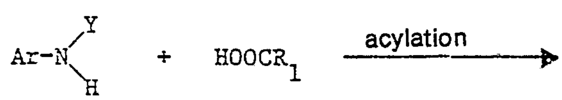


(I)

(II) (III)

or

B) reacting a compound of formula IV with a compound of formula V, wherein A is a customary leaving group, in accordance with the reaction scheme

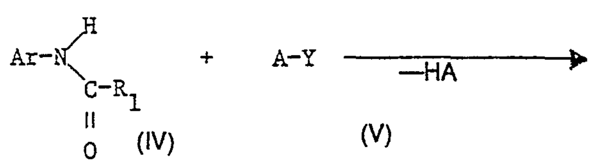


(I)

(IV) (V)

8. A process for the preparation of a compound of formula Ia, wherein $R_3$ is the $-N_3$ group and $R_1$, $R_2$ and $R_4$ to $R_{12}$ and X and Y are as defined for formula I, which comprises forming the diazonium salt of a compound of formula VI

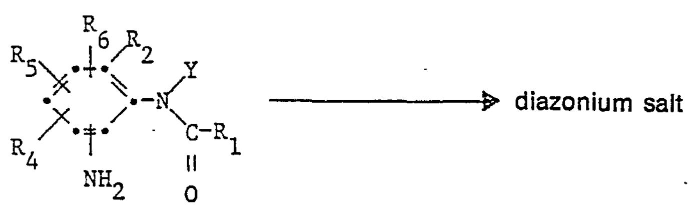


(VI)

and subsequently carrying out the variable reaction

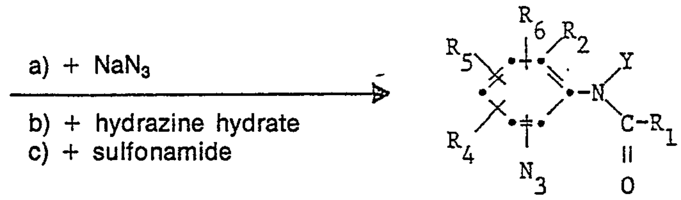


(Ia)

9. A process for the preparation of a compound of formula Ib, wherein $R_3$ is the $HOCH_2$ group and $R_1$, $R_2$ and $R_4$ to $R_{12}$ and X and Y are as defined for formula I, which comprises reacting a compound of formula VII with NaOH, in acetone, in accordance with the reaction scheme

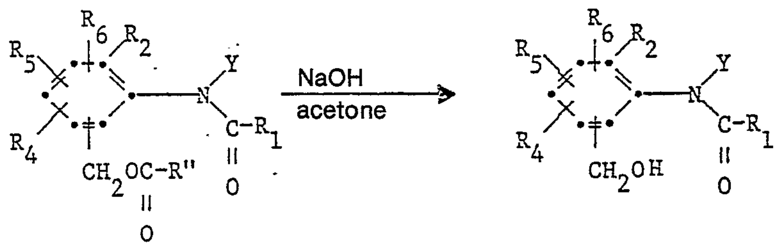


(VII) (Ib)

wherein R″ is $C_1$-$C_4$ alkyl.

10. A process for the preparation of a compound of formula Ic, Id or Ie, wherein $R_3$ is a

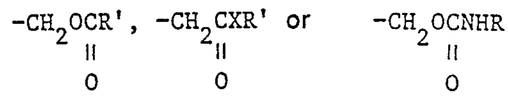

group, and $R_1$, $R_2$, $R_4$ to $R_{12}$ and X and Y are as defined for formula I, which process comprises reacting a compound of formula Ib with (a) Hal—CO—R′ or (b) $\mathrm{Hal{-}\underset{\underset{O}{\|}}{C}X{-}R'}$ or (c) OCN—R′, in accordance with the reaction scheme

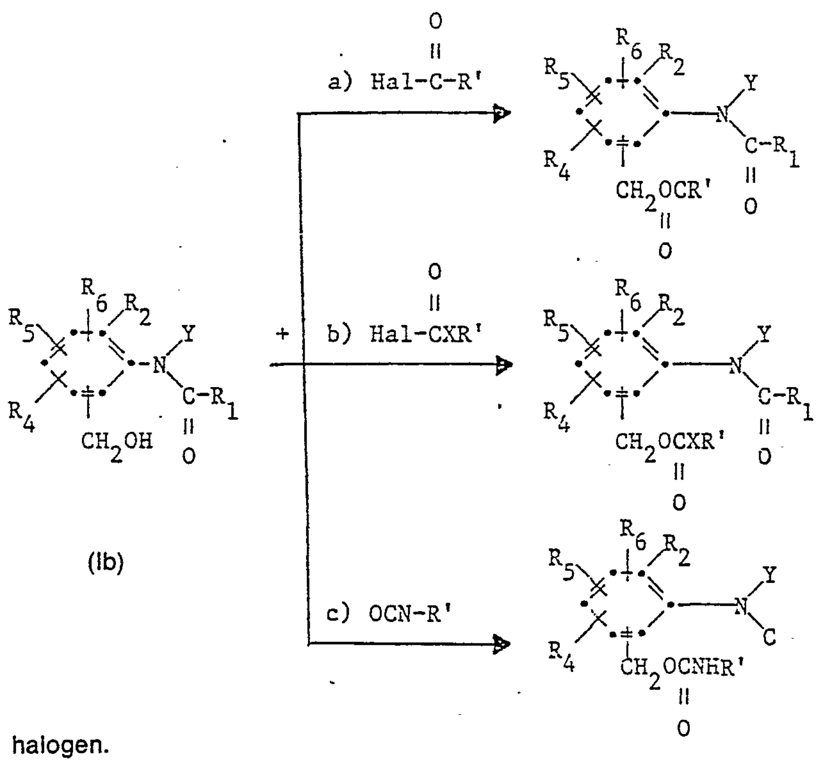


(Ib)

(Ic)

(Id)

(Ie)

wherein Hal is halogen.

11. A process according to claim 7, wherein the reactive acid derivative of the formula III employed in method A) is an acid halide, an acid anhydride or an ester.

12. A process according to claim 11, wherein the acid halide is an acid chloride or acid bromide.

13. A process according to any one of claims 7, 11 or 12, wherein method A) is carried out in the presence of an acid acceptor or of a condensing agent.

14. A process according to any one of claims 7, 11, 12 or 13, wherein method A) is carried out in the presence of an inert solvent or solvent mixture.

15. A process according to claim 7, wherein method A) is carried out in the temperature range from 0° to 180 °C.

16. A process according to claim 15, wherein the reaction temperature is in the range from 0° to 150 °C.

17. A process according to claim 7, wherein the leaving group A in method B) is alkoxy, benzenesulfonyloxy, p-bromobenzenesulfonyloxy, p-tolyloxy, trifluoroacetyloxy, lower alkylsulfonyloxy or halogen.

18. A process for the preparation of a compound of formula VI, which comprises nitrating a compound of formula VIII with a reactive nitrating reagent to give a compound of formula IX, and subsequently hydrogenating said compound of formula IX with a conventional hydrogenating agent, in accordance with the reaction scheme :

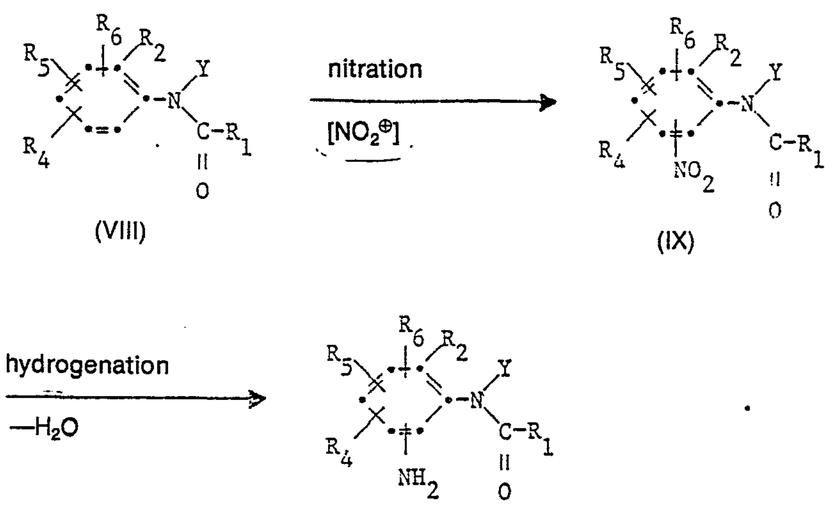


(VIII)

(IX)

(VI)

19. A composition for controlling and/or preventing infestation by harmful microorganisms, which composition contains as active ingredient at least one compound of the formula I according to claim 1.

20. A composition according to claim 19, which contains 0.1 to 99 per cent by weight of a compound of the formula I according to claim 1, 1 to 99 per cent by weight of adjuvants, and 0 to 25 per cent by weight of a surfactant.

21. A composition according to claim 19, which contains 0.1 to 95 per cent by weight of a compound of the formula I according to claim 1.

22. A composition according to either of claims 20 or 21, which contains 0.1 to 25 per cent by weight of a surfactant.

23. A composition according to any one of claims 19 to 22, which contains as active ingredient a compound of the formula I according to any one of claims 2 to 6.

24. A process for the preparation of a composition for controlling and/or preventing infestation by microorganisms, which process comprises homogeneously mixing a compound of formula I according to any one of claims 1 to 6 with suitable carriers and/or surfactants.

25. A method of regulating plant growth and/or of controlling and/or preventing infestation by phytopathogenic microorganisms, which comprises the use of a compound of formula I according to claim 1.

26. A method according to claim 25, which comprises the use of a compound of the formula I according to any one of claims 2 to 6.

27. A method according to claim 25, wherein the microorganisms are phytopathogenic fungi.

28. A method of controlling and/or preventing infestation of plants by phytopathogenic fungi, which process comprises applying a compound of the formula I according to any one of claims 1 to 6, in diluted form, to said plants or to the locus thereof.

**Claims** (for the Contracting State AT)

1. A composition for controlling and/or preventing infestation by harmful microorganisms, which contains as active component at least one compound of the general formula I

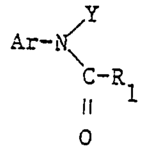 (I)

wherein Ar is a group of the formula

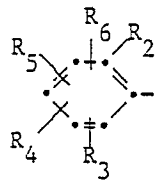 or 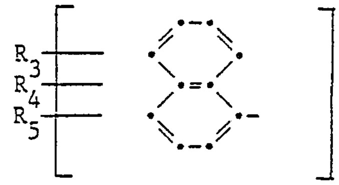

$R_1$ is 2-furyl, 2-tetrahydrofuryl, $C_2$-$C_4$ alkenyl, cyclopropyl, β-($C_1$-$C_4$) alkoxyethyl or $—CH_2Z$, each being unsubstituted or substituted by halogen, and Z in the group $—CH_2Z$ being

a) —OH,

b) —1H-1,2,4-triazolyl, 1-imidazolyl or 1-pyrazolyl,

c) $—S(O)_n—R_{13}$ where $R_{13}$ is $C_1$-$C_4$ alkyl, and n is 1 or 2,

d) $—X—R_{14}$ where X is oxygen or sulfur, and $R_{14}$ is $C_1$-$C_6$-alkyl, $C_3$-$C_4$ alkenyl or $C_3$-$C_4$ alkynyl, each unsubstituted or substituted by $C_1$-$C_2$ alkoxy,

e) $—OSO_2—R_{15}$ where $R_{15}$ is $C_1$-$C_4$ alkyl, or mono- or di ($C_1$-$C_3$)-alkylamine,

f)

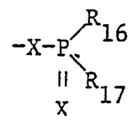

where X is oxygen or sulfur, $R_{16}$ is $C_1$-$C_4$ alkyl, $C_1$-$C_4$ alkoxy or $C_1$-$C_4$ alkylamino, and $R_{17}$ is $C_1$-$C_4$ alkoxy or $C_1$-$C_4$ alkylamino, or

g) 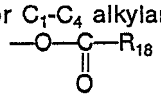 where $R_{18}$ is $C_1$-$C_3$ alkyl which is unsubstituted or substituted by $C_1$-$C_2$ alkoxy,

$R_2$ is $C_1$-$C_3$-alkyl, $C_1$-$C_3$-alkoxy or halogen,

$R_3$ is

$-CH_2OH$, $-N_3$, 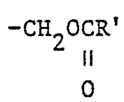, 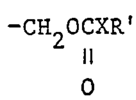 or 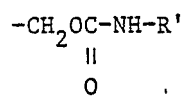.

where R' is $C_1$-$C_6$ alkyl or $C_2$-$C_4$ alkenyl, each unsubstituted or substituted by halogen or $C_1$-$C_3$-alkoxy, or is phenyl, and X is oxygen or sulfur,

$R_4$ is hydrogen, $C_1$-$C_3$ alkyl, $C_1$-$C_3$ alkoxy, halogen, $—NO_2$ or $—NH_2$,

$R_5$ is hydrogen or $C_1$-$C_3$ alkyl,

$R_6$ is hydrogen or $C_1$-$C_3$ alkyl,

Y is

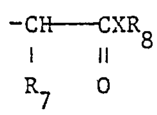, 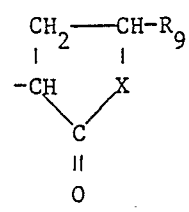, 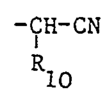

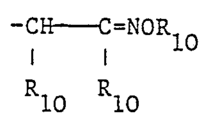, 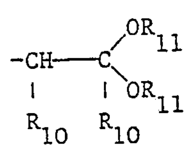 or

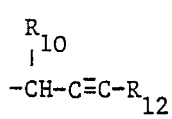

wherein

$R_7$ is hydrogen, or $C_1$-$C_2$ alkyl which is unsubstituted or substituted by halogen, —OH, —$OC_1$-$C_3$ alkyl or $-O\underset{\underset{O}{\|}}{C}NH-C_1$-$C_3$ alkyl,

$R_8$ is $C_1$-$C_4$-alkyl which is unsubstituted or substituted by $C_1$-$C_2$ alkoxy,

$R_9$ is hydrogen or methyl,

$R_{10}$ is hydrogen or $C_1$-$C_3$ alkyl,

$R_{11}$ is $C_1$-$C_4$ alkyl, and both substituents $R_{11}$ together can form a $C_2$-$C_3$ alkylene bridge which is substituted by one or more $C_1$-$C_3$ alkyl groups,

$R_{12}$ is hydrogen, halogen, $C_1$-$C_2$ alkyl or phenyl, and X is oxygen or sulfur.

2. A composition according to claim 1 containing as active component a compound of formula I, wherein

$R_1$ is 2-furyl, 2-tetrahydrofuryl, $C_2$-$C_4$ alkenyl, cyclopropyl, β-($C_1$-$C_2$) alkoxyethyl or $CH_2$—Z, where Z is

a) —OH,

b) 1H-1,2,4-triazolyl or 1-imidazolyl,

c) —$S(O)_n$—$R_{13}$ where $R_{13}$ is $C_1$-$C_2$ alkyl, and n is 2,

d) —X′—$R_{14}$ where X′ is oxygen or sulfur, and $R_{14}$ is $C_1$-$C_6$-alkyl, $C_3$-$C_4$ alkenyl or $C_3$-$C_4$ alkynyl, each unsubstituted or substituted by $C_1$-$C_2$ alkoxy,

e) —$OSO_2$—$R_{15}$ where $R_{15}$ is $C_1$-$C_2$ alkyl, or mono- or di ($C_1$-$C_2$)-alkylamine,

f)

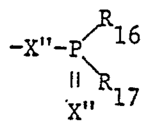

where X″ is sulfur, $R_{16}$ is $C_1$-$C_2$ alkyl, $C_1$-$C_4$-alkoxy or $C_1$-$C_2$ alkylamino, and $R_{17}$ is $C_1$-$C_4$ alkoxy or $C_1$-$C_4$ alkylamino, or

g) $-O-\underset{\underset{O}{\|}}{C}-R_{18}$ where $R_{18}$ is $C_1$-$C_2$ alkyl which is unsubstituted or substituted by $C_1$-$C_2$ alkoxy,

$R_2$ is $C_1$-$C_2$ alkyl, $C_1$-$C_2$ alkoxy or halogen,

$R_3$ is

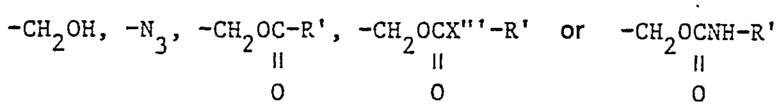

where R′ is $C_1$-$C_3$ alkyl or $C_2$-$C_3$ alkenyl, each unsubstituted or substituted by chlorine or methoxy, and X‴ is oxygen or sulfur,

$R_4$ is hydrogen, $C_1$-$C_2$ alkyl, $C_1$-$C_2$ alkoxy, —$NO_2$ or —$NH_2$,

$R_5$ is hydrogen or $C_1$-$C_2$ alkyl,

$R_6$ is hydrogen or $C_1$-$C_2$ alkyl,

Y is as defined for formula I, and $R_7$ is hydrogen, or $C_1$-$C_2$ alkyl which is unsubstituted or substituted by halogen, —OH, —O($C_1$-$C_2$) alkyl or $-O\underset{\underset{O}{\|}}{C}NH-(C_1$-$C_2)$ alkyl,

$R_8$ is $C_1$-$C_3$ alkyl which is unsubstituted or substituted by $C_1$-$C_2$ alkoxy,

$R_9$ is hydrogen,

$R_{10}$ is hydrogen or $C_1$-$C_2$ alkyl,

$R_{11}$ is $C_1$-$C_2$ alkyl, and the substituents $R_{11}$ together can also be $C_2$-$C_3$ alkylene,

$R_{12}$ is hydrogen, halogen or $C_1$-$C_2$ alkyl, and

X is oxygen or sulfur.

3. A composition according to either of claims 1 or 2, containing as active component a compound of formula I, wherein

$R_1$ is 2-furyl, 2-tetrahydrofuryl, $C_2$-$C_4$ alkenyl, cyclopropyl, 4-methoxyethyl or —$CH_2$Z, where Z is

a) 1H-1,2,4-triazolyl,

b) —$S(O)_n$—$R_{13}$ where $R_{13}$ is $CH_3$, and n is 2,

c) —$X'^V$—$R_{14}$ where $X'^V$ is oxygen, and $R_{14}$ is $C_1$-$C_4$ alkyl, $C_3$-$C_4$ alkenyl or $C_3$-$C_4$ alkynyl, each unsubstituted or substituted by $C_1$-$C_2$ alkoxy,

d) —$OSO_2$—$R_{15}$ where $R_{15}$ is mono ($C_1$-$C_3$) alkylamine,

e)

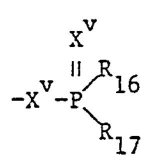

where $X^V$ is sulfur, and $R_{16}$ and $R_{17}$ are each independently of the other $C_1$-$C_3$ alkoxy, or

f) $-OC(O)-R_{18}$ where $R_{18}$ is $C_1$-$C_3$ alkyl,

$R_2$ is methyl, methoxy or chlorine,

$R_3$ is $-CH_2OH$, $-N_3$ or $-CH_2OC(O)CH_3$

$R_4$ is hydrogen, methyl, methoxy, $NO_2$ or $NH_2$,

$R_5$ is hydrogen or methyl,

$R_6$ is hydrogen or methyl,

Y is as defined for formula I, and $R_7$ is $C_1$-$C_2$ alkyl which is unsubstituted or substituted by chlorine, $-OH$, $-OC_1$-$C_2$ alkyl or $-OC(O)NH-C_1$-$C_2$ alkyl,

$R_8$ is $C_1$-$C_3$ alkyl,

$R_9$ is hydrogen,

$R_{10}$ is methyl,

$R_{11}$ is $C_1$-$C_2$ alkyl, and the substituents $R_{11}$ together can also be $C_2$-$C_3$ alkylene,

$R_{12}$ is hydrogen, bromine, iodine or $C_1$-$C_2$ alkyl, and

X is oxygen.

4. A composition according to any one of claims 1 to 3, containing as active component a compound of formula I, wherein

$R_3$ is the azido group ($-N_3$), and the remaining symbols $R_1$ to $R_{12}$, X and Y are as defined in claim 3.

5. A composition according to any one of claims 1 to 4, containing as active component a compound of formula I, wherein

Y is the group $-CH(CH_3)COOH_3$ or

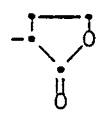

$R_3$ is the azido group $-(N_3)$, and the remaining symbols $R_1$ to $R_{12}$ and X are as defined in claim 3.

6. A composition according to any one of claims 1 to 5, which contains 0.1 to 99 per cent by weight of a compound of the formula I according to claims 1 to 5, 1 to 99 per cent by weight of adjuvants, and 0 to 25 per cent by weight of a surfactant.

7. A composition according to any one of claims 1 to 5, which contains 0.1 to 95 per cent by weight of a compound of the formula I according to claim 1.

8. A composition according to either of claims 6 or 7, which contains 0.1 to 25 per cent by weight of a surfactant.

9. A composition according to any one of claims 1 to 8, which contains as active ingredient a compound of formula I as claimed in any one of claims 2 to 6.

10. A process for the preparation of a composition for controlling and/or preventing infestation by microorganisms, which process comprises homogeneously mixing a compound of formula I according to any one of claims 1 to 5 with suitable carriers and/or surfactants.

11. A method of regulating plant growth and/or of controlling and/or preventing infestation by phytopathogenic microorganisms, which comprises the use of a compound of formula I according to any one of claims 1 to 5.

12. A method according to claim 11, which comprises the use of a compound of the formula I according to any one of claims 2 to 5.

13. A method according to claim 11, wherein the microorganisms are phytopathogenic fungi.

14. A method of controlling and/or preventing infestation of plants by phytopathogenic fungi, which process comprises applying a compound of the formula I according to any one of claims 1 to 5, in diluted form, to said plants or to the locus thereof.

15. A process for the preparation of a compound of formula I according to claim 1, which comprises

A) acylating a compound of formula II with a carboxylic acid of formula III or a reactive derivative thereof, in accordance with the reaction scheme

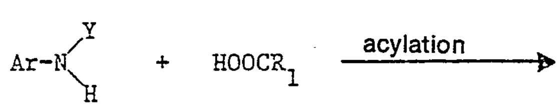


(I)

(II) (III)

or

B) reacting a compound of formula IV with a compound of formula V, wherein A is a customary leaving group, in accordance with the reaction scheme

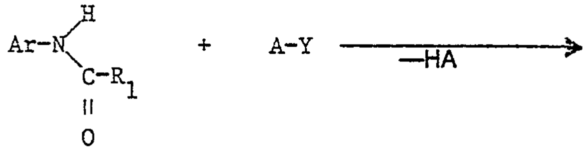


(IV) (V) (I)

16. A process for the preparation of a compound of formula Ia, wherein $R_3$ is the $—N_3$ group and $R_1$, $R_2$ and $R_4$ to $R_{12}$ and X and Y are as defined for formula I, which comprises forming the diazonium salt of a compound of formula VI

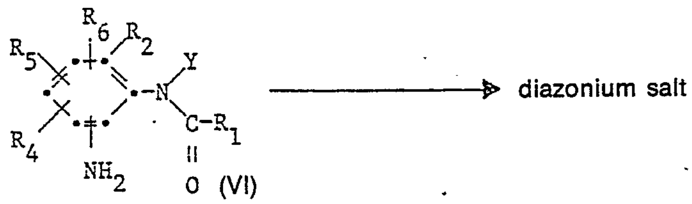


(VI)

and subsequently carrying out the variable reaction

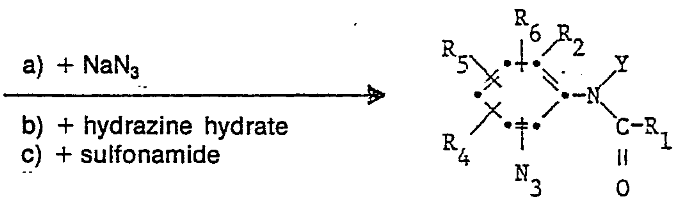


(Ia)

17. A process for the preparation of a compound of formula Ib, wherein $R_3$ is the $HOCH_2$ group and $R_1$, $R_2$ and $R_4$ to $R_{12}$ and X and Y are as defined for formula I, which comprises reacting a compound of formula VII with NaOH, in acetone, in accordance with the reaction scheme

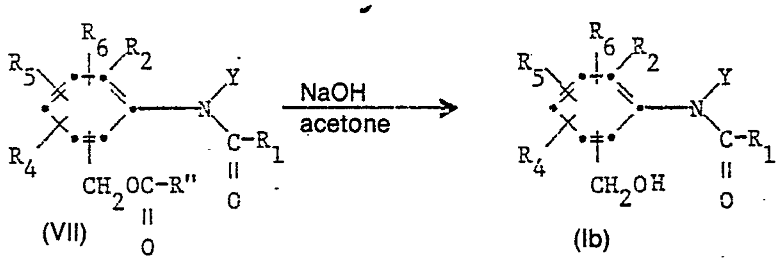


(VII) (Ib)

wherein R" is $C_1$-$C_4$ alkyl.

18. A process for the preparation of a compound of formula Ic, Id or Ie, wherein $R_3$ is a

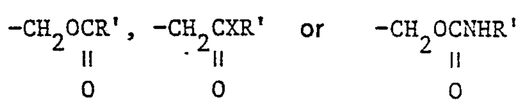


group, and $R_1$, $R_2$, $R_4$, to $R_{12}$ and X and Y are as defined for formula I, which process comprises reacting a compound of formula Ib with (a) Hal—CO—R' or (b) Hal—C(O)X—R' or (c) OCN—R', in accordance with the reaction scheme

(See scheme page 53)

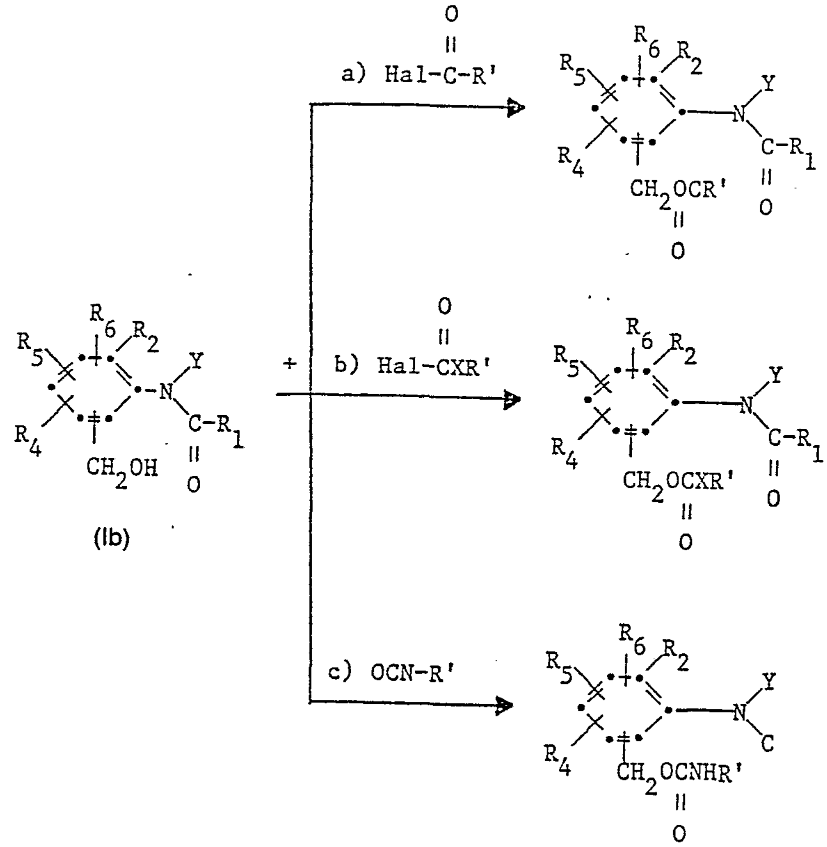


(Ib) (Ic) (Id) (Ie)

wherein Hal is halogen.

19. A process according to claim 15, wherein the reactive acid derivative of the formula III employed in method A) is an acid halide, an acid anhydride or an ester.

20. A process according to claim 19, wherein the acid halide is an acid chloride or acid bromide.

21. A process according to any one of claims 15, 19 or 20, wherein method A) is carried out in the presence of an acid acceptor or of a condensing agent.

22. A process according to any one of claims 15, 19, 20 or 21, wherein method A) is carried out in the presence of an inert solvent or solvent mixture.

23. A process according to claim 15, wherein method A) is carried out in the temperature range from 0° to 180 °C.

24. A process according to claim 23, wherein the reaction temperature is in the range from 0° to 150 °C.

25. A process according to claim 15, wherein the leaving group A in method B) is alkoxy, benzenesulfonyloxy, p-bromobenzenesulfonyloxy, p-tolyloxy, trifluoroacetyloxy, lower alkylsulfonyloxy or halogen.

26. A process for the preparation of a compound of formula VI, which comprises nitrating a compound of formula VIII with a reactive nitrating reagent to give a compound of formula IX, and subsequently hydrogenating said compound of formula IX with a conventional hydrogenating agent, in accordance with the reaction scheme :

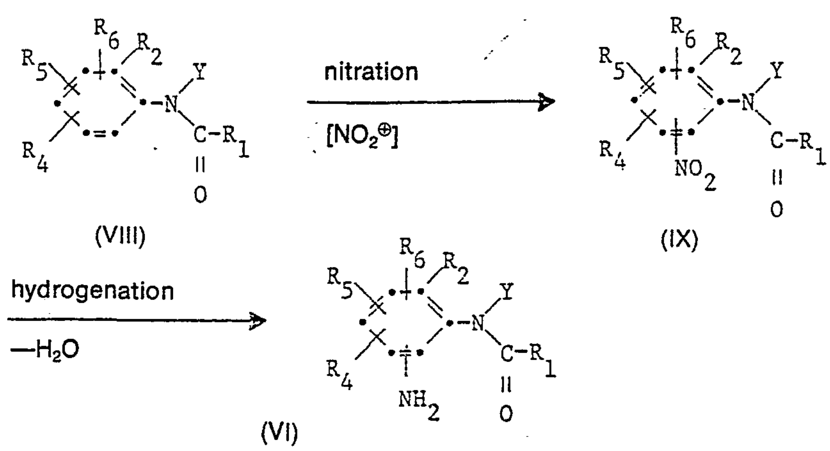


(VIII) (IX) (VI)

**Revendications** (pour les Etats contractants : BE, CH, DE, FR, GB, IT, LI, LU, NL, SE)

1. Composés de formule générale I

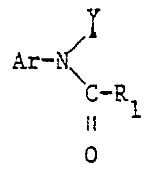

(I)

dans laquelle Ar représente un reste de formule

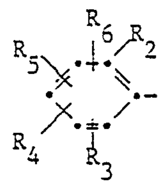

ou

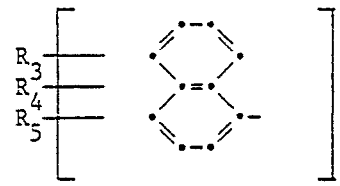

$R_1$ représente un groupe 2-furyle, 2-tétrahydrofuryle, alcényle en $C_2$-$C_4$, cyclopropyle, bêta-(alcoxy en $C_1$-$C_4$)-éthyle éventuellement substitué par des halogènes, ou le reste $—CH_2Z$ dans lequel Z représente :

a) —OH

b) un groupe —1H-1,2,4-triazolyle, 1-imidazolyle, 1-pyrazolyle,

c) un groupe $—S(O)_nR_{13}$ dans lequel $R_{13}$ représente un groupe alkyle en $C_1$-$C_4$ et n est égal à 1 ou 2,

d) un groupe $—X—R_{14}$ dans lequel X représente l'oxygène le soufre et $R_{14}$ représente un groupe alkyle en $C_1$-$C_6$, alcényle en $C_3$-$C_4$ ou alcynyle en $C_3$-$C_4$ éventuellement substitué par un groupe alcoxy en $C_1$-$C_2$,

e) un groupe $—OSO_2—R_{15}$ dans lequel $R_{15}$ représente un groupe alkyle en $C_1$-$C_4$, mono- ou di-(alkyle en $C_1$-$C_3$)-amine,

f) un groupe

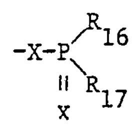

dans lequel X représente l'oxygène ou le soufre, $R_{16}$ représente un groupe alkyle en $C_1$-$C_4$, alcoxy en $C_1$-$C_4$ ou alkylamino en $C_1$-$C_4$ et $R_{17}$ représente un groupe alcoxy en $C_1$-$C_4$ ou alkylamino en $C_1$-$C_4$,

g) un groupe $—O—\underset{\underset{O}{\|}}{C}—R_{18}$ dans lequel $R_{18}$ représente un groupe alkyle en $C_1$-$C_3$ éventuellement substitué par un groupe alcoxy en $C_1$-$C_2$,

$R_2$ représente un groupe alkyle en $C_1$-$C_3$, alcoxy en $C_1$-$C_3$ ou un halogène,

$R_3$ représente un groupe

$$-CH_2OH,\ -N_3,\ -CH_2O\underset{\underset{O}{\|}}{C}R',\ -CH_2O\underset{\|}{C}XR'\ \text{ou}\ -CH_2O\underset{\|}{C}-NH-R',$$

dans lequel R' représente un groupe alkyle en $C_1$-$C_6$, alcényle en $C_2$-$C_4$ ou phényle éventuellement substitué par des halogènes ou des groupes alcoxy en $C_1$-$C_3$, et X représente l'oxygène ou le soufre,

$R_4$ représente l'hydrogène, un groupe alkyle en $C_1$-$C_3$, alcoxy en $C_1$-$C_3$, un halogène, un groupe $—NO_2$ ou $—NH_2$,

$R_5$ représente l'hydrogène ou un groupe alkyle en $C_1$-$C_3$,

$R_6$ représente l'hydrogène ou un groupe alkyle en $C_1$-$C_3$,

Y représente un groupe

$-\underset{\underset{R_7}{|}}{CH}—\underset{\underset{O}{\|}}{C}XR_8$ ,

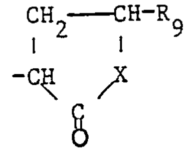

, $-\underset{R_{10}}{CH}-CN$

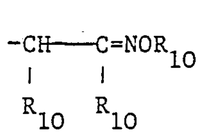 , 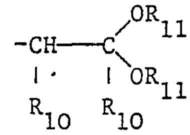 ou

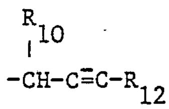

dans lequel

$R_7$ représente l'hydrogène, un groupe alkyle en $C_1$-$C_2$ éventuellement substitué par des halogènes, des groupes —OH, —O—alkyle en $C_1$-$C_3$ ou $-O\underset{\underset{O}{\|}}{C}NH$-alkyle en $C_1$-$C_3$,

$R_8$ représente un groupe alkyle en $C_1$-$C_4$ éventuellement substitué par un groupe alcoxy en $C_1$-$C_2$,

$R_9$ représente l'hydrogène ou un groupe méthyle,

$R_{10}$ représente l'hydrogène ou un groupe alkyle en $C_1$-$C_3$,

$R_{11}$ représente un groupe alkyle en $C_1$-$C_4$, les deux symboles $R_{11}$ pouvant également former ensemble un pont alkylène en $C_2$-$C_3$ mono- ou poly-substitué par des groupes alkyle en $C_1$-$C_3$,

$R_{12}$ représente l'hydrogène, un halogène, un groupe alkyle en $C_1$-$C_2$ ou phényle, et

X représente l'oxygène ou le soufre.

2. Composés de formule I selon la revendication 1, dans lesquels

$R_1$ représente un groupe 2-furyle, 2-tétrahydrofuryle, alcényle en $C_2$-$C_4$, cyclopropyle, bêta-(alcoxy en $C_1$-$C_2$)-éthyle ou $CH_2$—Z dans lequel Z représente :

a) un groupe —OH,

b) un groupe 1H-1,2,4-triazolyle, 1-imidazolyle,

c) un groupe —$S(O)_n$—$R_{13}$ dans lequel $R_{13}$ est un groupe alkyle en $C_1$-$C_2$ et n est égal à 2,

d) un groupe —X'—$R_{14}$ dans lequel X' représente l'oxygène ou le soufre et $R_{14}$ représente un groupe alkyle en $C_1$-$C_6$, alcényle en $C_3$-$C_4$ ou alcynyle en $C_3$-$C_4$ éventuellement substitué par un groupe alcoxy en $C_1$-$C_2$,

e) un groupe —$OSO_2$—$R_{15}$ dans lequel $R_{15}$ est un groupe alkyle en $C_1$-$C_2$, mono- ou di-(alkyle en $C_1$-$C_2$)-amine,

f) un groupe

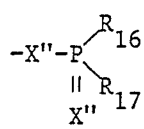

dans lequel X" représente le soufre, $R_{16}$ représente un groupe alkyle en $C_1$-$C_2$, alcoxy en $C_1$-$C_4$, alkylamino en $C_1$-$C_2$ et $R_{17}$ représente un groupe alcoxy en $C_1$-$C_4$ ou alkylamino en $C_1$-$C_4$, ou bien

g) un groupe $-O-\underset{\underset{O}{\|}}{C}-R_{18}$ dans lequel $R_{18}$ est un groupe alkyle en $C_1$-$C_2$ éventuellement substitué par un groupe alcoxy en $C_1$-$C_2$,

$R_2$ représente un groupe alkyle en $C_1$-$C_2$, alcoxy en $C_1$-$C_2$ ou un halogène,

$R_3$ représente un groupe

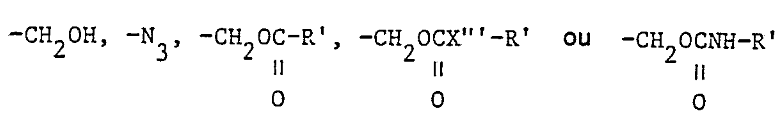

dans lequel R' représente un groupe alkyle en $C_1$-$C_3$ ou alcényle en $C_2$-$C_3$ éventuellement substitué par le chlore ou des groupes méthoxy et X'" représente l'oxygène ou le soufre,

$R_4$ représente l'hydrogène, un groupe alkyle en $C_1$-$C_2$, alcoxy en $C_1$-$C_2$, $NO_2$ ou $NH_2$,

$R_5$ représente l'hydrogène ou un groupe alkyle en $C_1$-$C_2$,

$R_6$ représente l'hydrogène ou un groupe alkyle en $C_1$-$C_2$,

Y a les significations indiquées en référence à la formule I,

$R_7$ représente l'hydrogène ou un groupe alkyle en $C_1$-$C_2$ éventuellement substitué par des halogènes, des groupes —OH, —O—(alkyle en $C_1$-$C_2$) ou $-O\underset{\underset{O}{\|}}{C}NH$-alkyle en $C_1$-$C_2$,

$R_8$ représente un groupe alkyle en $C_1$-$C_3$ éventuellement substitué par des groupes alcoxy en $C_1$-$C_2$,

$R_9$ représente l'hydrogène,

$R_{10}$ représente l'hydrogène ou un groupe alkyle en $C_1$-$C_2$,

$R_{11}$ représente un groupe alkyle en $C_1$-$C_2$, les deux symboles $R_{11}$ pouvant également représenter ensemble un groupe alkylène en $C_2$-$C_3$,

$R_{12}$ représente l'hydrogène, un halogène ou un groupe alkyle en $C_1$-$C_2$, et

X représente l'oxygène ou le soufre.

3. Composés de formule I selon la revendication 1, dans lesquels

$R_1$ représente un groupe 2-furyle, 2-tétrahydrofuryle, alcényle en $C_2$-$C_4$, cyclopropyle, 4-méthoxyéthyle ou $—CH_2Z$ dans lequel Z représente

a) un groupe 1H-1,2,4-triazolyle,

b) un groupe $—S(O)_n—R_{13}$ dans lequel $R_{13}$ représente $CH_3$ et n est égal à 2,

c) un groupe $—X'^{v}—R_{14}$ dans lequel $X'^{v}$ représente l'oxygène et $R_{14}$ représente un groupe alkyle en $C_1$-$C_4$, alcényle en $C_3$-$C_4$, ou alcynyle en $C_3$-$C_4$ éventuellement substitué par des groupes alcoxy en $C_1$-$C_2$,

d) un groupe $—OSO_2—R_{15}$ dans lequel $R_{15}$ est un groupe mono-(alkyle en $C_1$-$C_3$)-amine,

e) un groupe

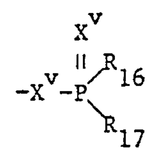

dans lequel $X^v$ représente le soufre, $R_{16}$ et $R_{17}$ représentent chacun, indépendamment l'un de l'autre, un groupe alcoxy en $C_1$-$C_3$, ou bien

f) un groupe $—O\underset{\overset{\|}{O}}{C}—R_{18}$ dans lequel $R_{18}$ représente un groupe alkyle en $C_1$-$C_3$,

$R_2$ représente un groupe méthyle, méthoxy ou le chlore,

$R_3$ représente un groupe $—CH_2OH$, $—N_3$ ou $—CH_2O\underset{\overset{\|}{O}}{C}CH_3$

$R_4$ représente l'hydrogène, un groupe méthyle, méthoxy, $NO_2$ ou $NH_2$,

$R_5$ représente l'hydrogène ou un groupe méthyle,

$R_6$ représente l'hydrogène ou un groupe méthyle,

Y a les significations indiquées en référence à la formule I, et

$R_7$ représente un groupe alkyle en $C_1$-$C_2$ éventuellement substitué par le chlore, des groupes —OH, —O—alkyle en $C_1$-$C_2$ ou $O\underset{\overset{\|}{O}}{C}NH$-alkyle en $C_1$-$C_2$,

$R_8$ représente un groupe alkyle en $C_1$-$C_3$,

$R_9$ représente l'hydrogène,

$R_{10}$ représente un groupe méthyle,

$R_{11}$ représente un groupe alkyle en $C_1$-$C_2$, les deux symboles $R_{11}$ pouvant également représenter ensemble un groupe alkylène en $C_2$-$C_3$,

$R_{12}$ représente l'hydrogène, le brome, l'iode ou un groupe alkyle en $C_1$-$C_2$, et

X représente l'oxygène.

4. Composés de formule I selon la revendication 3, dans lesquels,

$R_3$ représente le groupe azido ($—N_3$) et les autres symboles $R_1$ à $R_{12}$, X et Y ont les significations indiquées dans la revendication 3.

5. Composés de formule I selon la revendication 4, dans lesquels Y représente les restes $—CH(CH_3)COOCH_3$ ou

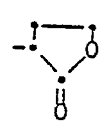

$R_3$ représente le groupe azido $—(N_3)$ et les autres symboles $R_1$ à $R_{12}$ et X ont les significations indiquées dans la revendication 3.

6. Un composé de formule I selon la revendication 4, choisi dans le groupe des composés en azido énumérés ci-après :

la N-(1'-méthoxycarbonyl-éthyl)-N-méthoxyacétyl-2-méthyl-6-azido-aniline,

la N-(tétrahydro-2'-one-fura-3'-yl)-N-méthoxyacétyl-2,6-diméthyl-3-azido-aniline,

la N-(1'-méthoxycarbonyl-éthyl)-N-méthoxyacétyl-2,6-diméthyl-3-aniline,

la N-(1'-méthoxycarbonyl-éthyl)-N-(2-tétrahydrofuryl-carbonyl)-2-méthyl-6-azido-aniline,
la N-(1'-méthoxycarbonyl-3'-hydroxy-n-propyl)-N-méthoxyacétyl-2-méthyl-6-azido-aniline,
la N-(1'-méthoxycarbonyl-éthyl)-N-méthylaminosulfonyloxyacétyl-2,6-diméthyl-3-azido-aniline,
la N-(1'-méthoxycarbonyl-3-hydroxy-n-propyl)-N-cyclopropylcarbonyl-2-méthyl-6-azido-aniline,
la N-(1'-méthoxycarbonyl-éthyl)-N-méthoxyacétyl-2,3-diméthyl-6-azido-aniline,
la N-(1'-cyanéthyl)-N-méthoxyacétyl-2-méthyl-6-azido-aniline,
la N-(1'-méthyl-2'-diméthoxy-éthyl)-N-méthoxyacétyl-2,6-diméthyl-3-azido-aniline,
la N-(2'-méthyl-3'-méthoximino-n-propyl)-N-méthoxy-acétyl-2-méthyl-6-azido-aniline,
la N-(tétrahydro-2'-one-fura-3'-yl)-N-méthoxyacétyl-2,3-diméthyl-4-azido-alpha-naphtylamine,
la N-(1'-cyanéthyl)-N-méthoxyacétyl-2,3-diméthyl-4-azido-alpha-naphtylamine,
la N-(1'-cyanéthyl)-N-(2-tétrahydrofuryl-carbonyl)-2,3-diméthyl-4-azido-alpha-naphtylamine.

7. Procédé de préparation des composés de formule I selon la revendication 1, caractérisé en ce que :

A) on acyle des composés de formule II

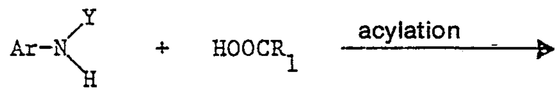


(I)

(II) (III)

par des acides carboxyliques de formule III ou leurs dérivés réactifs, ou bien

B) on fait réagir des composés de formule IV

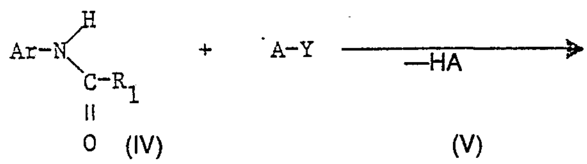


(I)

(IV) (V)

avec des composés de formule V dans laquelle A représente un groupe éliminable usuel.

8. Procédé de préparation des composés de formule Ia dans lesquels $R_3$ représente le groupe $N_3$ et les symboles $R_1$, $R_2$ et $R_4$ à $R_{12}$ et X et Y ont les significations indiquées en référence à la formule I, par formation des sels de diazonium des composés de formule VI

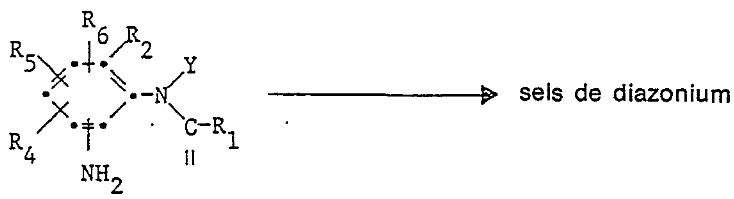


et réaction subséquente au choix

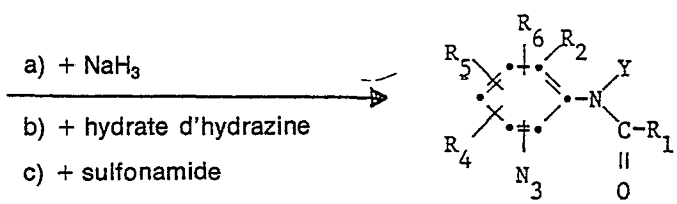


(Ia)

9. Procédé de préparation des composés de formule Ib dans lesquels $R_3$ représente le groupe $HOCH_2$ et les symboles $R_1$, $R_2$ et $R_4$ à $R_{12}$ et X et Y ont les significations indiquées en référence à la formule I, par réaction des composés de formule VII avec NaOH dans l'acétone

(VII)

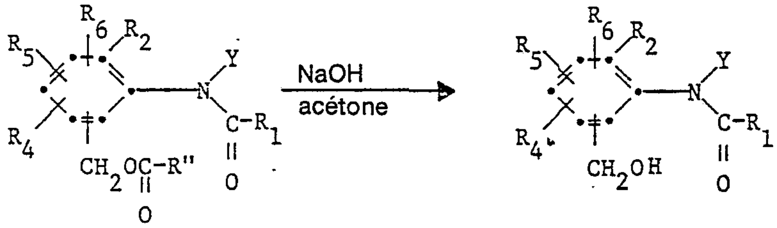


(Ib)

R" représentant un groupe alkyle en $C_1$-$C_4$.

10. Procédé de préparation des composés de formules Ic, Id et Ie dans lesquels $R_3$ représente l'un des groupes

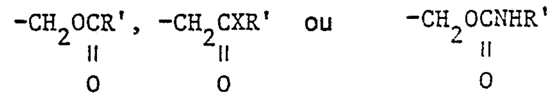

et les symboles $R_1$, $R_2$ et $R_4$ à $R_{12}$ et X et Y ont les significations indiquées en référence à la formule I, par réaction des composés de formule Ib avec (a) Hal—CO—R' ou (b) $Hal—\underset{\overset{\|}{O}}{C}X—R'$ ou (c) OCN—R'

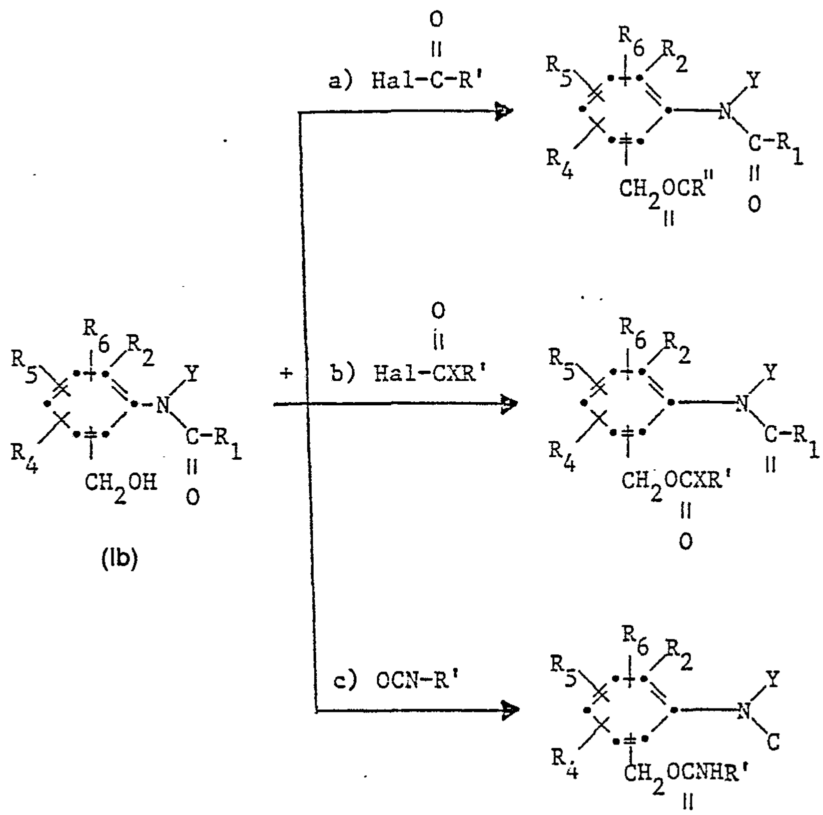


(Ib) (Ic) (Id) (Ie)

Hal représentant un halogène.

11. Procédé selon la revendication 7, caractérisé en ce que, dans le procédé A), on utilise en tant que dérivé d'acide réactif de formule III un halogénure d'acide, un anhydride ou un ester.

12. Procédé selon la revendication 11, caractérisé en ce que l'halogénure d'acide est le chlorure d'acide ou le bromure d'acide.

13. Procédé selon les revendications 7, 11 et 12, caractérisé en ce que le procédé A) est mis en œuvre en présence d'un agent fixant les acides ou d'un agent de condensation.

14. Procédé selon les revendications 7, 11, 12 et 13, caractérisé en ce que le procédé A) est mis en œuvre en présence d'un solvant ou mélange solvant inerte dans la réaction.

15. Procédé selon la revendication 7, caractérisé en ce que le procédé A) est mis en œuvre à des températures de 0 à 180 °C.

16. Procédé selon la revendication 15, caractérisé en ce que la température de réaction est de 0 à 150 °C.

17. Procédé selon la revendication 7, caractérisé en ce que, dans le procédé B), on utilise en tant que groupe éliminable A) un groupe alcoxy, benzène-sulfonyloxy, p-bromobenzène-sulfonyloxy, p-tosyloxy, trifluoracétyloxy, alkylsulfonyloxy inférieur ou un halogène.

18. Procédé de préparation des composés de formule VI, caractérisé en ce que l'on convertit des composés de formule VIII, par nitration à l'aide d'un agent nitrant réactif, en composés de formule IX qu'on hydrogène ensuite à l'aide d'un agent hydrogénant usuel

(Voir schéma page 59)

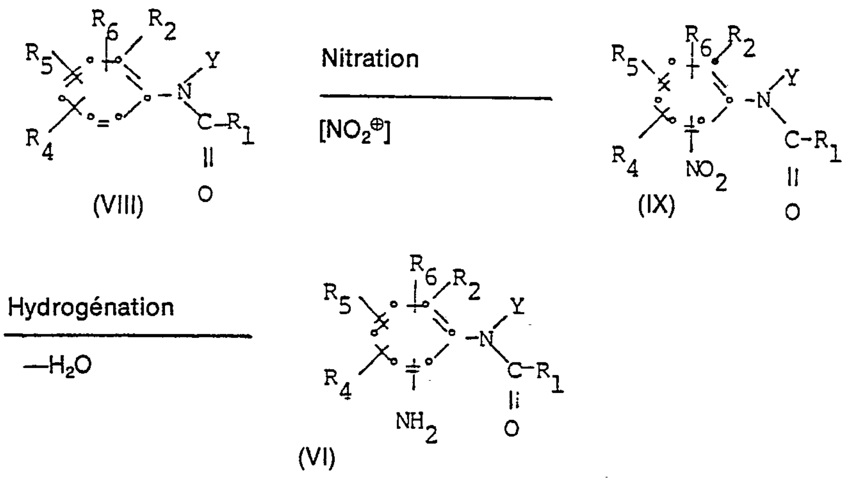


(VIII) (IX) (VI)

19. Produit pour combattre et/ou prévenir une infestation par des microorganismes nuisibles, caractérisé en ce qu'il contient en tant que composant actif au moins une substance active de formule I selon la revendication 1.

20. Produit selon la revendication 19, caractérisé en ce qu'il contient de 0,1 à 99 % en poids d'une substance active de formule I selon la revendication 1, de 1 à 99 % en poids d'additifs et de 0 à 25 % en poids d'un agent tensioactif.

21. Produit selon la revendication 19, caractérisé en ce qu'il contient de 0,1 à 95 % en poids d'une substance active de formule I selon la revendication 1.

22. Produit selon les revendications 20 et 21, caractérisé en ce qu'il contient de 0,1 à 25 % en poids d'un agent tensioactif.

23. Produit selon l'une des revendications 19 à 22, caractérisé en ce qu'il contient en tant que substance active l'un des composés de formule I selon l'une des revendications 2 à 6.

24. Procédé de préparation de produits servant à combattre et/ou prévenir une infestation par des microorganismes, caractérisé en ce que l'on mélange intimement un composé de formule I selon l'une des revendications 1 à 6, avec des véhicules et/ou agents tensioactifs appropriés.

25. Utilisation des composés de formule I selon la revendication 1, pour la régulation de la croissance des végétaux et/ou pour combattre et/ou prévenir une infestation par des microorganismes phytopathogènes.

26. Utilisation selon la revendication 25 de composés de formule I selon l'une des revendications 2 à 6.

27. Utilisation selon la revendication 25, caractérisée en ce que les microorganismes sont des mycètes phytopathogènes.

28. Procédé pour combattre et/ou prévenir une infestation de végétaux par des mycètes phytopathogènes, caractérisé en ce que l'on applique sur la plante ou sur son habitat un composé de formule I selon l'une des revendications 1 à 6, à l'état dilué.

**Revendications** (pour l'Etat contractant AT)

1. Produit pour combattre et/ou prévenir une infestation par des microorganismes nuisibles, caractérisé en ce qu'il contient en tant que composant actif au moins une substance active de formule générale I

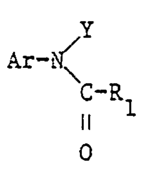

(I)

dans laquelle Ar représente un reste de formule

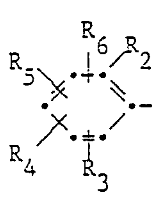

ou

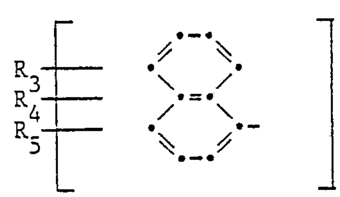

$R_1$ représente un groupe 2-furyle, 2-tétrahydrofuryle, alcényle en $C_2$-$C_4$, cyclopropyle, bêta-(alcoxy en $C_1$-$C_4$)-éthyle éventuellement substitué par des halogènes, ou le reste $—CH_2Z$ dans lequel Z représente :

a) —OH

b) un groupe —1H-1,2,4-triazolyle, 1-imidazolyle, 1-pyrazolyle,

c) un groupe $—S(O)_n—R_{13}$ dans lequel $R_{13}$ représente un groupe alkyle en $C_1$-$C_4$ et n est égal à 1 ou 2,

d) un groupe $—X—R_{14}$ dans lequel X représente l'oxygène le soufre et $R_{14}$ représente un groupe alkyle en cynyle en $C_1$-$C_6$, alcényle en $C_3$-$C_4$ ou alcynyle en $C_3$-$C_4$ éventuellement substitué par un groupe alcoxy en $C_1$-$C_2$,

e) un groupe $—OSO_2—R_{15}$ dans lequel $R_{15}$ représente un groupe alkyle en $C_1$-$C_4$, mono- ou di-(alkyle en $C_1$-$C_3$)-amine,

f) un groupe

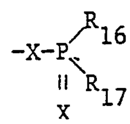

dans lequel X représente l'oxygène ou le soufre, $R_{16}$ représente un groupe alkyle en $C_1$-$C_4$, alcoxy en $C_1$-$C_4$ ou alkylamino en $C_1$-$C_4$ et $R_{17}$ représente un groupe alcoxy en $C_1$-$C_4$ ou alkylamino en $C_1$-$C_4$,

g) un groupe $—O—C(=O)—R_{18}$ dans lequel $R_{18}$ représente un groupe alkyle en $C_1$-$C_3$ éventuellement substitué par un groupe alcoxy en $C_1$-$C_2$,

$R_2$ représente un groupe alkyle en $C_1$-$C_3$, alcoxy en $C_1$-$C_3$ ou un halogène,

$R_3$ représente un groupe

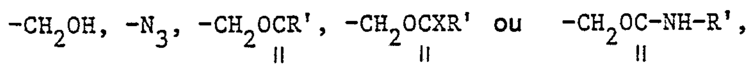

dans lequel R' représente un groupe alkyle en $C_1$-$C_6$, alcényle en $C_2$-$C_4$ ou phényle éventuellement substitué par des halogènes ou des groupes alcoxy en $C_1$-$C_3$, et X représente l'oxygène ou le soufre,

$R_4$ représente l'hydrogène, un groupe alkyle en $C_1$-$C_3$, alcoxy en $C_1$-$C_3$, un halogène, un groupe $—NO_2$ ou $—NH_2$,

$R_5$ représente l'hydrogène ou un groupe alkyle en $C_1$-$C_3$,

$R_6$ représente l'hydrogène ou un groupe alkyle en $C_1$-$C_3$,

Y représente un groupe

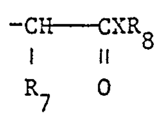 , 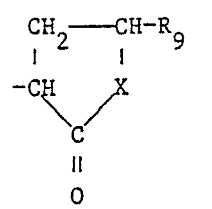 , 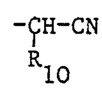

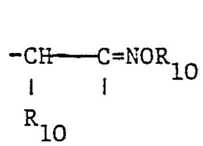 , 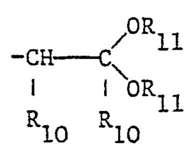 ou

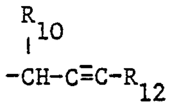

dans lequel

$R_7$ représente l'hydrogène, un groupe alkyle en $C_1$-$C_2$ éventuellement substitué par des halogènes, des groupes —OH, —O-alkyle en $C_1$-$C_3$ ou $—OC(=O)NH$-alkyle en $C_1$-$C_3$,

$R_8$ représente un groupe alkyle en $C_1$-$C_4$ éventuellement substitué par un groupe alcoxy en $C_1$-$C_2$,
$R_9$ représente l'hydrogène ou un groupe méthyle,
$R_{10}$ représente l'hydrogène ou un groupe alkyle en $C_1$-$C_3$,
$R_{11}$ représente un groupe alkyle en $C_1$-$C_4$, les deux symboles $R_{11}$ pouvant également former ensemble un pont alkylène en $C_2$-$C_3$ mono- ou poly-substitué par des groupes alkyle en $C_1$-$C_3$,
$R_{12}$ représente l'hydrogène, un halogène, un groupe alkyle en $C_1$-$C_2$ ou phényle, et
X représente l'oxygène ou le soufre.

2. Produit selon la revendication 1, contenant en tant que composant actif un composé de formule I dans laquelle $R_1$ représente un groupe 2-furyle, 2-tétrahydrofuryle, alcényle en $C_2$-$C_4$, cyclopropyle, bêta-(alcoxy en $C_1$-$C_2$)-éthyle ou $CH_2$—Z dans lequel Z représente :
a) un groupe —OH,
b) un groupe 1H-1,2,4-triazolyle, 1-imidazolyle,
c) un groupe $—S(O)_nR_{13}$ dans lequel $R_{13}$ est un groupe alkyle en $C_1$-$C_2$ et n est égal à 2,
d) un groupe —X'—$R_{14}$ dans lequel X' représente l'oxygène ou le soufre et $R_{14}$ représente un groupe alkyle en $C_1$-$C_6$, alcényle en $C_3$-$C_4$ ou alcynyle en $C_3$-$C_4$ éventuellement substitué par un groupe alcoxy en $C_1$-$C_2$,
e) un groupe $—OSO_2—R_{15}$ dans lequel $R_{15}$ est un groupe alkyle en $C_1$-$C_2$, mono- ou di-(alkyle en $C_1$-$C_2$)-amine,
f) un groupe

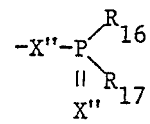

dans lequel X" représente le soufre, $R_{16}$ représente un groupe alkyle en $C_1$-$C_2$, alcoxy en $C_1$-$C_4$, alkylamino en $C_1$-$C_2$ et $R_{17}$ représente un groupe alcoxy en $C_1$-$C_4$ ou alkylamino en $C_1$-$C_4$, ou bien
g) un groupe $—O—\underset{\underset{O}{\|}}{C}—R_{18}$ dans lequel $R_{18}$ est un groupe alkyle en $C_1$-$C_2$ éventuellement substitué par un groupe alcoxy en $C_1$-$C_2$,
$R_2$ représente un groupe alkyle en $C_1$-$C_2$, alcoxy en $C_1$-$C_2$ ou un halogène,
$R_3$ représente un groupe

$$-CH_2OH,\ -N_3,\ -CH_2O\underset{\underset{O}{\|}}{C}-R',\ -CH_2O\underset{\underset{O}{\|}}{C}X'''-R' \quad \text{ou} \quad -CH_2O\underset{\underset{O}{\|}}{C}NH-R'$$

dans lequel R' représente un groupe alkyle en $C_1$-$C_3$ ou alcényle en $C_2$-$C_3$ éventuellement substitué par le chlore ou des groupes méthoxy et X'" représente l'oxygène ou le soufre,
$R_4$ représente l'hydrogène, un groupe alkyle en $C_1$-$C_2$, alcoxy en $C_1$-$C_2$, $NO_2$ ou $NH_2$,
$R_5$ représente l'hydrogène ou un groupe alkyle en $C_1$-$C_2$,
$R_6$ représente l'hydrogène ou un groupe alkyle en $C_1$-$C_2$,
Y a les significations indiquées en référence à la formule I,
$R_7$ représente l'hydrogène ou un groupe alkyle en $C_1$-$C_2$ éventuellement substitué par des halogènes, des groupes —OH, —O—(alkyle en $C_1$-$C_2$) ou $—O\underset{\underset{O}{\|}}{C}NH$-alkyle en $C_1$-$C_2$,
$R_8$ représente un groupe alkyle en $C_1$-$C_3$ éventuellement substitué par des groupes alcoxy en $C_1$-$C_2$,
$R_9$ représente l'hydrogène,
$R_{10}$ représente l'hydrogène ou un groupe alkyle en $C_1$-$C_2$,
$R_{11}$ représente un groupe alkyle en $C_1$-$C_2$, les deux symboles $R_{11}$ pouvant également représenter ensemble un groupe alkylène en $C_2$-$C_3$,
$R_{12}$ représente l'hydrogène, un halogène ou un groupe alkyle en $C_1$-$C_2$, et
X représente l'oxygène ou le soufre.

3. Produit selon les revendications 1 et 2, contenant en tant que composant actif un composé de formule 1, dans laquelle
$R_1$ représente un groupe 2-furyle, 2-tétrahydrofuryle, alcényle en $C_2$-$C_4$, cyclopropyle, 4-méthoxyéthyle ou $—CH_2Z$ dans lequel Z représente
a) un groupe 1H-1,2,4-triazolyle,
b) un groupe $—S(O)_n—R_{13}$ dans lequel $R_{13}$ représente $CH_3$ et n est égal à 2,
c) un groupe $—X'^{v}—R_{14}$ dans lequel $X'^{v}$ représente l'oxygène et $R_{14}$ représente un groupe alkyle en $C_1$-$C_4$, alcényle en $C_3$-$C_4$, ou alcynyle en $C_3$-$C_4$ éventuellement substitué par des groupes alcoxy en $C_1$-$C_2$,

d) un groupe $-OSO_2-R_{15}$ dans lequel $R_{15}$ est un groupe mono-(alkyle en $C_1$-$C_3$)-amine,
e) un groupe

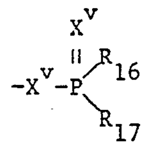

dans lequel $X^v$ représente le soufre, $R_{16}$ et $R_{17}$ représentent chacun, indépendamment l'un de l'autre, un groupe alcoxy en $C_1$-$C_3$, ou bien

f) un groupe $-O\underset{\underset{O}{\|}}{C}-R_{18}$ dans lequel $R_{18}$ représente un groupe alkyle en $C_1$-$C_3$,

$R_2$ représente un groupe méthyle, méthoxy ou le chlore,
$R_3$ représente un groupe $-CH_2OH$, $-N_3$ ou $-CH_2O\underset{\underset{O}{\|}}{C}CH_3$

$R_4$ représente l'hydrogène, un groupe méthyle, méthoxy, $NO_2$ ou $NH_2$,
$R_5$ représente l'hydrogène ou un groupe méthyle,
$R_6$ représente l'hydrogène ou un groupe méthyle,
Y a les significations indiquées en référence à la formule I, et
$R_7$ représente un groupe alkyle en $C_1$-$C_2$ éventuellement substitué par le chlore, des groupes $-OH$, $-O-$alkyle en $C_1$-$C_2$ ou $O\underset{\underset{O}{\|}}{C}NH-$alkyle en $C_1$-$C_2$,

$R_8$ représente un groupe alkyle en $C_1$-$C_3$,
$R_9$ représente l'hydrogène,
$R_{10}$ représente un groupe méthyle,
$R_{11}$ représente un groupe alkyle en $C_1$-$C_2$, les deux symboles $R_{11}$ pouvant également représenter ensemble un groupe alkylène en $C_2$-$C_3$,
$R_{12}$ représente l'hydrogène, le brome, l'iode ou un groupe alkyle en $C_1$-$C_2$, et
X représente l'oxygène.

4. Produits selon les revendications 1 à 3, contenant en tant que composant actif un composé de formule I dans laquelle
$R_3$ représente le groupe azido ($-N_3$) et les autres symboles $R_1$ à $R_{12}$, X et Y ont les significations indiquées dans la revendication 3.

5. Produit selon les revendications 1 à 4, contenant en tant que composant actif un composé de formule I dans laquelle
Y représente les restes $-CH(CH_3)COOCH_3$ ou

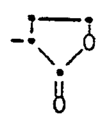

$R_3$ représente le groupe azido $-(N_3)$ et les autres symboles $R_1$ à $R_{12}$ et X ont les significations indiquées dans la revendication 3.

6. Produit selon les revendications 1 à 5, caractérisé en ce qu'il contient de 0,1 à 99 % en poids d'une substance active de formule I selon les revendications 1 à 5, 1 à 99 % en poids d'additifs et 0 à 25 % en poids d'un agent tensioactif.

7. Produit selon les revendications 1 à 5, caractérisé en ce qu'il contient de 0,1 à 95 % en poids d'une substance active de formule I selon la revendication 1.

8. Produit selon les revendications 6 et 7, caractérisé en ce qu'il contient de 0,1 à 25 % en poids d'un agent tensioactif.

9. Produit selon les revendications 1 à 8, caractérisé en ce qu'il contient en tant que substance active l'un des composés de formule I selon l'une des revendications 2 à 6.

10. Procédé de préparation de produits pour combattre et/ou prévenir une infestation par des microorganismes, caractérisé en ce que l'on mélange intimement un composé de formule I selon l'une des revendications 1 à 5, avec des véhicules et/ou agents tensioactifs appropriés.

11. Utilisation des composés de formule I selon les revendications 1 à 5 pour la régulation de la croissance des végétaux et/ou pour combattre et/ou prévenir une infestation par des microorganismes phytopathogènes.

12. Utilisation selon la revendication 11 de composés de formule I selon l'une des revendications 2 à 5.

13. Utilisation selon la revendication 11, caractérisée en ce que les microorganismes sont des mycètes phytopathogènes.

14. Procédé pour combattre et/ou prévenir une infestation de végétaux par des mycètes phytopathogènes, caractérisé en ce que l'on applique sur la plante ou son habitat un composé de formule I selon l'une des revendications 1 à 5, à l'état dilué.

15. Procédé de préparation des composés de formule I de la revendication 1, caractérisé en ce que :

A) on acyle des composés de formule II

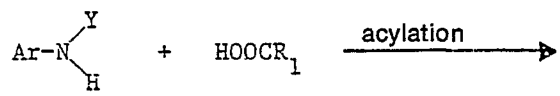


(I)

(II) (III)

par des acides carboxyliques de formule III ou leurs dérivés réactifs, ou bien

B) on fait réagir des composés de formule IV

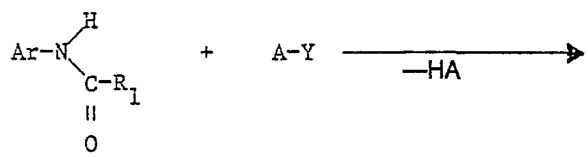


(I)

(IV)

avec des composés de formule V dans laquelle A représente un groupe éliminable usuel.

16. Procédé de préparation des composés de formule Ia dans laquelle $R_3$ représente le groupe $N_3$ et les symboles $R_1$, $R_2$ et $R_4$ à $R_{12}$ et X et Y ont les significations indiquées en référence à la formule I, par formation des sels de diazonium des composés de formule VI

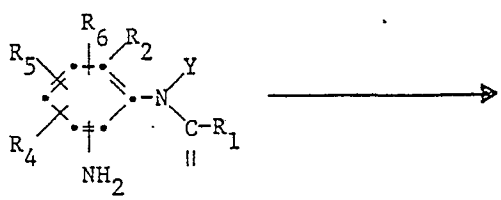

sels de diazonium

et réaction subséquente au choix.

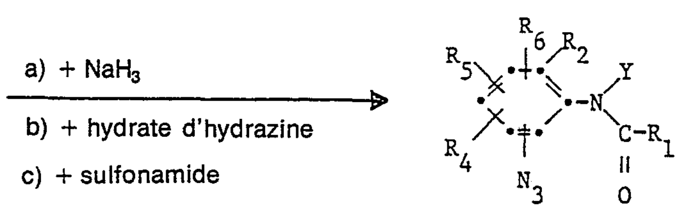


(Ia)

17. Procédé de préparation des composés de formule Ib dans laquelle $R_3$ représente le groupe $HOCH_2$ et les symboles $R_1$, $R_2$ et $R_4$ à $R_{12}$ et X et Y ont les significations indiquées en référence à la formule I, par réaction des composés de formule VII avec NaOH dans l'acétone

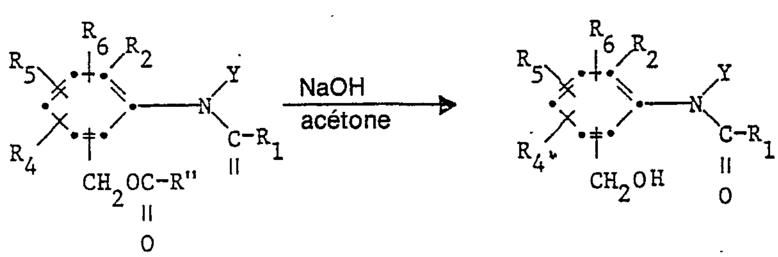


(VII) (Ib)

R" représentant un groupe alkyle en $C_1$-$C_4$.

18. Procédé de préparation des composés de formules Ic, Id et Ie dans lesquelles $R_3$ représente l'un des groupes

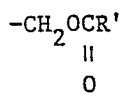, 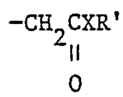 ou 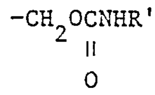

et les symboles $R_1$, $R_2$ et $R_4$ à $R_{12}$ et X et Y ont les significations indiquées en référence à la formule I, par réaction des composés de formule Ib avec (a) Hal—CO—R' ou (b) 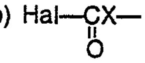 (c) OCN—R'

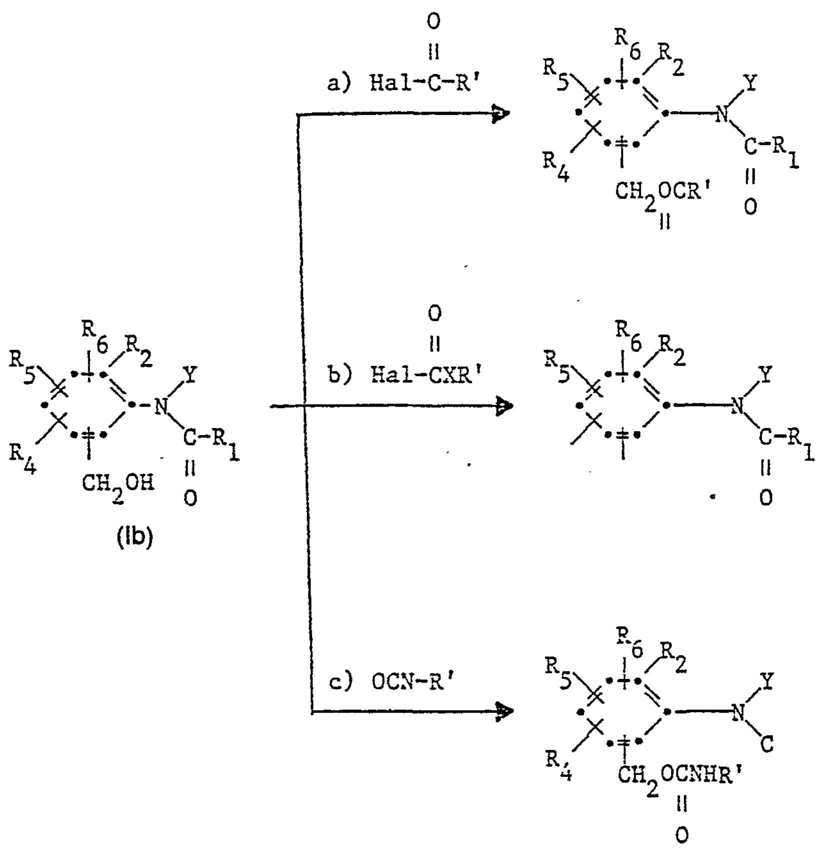

(Ib)

(Ic)

(Id)

(Ie)

Hal représentant un halogène.

19. Procédé selon la revendication 15, caractérisé en ce que, dans le procédé A), on utilise en tant que dérivé d'acide réactif de formule III un halogénure d'acide, un anhydride ou un ester.

20. Procédé selon la revendication 19, caractérisé en ce que l'halogénure d'acide est le chlorure d'acide ou le bromure d'acide.

21. Procédé selon des revendications 15, 19 et 20, caractérisé en ce que le procédé A) est mis en œuvre en présence d'un agent fixant les acides ou d'un agent de condensation.

22. Procédé selon les revendications 15, 19, 20 et 21, caractérisé en ce que le procédé A) est mis en œuvre en présence d'un solvant ou mélange solvant inerte dans la réaction.

23. Procédé selon la revendication 15, caractérisé en ce que le procédé A) est mis en œuvre à des températures de 0 à 180 °C.

24. Procédé selon la revendication 23, caractérisé en ce que la température de réaction va de 0 à 150 °C.

25. Procédé selon la revendication 15, caractérisé en ce que, dans le procédé B), on utilise en tant que groupe éliminable A) un groupe alcoxy, benzène-sulfonyloxy, p-bromo-benzène-sulfonyloxy, p-tosyloxy, trifluoracétyloxy, alkylsulfonyloxy inférieur ou un halogène.

26. Procédé de préparation des composés de formule VI, caractérisé en ce que l'on convertit des composés de formule VIII, par nitration à l'aide d'un agent nitrant réactif, en composés de formule IX qu'on hydrogène ensuite à l'aide d'un agent hydrogénant usuel.

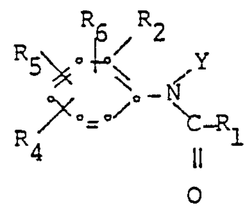

(VIII)

Nitration

$[NO_2^{\oplus}]$

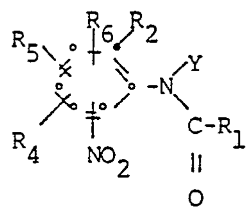

(IX)

Hydrogénation

$-H_2O$

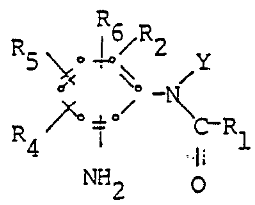

(VI)